# EUROPEAN PATENT SPECIFICATION

(11) **EP 3 626 193 B1**
(45) Date of publication and mention of the grant of the patent: **09.10.2024**
(21) Application number: 19208137.0
(22) Date of filing: 11.03.2014
(51) Int. Cl.: A61B 18/02, A61B 18/00, A61B 18/04

(54) **CRYOGENIC TREATMENT SYSTEMS**
KRYOGENE BEHANDLUNGSSYSTEME
SYSTÈMES DE TRAITEMENT CRYOGÉNIQUE

(30) Priority: 23.05.2013 US 201313900916; 06.09.2013 US 201314020265; 06.09.2013 US 201314020306; 06.09.2013 US 201314020350; 06.09.2013 US 201314020397; 06.09.2013 US 201314020452; 06.09.2013 US 201314019898; 06.09.2013 US 201314019928; 21.11.2013 US 201314086088; 21.11.2013 US 201314086050
(43) Date of publication of application: 25.03.2020
(62) Divisional of application: 14800541.6
(73) Proprietor: Channel Medsystems, Inc., Berkeley, CA 94710 (US)
(72) Inventor: BURNETT, Daniel Rogers, San Francisco, CA California 94127 (US); COTE, Ric, Oakland, CA California 94802 (US); MALECKI, William Walter, Piedmont, CA California 94611 (US); NEIL, Brian Michael, San Francisco, CA California 94122 (US); BEAULIEU, David, El Cerrito, CA California 94530 (US); VOILES, Benjamin D., San Francisco, CA California 94103 (US)
(74) Representative: J A Kemp LLP

(56) References cited:
- WO-A2-2013/019947
- US-A- 4 968 306
- US-A1- 2005 215 989
- US-A1- 2012 197 245
- US-B1- 6 547 784
- US-B1- 6 575 932
- US-B2- 7 794 454

## Description

### FIELD OF THE INVENTION

The present invention relates to medical devices. In particular, the present invention relates to apparatus for therapeutic devices capable of exposing areas of the body to elevated or decreased temperatures, in a highly controlled manner.

### BACKGROUND OF THE INVENTION

In the last few decades, therapeutic intervention within a body cavity or lumen has developed rapidly with respect to delivery of energy via radiofrequency ablation. While successful in several arenas, radiofrequency ablation has several major downsides, including incomplete ablation, frequent lack of visualization during catheter insertion, potential for overlap during treatment (with some areas receiving twice as much energy as other areas), charring of tissues and requirements for frequent debridement, frequent requirements for additional doses of energy after debridement, and potential perforation of the body cavity or lumen due to the rigidity of the RF electrodes.

The current state of the art would benefit from minimally invasive devices and methods which deliver thermal energy to a desired area or extract energy from a desired area, in a consistent, controlled manner that does not char or inadvertently freeze certain tissues or create excessive risk of unwanted organ or lumen damage.

US 2012/197245A discloses methods and apparatus for the treatment of a body cavity or lumen where a heated fluid and/or gas may be introduced through a catheter and into treatment area within the body contained between one or more inflatable/expandable members. The catheter may also have optional pressure and temperature sensing elements which may allow for control of the pressure and temperature within the treatment zone and also prevent the pressure from exceeding a pressure of the inflatable/expandable members to thereby contain the treatment area between these inflatable/expandable members.

WO 2013/019947A discloses an infusion catheter assembly which has the ability to infinitely titrate the length of a porous fluid distribution section in situ. The infusion catheter assembly thereby provides a length of infusion that can be infinitely varied over a prescribed range, to control the effective infusion length and the rate of infusion. Using the infusion catheter assembly, a physician has the capability to treat only the length of the vessel desired, thereby preventing excessive drug/patient exposures. The porous fluid distribution section can include an array of spaced apart apertures that vary in size and/or density along the length of the porous fluid distribution section, such that the flow rate remains essentially constant for a given inlet pressure independent of the effective infusion length.

### SUMMARY OF THE INVENTION

When bodily tissues are exposed to even slightly elevated temperatures (e.g., 42 degrees C or greater), focal damage may occur. If the tissues are exposed to temperatures greater than, e.g., 50 degrees C, for an extended period of time, tissue death will occur. The energy delivered by RF can then be excessive while a more controlled treatment can be achieved with heated fluids and/or vapors.

The present invention provides a tissue treatment system as set out in claim 1. Additional aspects of the invention are set out in the dependent claims.

### BRIEF DESCRIPTION OF THE DRAWINGS

For the purposes of the drawings and preferred embodiments, applications to the esophagus and uterus will be shown. However, the apparatus may be applied to any body cavity/lumen which may be visualized with an endoscope or other visualization mechanism.
Figure 1 shows an example of a device advanced through an endoscope, e.g., a nasally or orally inserted scope.
Figure 2 shows an example of a device advanced through the working channel of nasal endoscope.
Figure 3 shows an example of a device attached to a logic controller.
Figure 4 shows an example of a device placed through working channel of nasal endoscope and deployed within an esophagus for treatment.
Figure 5 shows an example of a device advanced alongside an endoscope.
Figures 6A to 6C show a device being introduced through an endoscope and deployed for treatment within the esophagus.
Figures 7A to 7C show examples of a device introduced through an endoscope for insertion within a bladder.
Figures 8A to 8C show examples of a device preparing the treatment area with a pre-treatment lavage prior to treatment.
Figure 9 shows an example of a distal occlude having an umbrella-like shape deployed in proximity to a gastroesophageal junction for treatment.
Figure 10 shows another example of an endoscopic balloon sheath having a distal occluder expanded distal to gastroesophageal junction for treatment.
Figure 11 shows another example where the treatment fluid is introduced between the deployed balloons for treatment.
Figure 12 shows another example of an adjustable size balloon device for treatment of the esophagus.
Figures 13A and 13B show another example of a single balloon device for ablation treatment within the uterus and/or endometrial lining.
Figures 14A and 14B show yet another example of conductive lattice/cage deployed for cryoablative treatment.
Figure 15 shows another example of an external cervical os occluding device.
Figure 16 shows another example of an internal cervical os occluding device.
Figures 17A and 17B show another example of a device having a deployable low-pressure conforming balloon used for cryogenic treatment of the uterus.
Figures 18A to 18D show another example of a conforming balloon which may also be filled partially or completely with a conductive material for cryoablative treatment.
Figure 19 shows another example of a cooling probe having one or more cooling members projecting from the distal end of a shaft.
Figure 20 shows another example of a cooling probe having a rotatable base and spray member.
Figure 21A shows a side view of an integrated treatment assembly.
Figure 21B shows an example of the assembly advanced through the cervix and into the uterus where the sheath may be retracted via the handle assembly to deploy the balloon.
Figure 21C shows a perspective view of a cryoablation assembly having a handle assembly which may integrate the electronics and pump assembly within the handle itself.
Figure 21D shows the handle assembly in a perspective exploded view illustrating some of the components which may be integrated within the handle.
Figure 21E shows an example of the system operation during a pre-treatment puff up process.
Figure 21F shows an example of the system operation during a treatment process.
Figure 21G shows an example of the system operation during a thawing and venting process.
Figure 22A shows a side view of a system which allows for adjustably setting a length of the balloon along the shaft.
Figure 22B shows a side view of the balloon everted within the shaft lumen for delivery.
Figures 23A and 23B show perspective and side views, respectively, of another example of a cooling probe assembly having a flat wire integrated through the probe.
Figure 24 shows a perspective view of the cooling probe assembly with one or more openings defined along the probe assembly.
Figures 25A and 25B show end views of a cross-section of the cooling probe and the distal end of the probe.
Figures 26A to 26L show perspective views of various tubular members which may be used for the cooling probe assembly.
Figures 27A and 27B show perspective views of a cooling probe assembly utilizing one or more discrete ring members linearly coupled to one another.
Figures 28A and 28B show cross-sectional end views of another variation of a cooling probe assembly coupled via a covering and/or insert members.
Figure 29 shows a perspective view of another variation of a cooling probe assembly having one or more insert members coupled along a wound spring body.
Figures 30A and 30B show cross-sectional side views of another variation of insert members supported along a spring body.
Figure 31 shows a detail side view of one variation of a pivotable cooling lumen body.
Figure 32 shows a side view of another variation of one or more insert members having an integrated covering.
Figure 33 shows a side view of yet another variation of one or more insert members having a slidable joint attached.
Figure 34 shows a side view of another variation of a spring body having one or more cooling lumens attached directly to the spring.
Figure 35 shows a side view of another variation of a spring body having the one or more insert members.
Figure 36 shows a side view of another variation of a spring body having the one or more cooling lumens and a secondary lumen.
Figure 37 show cross-sectional end views of variations of the secondary lumen.
Figures 38A and 38B show perspective views of another variation of the cooling probe utilizing a main delivery line and at least two side delivery lines.
Figure 38C shows a detail view of the side delivery line having an adjustable mandrel slidably positioned within.
Figure 39 shows a cross-sectional side view of another variation of the cooling probe assembly where the main delivery line and side delivery lines are in fluid communication through a common chamber.
Figure 40A and 40B show cross-sectional end views of variations of the exhaust lumen and the respective cooling lumens.
Figure 41 shows a cross-sectional side view of another variation of a cooling probe assembly having a single introduction line and a single delivery line.
Figure 42 shows a cross-sectional side view of a cooling probe assembly inserted within a balloon within the uterus.
Figures 43A and 43B show side views of various examples of side delivery lines having the openings aligned in different directions.
Figure 44 shows a side view of a cooling probe variation having a skived window for facilitating visualization.
Figure 45 shows a side view of an example of a balloon having one or more supporting arms extendable within the balloon.
Figure 46 shows a side view of another example of a balloon having one or more supporting arms attached to the cooling probe assembly.
Figure 47 shows a side view of another example of a balloon having one or more supporting arms also defining one or more openings for delivering the cryoablative fluid.
Figure 48 shows a side view of yet another example of a balloon having the one or more supporting arms positioned within elongate channels along the interior of the balloon.
Figures 49A and 49B show cross-sectional side views of yet another variation of a cooling probe which utilizes a single infusion line in combination with a translatable delivery line.
Figures 50A and 50B show top and perspective views of the expanded liner with four pairs of the open delivery ports exposed in apposed direction.
Figure 50C shows a perspective view of an expanded liner with additional open delivery ports exposed along an anterior portion of the liner.
Figure 51A shows one variation of a probe incorporating a transmitter to facilitate probe positioning within the liner.
Figure 51B shows another variation of a probe incorporating one or more transmitters to monitor tissue cavity expansion.
Figures 52A and 52B show a schematic illustration of a system utilizing a 5-port, 2 position, 4-way valve.
Figures 53A to 53C show a schematic illustration of a system utilizing a non-reversible pump for both inflation and deflation.
Figures 54A and 54B show top and perspective views of a liner illustrating its curved features when flattened and expanded and then deployed in a consistent manner.
Figure 54C shows a perspective view of a liner which may be pleated to fold and collapse in a consistent manner.
Figures 55A and 55B show side and top views of a probe which is configured to flex in the anterior and posterior directions.
Figure 55C shows a perspective view of a probe having multiple probe sections (e.g., four sections in this variation) separated by H-slots.
Figures 56A and 56B show perspective and detail cross-sectional views of a sheath assembly.
Figure 56C shows a cross-sectional view of a sheath assembly incorporating a sensor, e.g., temperature sensor.
Figure 57 shows one variation of a sheath bearing tube slidingly passing through a sheath bearing assembly and then attached to a slider base block assembly positioned within the handle assembly.
Figure 58 shows a detail perspective view of the connection between the sheath bearing tube and slider base block assembly.
Figures 59A and 59B illustrate how the slider base block assembly may be advanced distally or proximally relative to the handle assembly to expose the probe length.
Figure 60 shows a perspective view of one or more optional pressure sensing lines incorporated along the probe.
Figure 61 shows a side view of an example of an inflatable liner or balloon located along the outside distal surface of the sheath.
Figure 62 shows a side view of another example of an inflatable liner or balloon located along the inside distal surface of sheath.
Figure 63 shows a side view of another example where expandable foam may be deployed via the outer sheath.
Figure 64 shows a side view of another example where a heating element may be located along the inner or outer surface of the elongate shaft.
Figure 65 shows a side view of another example where a ring balloon may be inflated along either the sheath or shaft to either insulate the surrounding cervical tissue or to ensure secure placement of the shaft and/or balloon during treatment.
Figure 66 shows a cross-sectional side view of another variation where the outer sheath may be formed as an inflatable structure.
Figures 67A and 67B show side views of variations of an outer sheath having a reconfigurable distal end.
Figure 68 shows a side view of another variation of a balloon positioned along an outer surface of the outer sheath.
Figure 69 shows a cross-sectional side view of one variation of a dual-sheath design.
Figure 70A and 70B show cross-sectional detail views of the sealing between the inner and outer sheaths.
Figure 71 shows a partial cross-sectional side view of another dual-sheath variation having an expandable balloon contained between the sheaths.
Figure 72 shows a side view of another variation of a sheath having a reinforced structure.
Figure 73 shows a cross-sectional side view of another variation of an outer sheath having an adjustable balloon member.
Figures 74A and 74B show cross-sectional side views of another variation of an outer sheath having a reconfigurable distal end.
Figure 75 shows a cross-sectional side view of the reconfigurable distal end having one or more lubricious surfaces.
Figure 76 shows a partial cross-sectional side view of another variation where the reconfigurable distal end may be attached as a separate component.
Figure 77 shows a cross-sectional side view of another variation where a distal end of the cooling probe has a tapered distal end.
Figure 78 shows a side view of another variation of an outer sheath having a radially expandable portion.
Figures 79A and 79B show cross-sectional side views of variations of the locking mechanism for the expandable portion.
Figures 80A and 80B show cross-sectional side views of an illustrative example of an overcenter linkage mechanism.
Figure 81 shows a cross-sectional side view of another variation of an outer sheath having one or more distal cam members.
Figure 82 shows a cross-sectional side view of the one or more cam members deployed in their expanded configuration and secured against the cervical tissue.
Figure 83 shows a cross-sectional side view of another variation where the cammed distal end positioned on a tapered outer sheath.
Figure 84 shows a side view of an example of how the outer sheath may be initially deployed and secured and the cooling probe assembly advanced separately.
Figure 85 shows a side view of another variation where the outer sheath is configured as a corrugated structure.
Figure 86 shows a partial cross-sectional side view of another variation of the outer sheath having an inflatable balloon along an inner surface.
Figure 87 shows a partial cross-sectional side view of another variation of the outer sheath having an inflatable balloon along an outer surface.
Figure 88A to 88D show cross-sectional end view of variations of the outer sheath having an integrated feature to provide further insulation to the surrounding tissue.
Figure 89 shows an exemplary schematic illustration of the treatment assembly integrated into a single cooling system.
Figures 90A and 90B show a device for closing the exhaust flow path to facilitate a liner integrity check and to also increase the pressure within the uterine cavity.
Figures 91A and 91B show a schematic side view of a flapper valve and a top view of a gasket within the flapper valve for maintaining exhaust back pressure within the system.
Figures 91C to 91E show schematic side views illustrating an example of the flapper valve operation.
Figures 92A and 92B show schematic side views of another variation of the flapper valve.
Figure 93 shows how the various algorithms may be programmed into the processor or microcontroller and how they may functionally interact with one another.

### DETAILED DESCRIPTION OF THE INVENTION

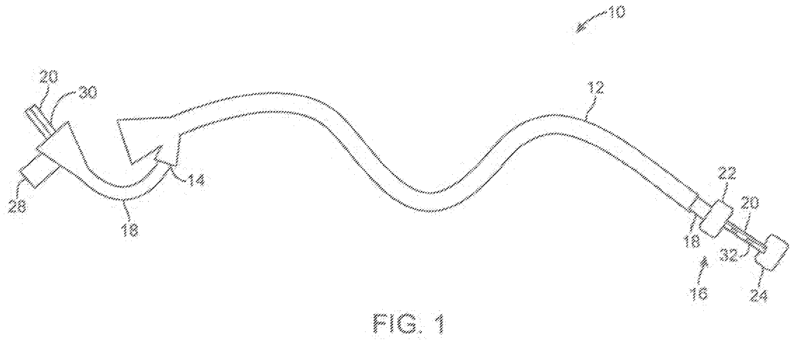

Figure 1 shows a perspective view of one example of the treatment assembly **10** positioned within a working channel **14** of an endoscope **12** (e.g., orally or nasally insertable scope). In this example, the treatment device **16** itself may utilize a first catheter **18** having an inflatable or expandable balloon member **22** and a second catheter **20** that may slide freely with respect to the first catheter **18** and also having an inflatable balloon member **24** at its distal end. The first catheter **18** as well as second catheter **20** may have a liquid and/or gas tight seal **30** formed at the proximal end of the catheters. The inflatable and/or expandable members **22, 24** (shown in this example as inflated balloons) may be pressurized to effectively and safely occlude the lumen. The balloons may be filled with chilled or room temperature fluid to prevent possible damage caused by balloon rupture or seepage around the balloon. Pressure within the inflatable or expandable balloon members may also be monitored to ensure that a tight seal has been formed within the lumen or body cavity.

Additionally, the liquid may be introduced into the treatment area through a liquid and/or gas port **28** and into the lumen of the catheter which terminates with the proximal balloon **22** and leaves the catheter through perforations or holes **32** within the second catheter **20** which terminates in the distal balloon **24,** although this flow path may easily be reversed if necessary. Alternatively, one or more ports can be designed into the lumen between the distal **24** and proximal **22** balloons, such that the heated or cooling fluid exits one or more ports **32** in the lumens near the distal balloon **24,** and is then evacuated in a port or ports designed within the lumen of the first catheter **18** nearest the proximal balloon **22.** In this variation, the endoscope **12** may insulate the catheters allowing the catheters to be much smaller than would be otherwise possible and allowing it to fit within the working channel **14** of a standard endoscope **12.** One or more pressure sensors may be used to detect both inflation pressures of the balloons and/or the pressure seen by the body cavity/lumen that is exposed to the treatment liquid/vapor. In the manner, liquid/vapor flow may be controlled by the pressure sensing elements within the body cavity/lumen to ensure that safe pressures are never exceeded. Manual controls may be used for creation and/or maintenance of these pressures (e.g. syringes with stopcocks) or automated and/or semi-automated systems can be used as well (e.g. pumps with PID loops and pressure sensing interconnectivity. Although the liquid and/or gas for tissue treatment may be heated or chilled prior to introduction into the treatment area in contact with the tissue, the liquid and/or gas may alternatively be heated or chilled after introduction into the treatment area and already in contact with the tissue.

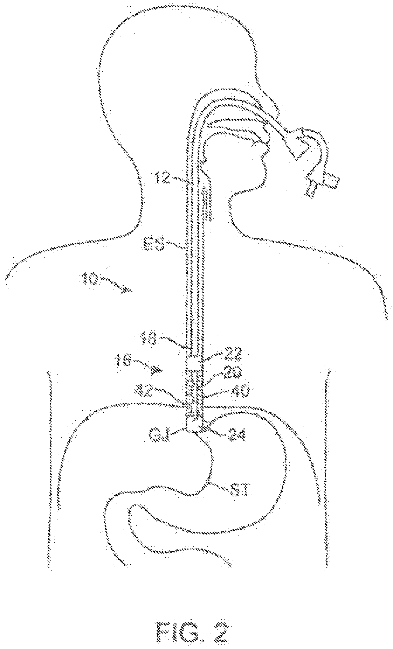

Figure 2 shows an example where the second catheter **20** and distal balloon member **24** is slidable relative to the proximal balloon **22.** This examples illustrates the endoscope inserted through the nasal cavity and advanced through the esophagus **ES** where the catheters **18, 20** may comprise single or multi-lumen catheters having inflation lumens for the distal **24** and proximal **22** inflatable/expandable elements, infusion port and extraction port. At least one of the catheters may be fitted with either a pressure transducer **42** or a lumen to carry the pressure signal from the treatment area back to the controller or dial gauge. Pressure sensing may be accomplished through a small, air capsule proximal to the distal balloon **24,** but within the treatment area. Both of the balloons **22, 24** may be inflated along the esophagus **ES** in the proximity to the gastroesophageal junction **GJ** proximal to the stomach **ST** to create a treatment space **40** which encompasses the tissue region to be treated.

In an alternative embodiment, an extraction lumen may be omitted as a preset dose of heated liquid and/or gas may be delivered, allowed to dwell and then either extracted through the same lumen or rendered harmless with the infusion of cold fluid. This treatment algorithm would provide an even simpler therapy and would rely on the exclusion of a certain area and exposure of that area to a liquid or vapor with the desired energy. Infusion of the liquid or vapor may be controlled to ensure that the treatment area is not exposed to excessive temperatures.

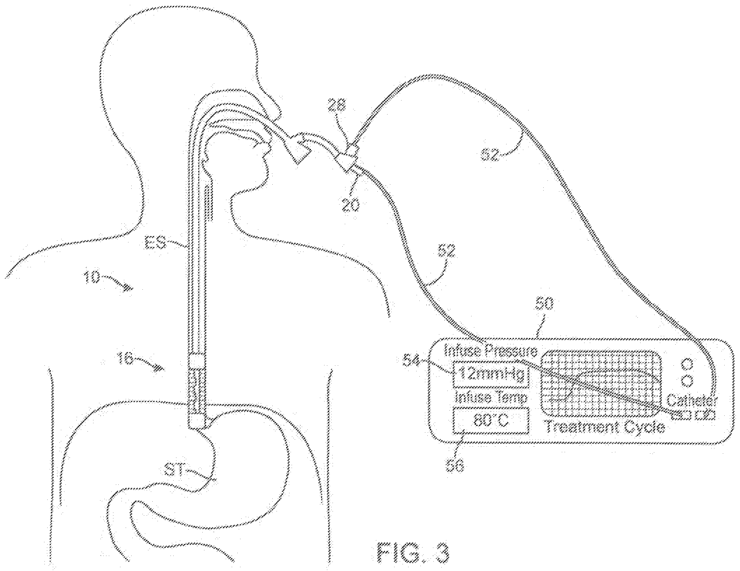

Figure 3 shows another example where the treatment assembly **10** may be in communication with a controller **50,** such as a logic controller. Controller **50** may control certain parameters such as infusion pressure **54** of the fluid as well as fluid temperature **56** and it may be coupled to the assembly by one or more cables **52.** The pressure in the treatment area, the elapsed time, the temperature of the fluid, and the extraction rate may also be monitored and controlled.

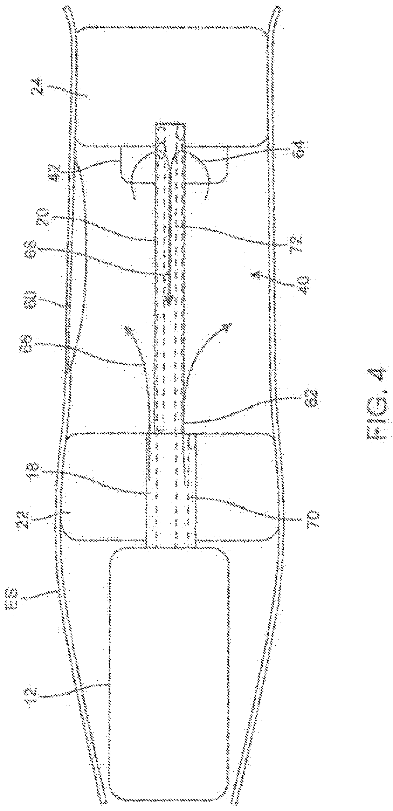

Figure 4 shows a detail view of the treatment area **40** defined, in this case, by two balloons **22, 24.** The first catheter **18** may open into the lumen just after the proximal balloon **22** and this catheter **18** may be inserted along with or prior to insertion of the second catheter **20.** The first catheter **18** internal diameter is greater than the outer diameter of the second catheter allowing for liquid (and/or vapor) to be infused or extracted around the outer diameter of the second catheter **20.** The second catheter **20** a first lumen for balloon inflation and a second lumen for evacuating the treatment region **40.** With the balloons inflated into contact against the esophagus **ES,** the treatment area **40** may encompass the tissue region to be treated, e.g., a lesion **60** such as Barrett's esophagus or esophageal cancer lesion and the distal end of the endoscope **12** may be positioned into close proximity to proximal balloon **22** and the treating liquid and/or gas **66** may be infused through the annular lumen **70** defined through first catheter **18** and between second catheter **20** such that the fluid **66** may enter through opening **62** into the treatment region **40** while contained by the balloons. Once treatment has been completed, the fluid may be evacuated **68** through one or more openings **64** located along the second catheter **20** proximal to distal balloon **24** and proximally through the second catheter **20** through the evacuation lumen **72.** As previously mentioned, a pressure sensor **42** (e.g., pressure measuring air capsule) may be positioned along either the first **18** and/or second 20 catheter for sensing the various parameters. Additionally, the treatment liquid and/or gas may include any number of liquids, vapors, or other chemically active (e.g., chemotherapeutic) or inactive compounds for additional treatments to the tissue.

In the event that the treatment is provided by a simple timed dwell, the extraction **72** and infusion **70** lumens may not both be utilized. The pressure sensing element **42** (solid-state, piezoelectric, or other method) may be located on either the first or second catheters and the second catheter and may comprise a simple slidable balloon. A pressure sensor for the treatment may be omitted so long as the pressure can be controlled by other mechanisms, e.g., a check valve or a simple gravity fluid column. An active pressure measurement, though, may ensure that safe pressures are not being exceeded.

The second catheter **20** may fit easily within the first catheter **18** and may be slid inside the first catheter **18** until its distal balloon **24** is distal to the first balloon **22.** The distal balloon **24** may then be inflated just beyond the distal portion of the treatment area **40** and the endoscope **12** may be pulled back. The most proximal extent of the lesion **60** may then be identified and the proximal balloon **22** may be inflated proximal to this area. Once the treatment area **40** has been enclosed (which may be verified by infusing liquid **66** and/or vapor under visualization and observing the seal around the balloon, balloons and/or expandable member) the lumen or body cavity may then be filled with the treatment liquid and/or vapor to a safe pressure. The liquid and/or vapor may also contain active agents (e.g. chemotherapeutic and/or anesthetic agents) and comprise more than simply an inactive liquid and/or vapor. Options would be for the active agents to be delivered prior to, during and/or post treatment of the heating (or cooling) liquid and/or vapor.

As the treatment assembly **16** does not contain the treatment liquid or vapor within a balloon(s) or expandable member and allows it to freely flow over the treatment area, the therapy may be applied consistently leaving no areas left untreated (as is frequently seen with balloon infusion-based or RF therapies). Additionally, treatment may be accomplished with a heated liquid (rather than a high energy electrode or excessively hot vapor) or a more controlled treatment can be achieved through the use of a relatively cooler liquid with a longer treatment time. In addition, the esophagus **ES** is a fluid transport type organ (lumen) and may be more compatible to fluid based therapies than with RF-based therapies. It is also believed that the safety margin of such treatments may be better than with an RF-based therapy.

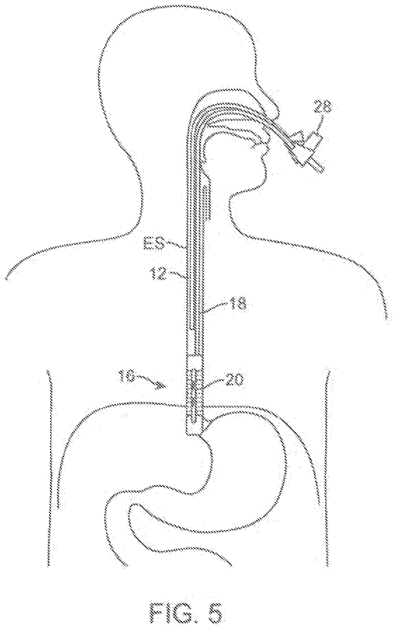

Figure 5 shows an alternative embodiment of the device in which the first catheter **18** and second catheter **20** of the treatment assembly **16** may be inserted alongside an endoscope **12** which may be used to provide for visualization. Due to the small size of the catheters, this embodiment is feasible.

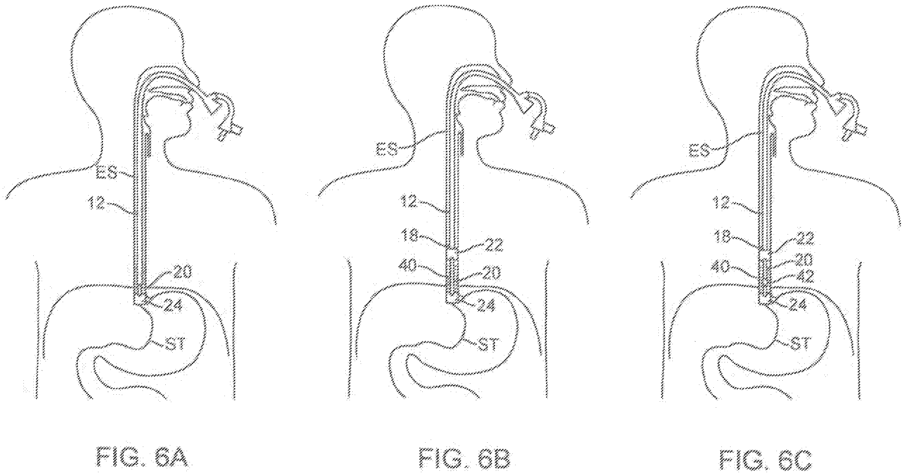

Figures 6A to 6C illustrate an example for a placement procedure for the assembly for the treatment of a body lumen such as the esophagus **ES.** The catheters may be inserted simultaneously or separately through the working channel of the endoscope **12.** In one example, the larger first catheter **18** may be inserted first followed by insertion of the second catheter **20** within the lumen of the first catheter **18.** Once both single or multi-lumen balloon catheters have been inserted and after the endoscope **12** has been advanced through the esophagus **ES** and into proximity to the tissue treatment region, the distal balloon **24** may be advanced to define the distal end of the treatment area and inflated (e.g., with chilled, room or body temperature fluid) while under visualization through the endoscope **12,** as shown in Figure 6A. The endoscope **12** may then be pulled back until the proximal end of the desired treatment area has been identified and the proximal balloon **22** may be slid over the shaft of the second catheter **20** and inflated (e.g., with chilled, room or body temperature fluid) at a site just proximal to the most proximal portion of the lesion, as shown in Figure 6B.

With the treatment area **40** now enclosed by these balloons, an optional pressure capsule **42** (e.g., solid state, piezoeletric or other pressure sensing method) may be inflated and the treatment may proceed, as shown in Figure 6C. The treatment session then exposes the lumen or body cavity to fluid pressurized to a positive pressure in the range of, e.g., 5-100 cmH2U (although this pressure may be maintained at a level below an inflation pressure of the inflation balloons) at temperatures between, e.g., 50 and 100 degrees Celsius, for a period of, e.g., 1 second to 10 minutes. Additionally and/or alternatively, the treatment area **40** may be lavaged for a period of time with an anesthetic (e.g., lidocaine or bupivicaine) to reduce pain with the procedure prior to the application of thermal energy or other active compounds. Accordingly, ablation may be accomplished at a consistent depth of, e.g., about 0.5 mm, throughout the esophagus **ES.**

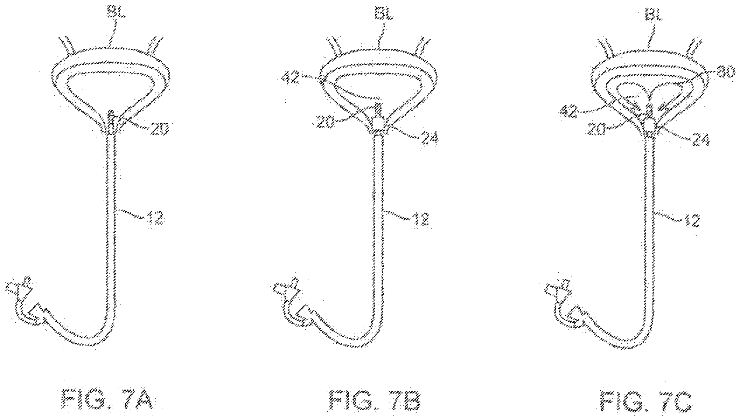

Figures 7A to 7C illustrate another example for treatment of an enclosed body cavity (shown here as a bladder **BL**). In this example, a single balloon may be used to effect infusion and extraction of the treatment fluid. Pressure may be monitored to ensure that the therapy is safe and a relatively lower temperature fluid may be used (e.g., 42-100 C) so that the entire cavity may see a controlled, uniform thermal load. The order or catheter placement may vary as may the sequence for balloon inflation or exposure to active or inactive liquid or vapors in this or any embodiment of the device. As shown in Figure 7A, an endoscope (or cystoscope) **12** may be inserted into the target organ **BL** then fluid catheter **20** may be advanced into the lumen. With the endoscope **12** inserted and occlusion balloon inflated **24** (e.g., with unheated fluid) to seal the organ, a pressure sensor **42** may also be optionally inflated to measure pressure, as shown in Figure 7B. Optionally, an anesthetic or pre-treatment medication may be delivered into the bladder **BL,** if so desired. Then, a high or low temperature fluid **80** may be circulated within the bladder **BL** under pressure adequate to safely distend the organ to ensure complete treatment, as shown in Figure 7C.

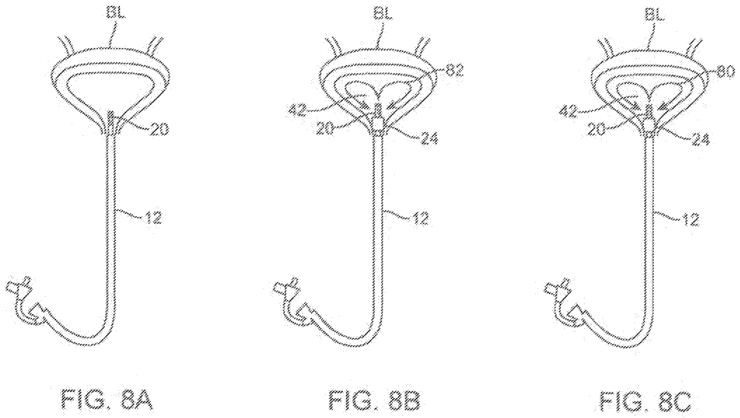

Figures 8A to 8C illustrate another example for treatment where the use a fluid lavage to prepare the treatment area (here shown as the bladder **BL**) may be accomplished prior to application of thermal (or cooling) energy and/or active compounds. As previously described, the endoscope **12** and catheter **20** may be introduced into the bladder **BL** and subsequently sealed with the occlusion balloon **24,** as shown in Figures 8A and 8B. Preparation of the treatment area may involve use of an anesthetic to decrease pain during therapy or the use of an arterial constrictor to reduce blood flow to the organ or lumen. Alternatively, other pre-treatment fluids **82** may include, e.g., anesthetic, vascular constrictor, chilled fluid, active component antidote, etc. The pre-treatment fluid **82** may be evacuated (or left within the bladder **BL**) and the lavage with the treatment fluid **80** may be introduced into the bladder **BL** for treatment, as shown in Figure 8C.

Alternatively, the pre-treatment fluid **82** may also be chilled (or heated) to cool (or warm) the lumen or organ prior to treatment so that the thermal (or cooling) energy may be applied to the internal surface of the lumen or body cavity with minimal transmission or conduction of the elevated (or cooling) temperatures to the submucosal tissues (or tissues lining the body organ or lumen). Utilizing the pre-treatment of the area may avoid damage to the underlying tissues to thereby avoid many of the complications of therapy. For example, strictures and/or stenosis (or tightening) of the tissue can be avoided by controlling the depth of penetration which may be controlled by pre-treating the area with a chilled fluid so that the submucosa can absorb significant amounts of heat without reaching damaging temperatures.

The depth of penetration may also be controlled through the use of a lower temperature fluid for thermal ablation so that the submucosa can cool itself with its robust vascular circulation (which is less robust in the mucosa and epithelium). In the event that an active compound is used, as well, an antidote to this compound may be delivered to the patient (either systemically or as a local pre-treatment) so that the underlying tissues and submucosa are not damaged. One example of this is the use of powerful antioxidants (systemically or locally) prior to lavage of the esophagus with, e.g., methotrexate. The methotrexate may have a powerful effect on the tissues to which it is directly exposed in the lumen or body cavity, but the anti-oxidants may prevent deeper penetration of the methotrexate. The neutralizing compound may also be placed within the balloon or in the lumen of surrounding lumens or body cavities to prevent exposure of these areas in the event of balloon rupture.

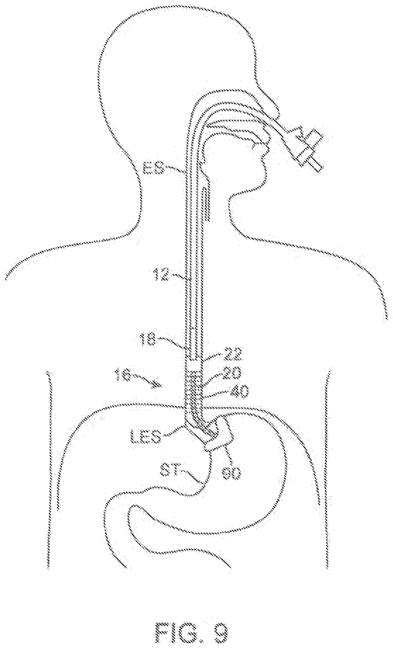

Figure 9 shows another example where the distal occlusion member may be configured into an umbrella-like element **90** which may be expanded in the stomach **ST** and placed over a tissue region which is typically difficult to occlude by a balloon. For instance, such a shape may allow for ablation of the lower esophageal sphincter **LES** at the gastroesophageal junction (or other sphincter region if used elsewhere). The expandable, umbrella-like structure **90** may form a firm seal at this site while allowing the ablation fluid (hot or cold) to contact the entire gastroesophageal junction. Once expanded, the umbrella-like element **90** maybe held firmly against the stomach **ST** by traction on the endoscope **12** or by a tensioning element on the catheter and balloon itself.

In addition, this element **90** may optionally incorporate a biased or springloaded element or other external force mechanism to provide steady pressure and a firm seal against the stomach lining. Alternative structures may also incorporate a more complex, nitinol cage (or other rigid material) connected by a thin, water-tight film. For example, nitinol may be used to decrease the overall profile of the obstruction element and increase its strength and durability.

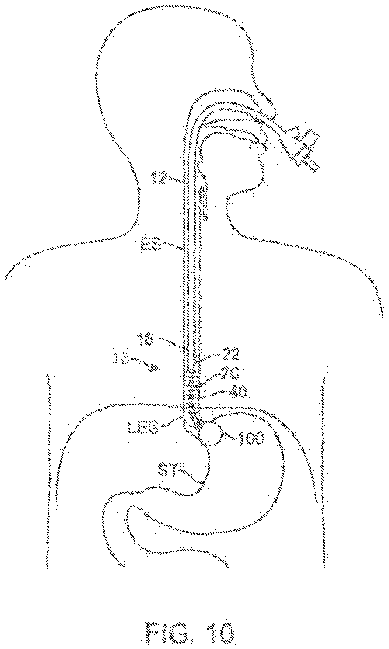

Figure 10 shows another example which utilizes an endoscopic balloon sheath utilized as a distal occluder allowing for exposure and treatment of the distal gastroesophageal junction. In this embodiment, the second catheter **20** may have a distal occlusion balloon **100** which may be passed through the working channel of the endoscope **12** or through a channel incorporated into the balloon sheath itself (outside of the actual endoscope). Once expanded into an enlarged shape, the balloon **100** may be retracted to fit entirely over the lower esophageal junction **LES** to form the distal seal by traction on the endoscope **12** or by a tensioning element on the catheter and balloon itself. This gastric occlusion balloon may allow for exposure of the gastroesophageal junction while preventing fluid flow into the stomach **ST.** The balloon **22** may be configured to be saddle-shaped, circular, wedge-shaped, etc. It may also be self-expanding and noninflatable.

Additionally, the proximal balloon **22** may be configured to be part of sheath that is placed over the tip of the endoscope **12** or it may be formed directly upon the endoscope tip itself. An inflation lumen may run inside the endoscope **12** or it may run alongside the endoscope **12** in a sheath or catheter. The balloon sheath may also incorporate a temperature sensor, pressure sensor, etc. Moreover, the proximal occlusion balloon **22** may optionally incorporate a temperature or pressure sensing element for the therapy and it may be positioned either through the working channel(s) of the endoscope **12** or alongside the endoscope **12** within the endoscopic balloon sheath.

In yet another embodiment, in order to reduce the risks associated with fluid flow and lavage, a fluid or gel may be infused into the esophagus between the balloons then heated or frozen in situ in order to provide the desired ablative effect without circulating any fluid or gel. In one example of this configuration, a gel may be infused into the esophagus and pressurized to a safe level (e.g., 30-100 mmHg) which may be then rapidly chilled using, for example, a compressed gas and/or a Peltier junction-type cooling element. The gel may freeze at a temperature below that of water and allow for rapid transmission of the ablative temperature to the tissues being treated. This gel may also be a liquid with a freezing point below that of water in which case the treatment zone may be lavaged with this fluid prior to treatment to remove free water and prevent crystal formation during therapy. Once the therapy has been completed, the gel or liquid may be removed or left in the esophagus to be passed into the stomach. In the event that a Peltier cooling or heating element is used, the polarity may be reversed once therapy is complete in order to reverse the temperature and terminate the ablation session.

The distance from the lower end of the distal most portion of the catheter can be on the order of about 150mm. The distance between the proximal and distal balloons are adjustable by the operator but can be adjusted, e.g., from as small as 0mm to as large as 25cm. The treatment zone may have a range of, e.g., 3 to 15cm.

In yet an additional embodiment, an energy generator (e.g., a RF electrode or hot wire or other energy source) may be advanced into the treatment area in a protective sheath (to prevent direct contact with body tissues) and energy may be applied to the treatment fluid to heat it to the desired temperature. Once the fluid is adequately heated and enough time has passed to achieve a controlled ablation, the fluid may then be evacuated or neutralized with the influx of colder fluid. This embodiment would allow for a very low-profile design and would not require any fluid heating element outside of the body.

In another variation, the cavity or lumen may be exposed to the hot water at a temperature of less than, e.g., 100 degrees Celsius, but greater than, e.g., 42 degrees Celsius, to allow for easier control of the treatment due a longer treatment period. Ranges for optimal hyperthermic treatment include temperatures between, e.g., 42 and 100 C and exposure periods ranging from, e.g., 15 seconds to 15 minutes. In this embodiment, treatment may be effected with an active (e.g., Methotrexate) or inactive fluid at a temperature of, e.g., 90 degrees C, for a period of e.g., 5-60 seconds, depending on the depth of penetration desired.

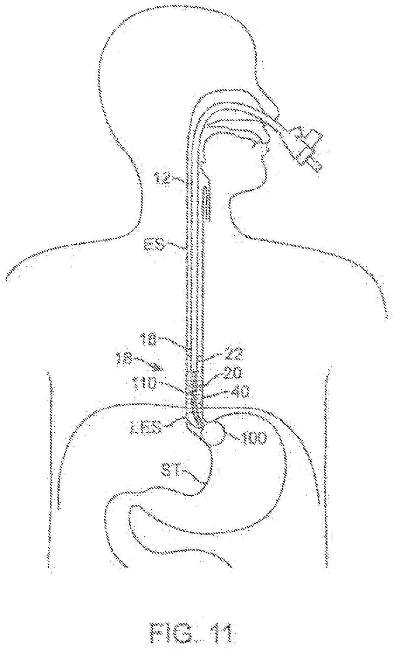

Figure 11 shows another example of an endoscopic balloon sheath which may be used to provide proximal occlusion of the treatment area **40** and may house one or more of the temperature and pressure sensors. This variation may incorporate a stirring/agitating or recirculation mechanism **110** incorporated into the device which may actuated within the treatment area **40** once the treatment fluid has been introduced to allow for even cooling/heating. The distal occlusion balloon **100** may be inflated within the stomach **ST** and pulled proximally with controlled traction against the gastric portion of the lower esophageal sphincter **LES,** as previously described.

in this example, a chilled liquid lavage (or vapor infusion) may then be initiated and the tissue ablated via freezing. A pre-treatment lavage, e.g., a hypertonic, hyperosmotic saline solution, may be introduced with above freezing temperatures followed by a sub-zero temperature lavage to ablate the tissues within the treatment area **40.** The hypertonic, hyperosmotic fluid may achieve temperatures down to, e.g., -40 degrees C, without creating ice crystals in the treatment area **40** due to the pre-treatment lavage removing any free water. The treatment fluid following the pre-treatment lavage may have temperatures of, e.g., -2 degrees C to -40 degrees C, for ablation or more particularly a temperature range of, e.g., -5 degrees C to -20 degrees C. This temperature range may allow for freezing and crystal formation in the exposed tissues without damaging the underlying submucosa (which is protected by the circulation of body temperature blood that prevents freezing). This temperature range can also be easily achieved with hypersalination of aqueous fluid using sodium chloride and may inhibit any undesired damage to tissues with brief contact. Also, the use of a heavily salinated or other sub-zero solution lavage may provide optimal sealing of the occluding balloons in that any sub-zero temperatures outside of the pre-lavaged treatment zone may form an impaction of ice crystals and prevent any further fluid flow outside of the treatment zone. This hypersalinated water solution is but one freezing solution, though, and any aqueous or non-aqueous liquid or vapor that can be infused and extracted at this temperature could be used. Alternatively, cryoablative fluid can simply comprise nitrous oxide (N₂O) or be formed by cooling ethanol or another aqueous or lipophilic fluid with subzero cooling temps with compressed gas or dry ice. In another alternative, compressed CO₂ or dry ice may be introduced into the fluid (e.g., ethanol, butylenes glycol, propylene glycol, etc) to cool it to, e.g., -50 degrees C or below.

Despite the potential for toxicity, ethanol may be used for a liquid lavage since ethanol resists freezing down to -118 C and is relatively biocompatible although ethanol is dose dependent for toxicity. A liquid lavage with about 75% to 99.9% ethanol concentrations may be utilized to good effect and have been demonstrated to show that a freeze layer develops very rapidly which also inhibits further ethanol absorption. For instance, a concentration of 95% ethanol may be introduced at a temperature of about, e.g., -80 to -50 degrees C, for a treatment time of about, e.g., 5 minutes, utilizing 0.25 to 0.5 liters of the cryogenic fluid. An ethanol copper composition may also be very useful since ethanol resists freezing whereas aqueous fluids will freeze and expand thereby moving the metal particle out of direct contact with the tissue.

In the event that nitrous oxide is used as the cryogenic fluid, the nitrous may be introduced through a nozzle or spray at a pressure of, e.g., 4136-5516 kPa (600-800 psi), at a temperature of about -88 degrees C. Such a temperature and pressure may be utilized for a treatment time of about, e.g., 3 minutes.

The use of a subzero solution within this range may also allow for fine control of the treatment depth as tissue damage would not begin to occur until a temperature differential of about 37 degrees C is achieved (assuming a body temperature of 37° C), but once this threshold is reached tissue damage occurs rapidly due to ice crystal formation. In contrast, tissue damage is on a continuous spectrum with hyperthermia and damage may begin to occur at a temperature differential of e.g., 5 degrees C. Thus, the ability of the vasculature to protect the underlying tissues from damage is greatly reduced due to the small difference between the temperature of protective blood versus the temperature of the ablating fluid. With hypothermic lavage, the protective blood may differ by, e.g., 37 degrees C, in temperature and may thus allow for control of ablation depth based on the temperature of the fluid lavage and the time of exposure.

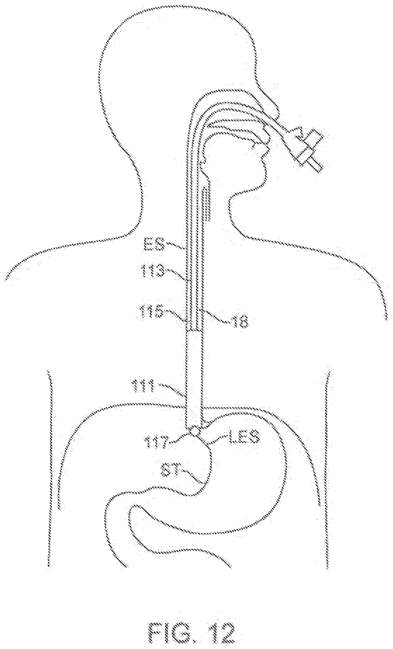

Figure 12 illustrates another variation where a conforming balloon **111** having an adjustable size in diameter as well as in length may be positioned along or near the distal end of the catheter **18.** The conforming balloon **111** may be advanced within the esophagus (shown here in the esophagus but applicable to any cavity) in a collapsed state. Once the balloon **111** has been desirably positioned along the length of the esophagus **ES** to be treated, the catheter **18** may optionally utilize a vacuum which may be drawn along the entire length of the balloon **111** through perforations or openings in the balloon **111** to serve as a safeguard to prevent migration of ablation liquid, gas, and/or conductive material in the event of balloon rupture. The vacuum may also be utilized to remove air, fluids or particulate between the outer wall of the balloon **111** and the tissue to improve contact and thermal transfer from the hyperthemic or cryogenic fluid and to the tissue. Additionally and/or alternatively, a distal vacuum may be drawn through a distal port **117** distal to the balloon **111** either alone or in conjunction with a proximal vacuum port **115** proximal to the balloon **115.**

With the catheter **18** and balloon **111** desirably positioned for treatment, an insulating sheath **113** may be advanced over the catheter **18** and over the length of the balloon **111** to vary an inflation length of the balloon **111** emerging from the insulating sheath **113.** The variable length of the inflated balloon **111** may be adjusted to allow for treatment of any varying lengths of the esophagus **ES** during a single ablation treatment. Such a design may prevent dangerous ablation overlap zones of ablated tissue.

The balloon **111** itself may be comprised of a compliant or non-compliant material but in either case be capable of directly contacting the tissues to be ablated. The balloon **111** may accordingly be filled with a hyperthemic or cryogenic material and/or may use liquid, gas, and/or conductive solids, as described herein.

Although illustrated esophageal therapy, this therapy could be used in any body cavity/lumen for therapeutic purposes including, but not limited to, gastrointestinal therapy, stomal tightening (e.g., post bariatric surgery), urogynecologic uses (treatment of cervical pre-cancers or cancers, endometrial lining treatment, stress incontinence therapy), prostate therapy, intravascular therapy (e.g., varicose veins) or treatment of any other body cavity/lumen. In the event that an entire body cavity is being treated (e.g., the entire uterus) a single balloon system may suffice to exclude the entire cavity. The fluid cycling or dwell may then be accomplished with use of a pressure-controlled exposure of the cavity or lumen.

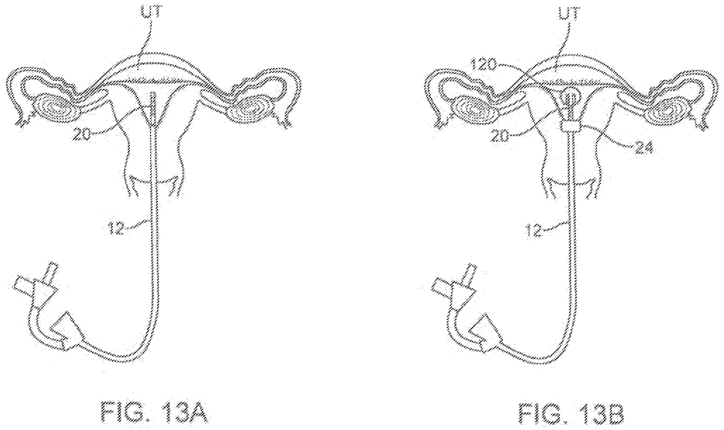

Figures 13A and 13B show another example of how the system may be introduced into, e.g., a uterus **UT,** through the cervix for treatment via the lavage catheter **20.** In this example, the catheter **20** may have a diameter of about, e.g., 8 mm, or in other examples, a diameter of about, e.g., less than 6 mm. Infusion of the lavage fluid may fully distend or partially distend the uterine walls. Optionally, catheter **20** may incorporate a tip **120** to perform one or more functions including, e.g., an expandable cage or scaffold to prevent direct exposure of a cryoprobe to the tissue walls of the uterus **UT,** an agitator or recirculator to ensure even distribution of cryoablation effect, etc. As previously described, the system may be used with lavage or with infusion then cryoprobe chilling of fluid. In an alternate embodiment, infusion of an antifreeze fluid and insertion of the cryprobe may be done separately with chilling of the anti-freeze done after the cryoprobe insertion.

In this and other examples, the therapy may be guided by time/temperature tracking or visualization (e.g., hysteroscope, endoscope, ultrasound, etc.). Pressure may be regulated by a pressure sensor in line with the infusion or extraction lumen or a dedicated pressure lumen in a multi-lumen catheter. Additionally, pressure may also be regulated by limiting infusion pressure (e.g., height of infusion bag, maximum pressure of infusion pump, etc.). Any organ, body cavity or lumen may be treated using the described lavage and/or infusion/cryoprobe technique described here for the uterus.

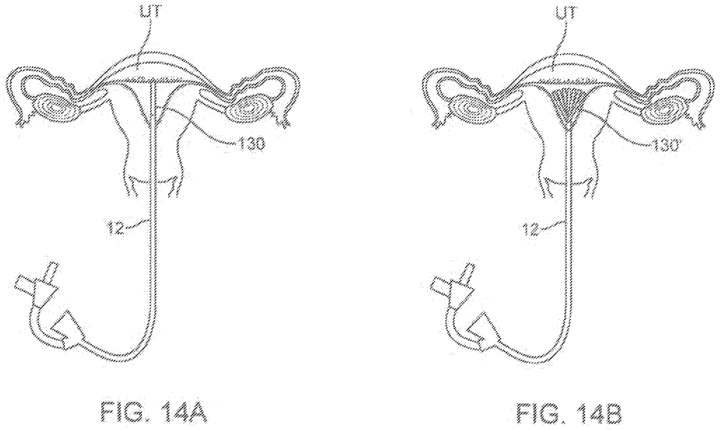

Figures 14A and 14B illustrate another variation of a treatment system which utilizes a thermally conductive array of fibers, cage, or lattice **130** which may be deployed within the uterus **UT.** In this variation, the endoscope **12** may be advanced through the cervix and at least partially into the uterus **UT** where the array of fibers or lattice **130** may be deployed from the endoscope **12** distal end where the array **130** may be positioned in a compressed state for delivery, as shown in Figure 14A. The array **130** may be advanced into the uterus **UT** where it may then be expanded into a deployed configuration **130',** as shown in Figure 14B. The individual cryogenic probes of the expanded array **130'** may be in fanned out relative to the distal end of the endoscope **12** in various directions to come into direct contact or close proximity to the tissue to be treated.

Following deployment, the deployed array **130'** may be cooled rapidly to transmit the heat within the uterine walls to the array **130'** to provide a consistent cryoablative effect throughout the body cavity or lumen. The members of the array **130'** may be cooled either via conductive cooling or by an infusion of a cooling fluid (as described herein) through the members of the array **130'.** Similar to the conductive fluid, the cooled array **130'** may provide for the consistent ablation of the entire lumen with a single application of the array **130'.** The individual members of the array **130'**

Additionally and/or alternatively, the array **130'** may be used in conjunction with a fluid infusion and/or lavage in order to optimize therapy. One or more sizes and shapes of the array **130'** may be available depending on the size and shape of the cavity to be treated. Moreover, the array **130'** may be formed from any material so long as it has a thermal conductivity greater than, e.g., 2 W/m-K, such as a metal with a relatively high thermal conductivity.

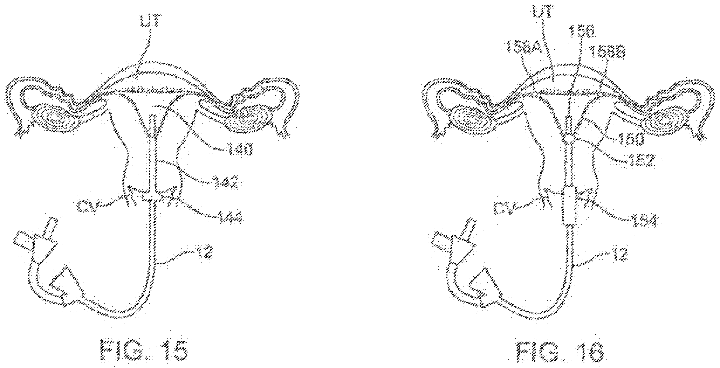

Figure 15 shows another variation of a device which may utilize cryogenic lavage treatment within the uterus **UT.** In this example, the distal end of the endoscope **12** may be advanced through the cervix **CV** and into the uterus **UT** where a cryoprobe **140** may be deployed, as shown. One or more inflatable balloons **144** may be expanded, e.g., within the external os, or a balloon **142** along the outer surface of the endoscope **12** may be inflated within the length of the os itself. Alternatively, a single balloon (e.g., having an hourglass or dumbbell shape) may be inflated to block both the external os and the length of the os itself. With the uterus **UT** obstructed, the cryogenic treatment or lavage may be performed within the uterine lumen.

Another variation is illustrated in Figure 16 which shows endoscope **12** advanced through the cervix **CV** with the distal end **156** positioned within the uterine lumen. An optional balloon **152** located near the endoscope distal end may be inflated within the uterus **UT** and then pulled proximally against the internal os with a fixed amount of tension to obstruct the opening. Additionally and/or alternatively, a proximal balloon **154** positioned along the endoscope **12** proximally of where the cervix **CV** is located may also inflated to further provide for obstruction of the entire os. Then external cervical engagement portion, e.g., proximal balloon **154,** may be fixed in place relative portion of the endoscope **12** spanning the cervical os to provide consistent tension. The proximal balloon **154** may also have a spring-type function to provide for consistent tension regardless of tissue relaxation and accommodation.

With the uterus **UT** obstructed, the endoscope **12** may then be used to provide for the cryogenic treatment or lavage. Optionally, the endoscope **12** may also incorporate one or more vacuum ports along the length of the shaft to seal and provide a safeguard against fluid flow out of the uterus **UT.**

Optionally, the uterine cornu may be temporarily obstructed to block the openings of one or both Fallopian tubes prior to the cryogenic treatment. The occlusive element(s) **158A, 158B** may comprise, e.g., balloons, inserts, energy-based ablation to contract the aperture, hydrophilic or hydrophobic gel-based solutions, or any other modality that is capable of reversibly or irreversibly sealing the Fallopian tube. The optional Fallopian tube occlusion may be temporary or permanent (if sterility is desired).

Once the cryogenic procedure has been completed, the occlusive elements **158A, 158B** may be removed or allowed to passively erode. Alternatively, they may be left occluded for those desiring sterility. Occluding the uterine cornu prior to a lavage may allow for greater fluid pressure and fluid flow within the uterus **UT.**

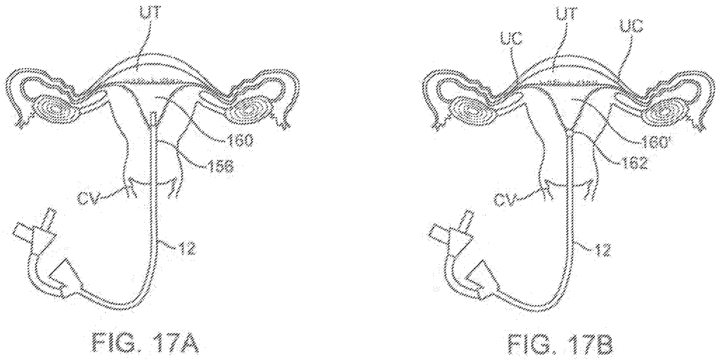

Figures 17A and 17B illustrate another variation of a low-pressure conforming balloon. In this variation, a conforming balloon **160** may be deployed from the distal end **156** of the endoscope **12** and then inflated with the cryogenic liquid/gas (as described herein) while in uterus **UT.** The balloon **160** may be formed to resist rupture at low and high temperatures and may be further configured to conform well to the anatomy of the uterus **UT.** For example, the balloon **160** when inflated may have a shape which approximates the lumen in which it is inflated and/or come in various sizes to accommodate different patient anatomies. In the present example, the expanded balloon **160'** may be formed to taper and have two portions rounded portions for expanding into intimate contact at the uterine cornu **UC,** as shown, without painful deformation or distention of the uterus **UT** at a pressure, e.g., less than 150 mmHg.

Moreover, the expanded balloon **160'** may have a wall which is relatively thin (e.g., 1.016 mm (0.040 in.) or less) to facilitate thermal conduction through the balloon. The balloon **160** may also be sufficiently thin such that folding of the balloon **160** on itself does not create a significant thermal barrier allowing for an even ablation in the event that a non-compliant balloon is used. For treatment, the expanded balloon **160'** may be filled with the cryogenic liquid, gas or a thermally conductive compound (as described above) to subject the contacted tissue to either cryogenic and/or hyperthermic injury (e.g., steam, plasma, microwave, RF, hot water, etc). Additionally and/or alternatively, the balloon **160'** may also be used to transmit photodynamic therapy light to the uterus **UT** or esophagus **ES.** This modality may be used to achieve ablation of any body cavity or lumen.

Additionally, one or more vacuum ports may be used anywhere along the length of the shaft to seal and provide a safeguard against fluid flow out of the uterus **UT** in the event of balloon rupture. Additionally, one or more inflatable os balloon **160** may also be used to block the internal or external os, as also described above.

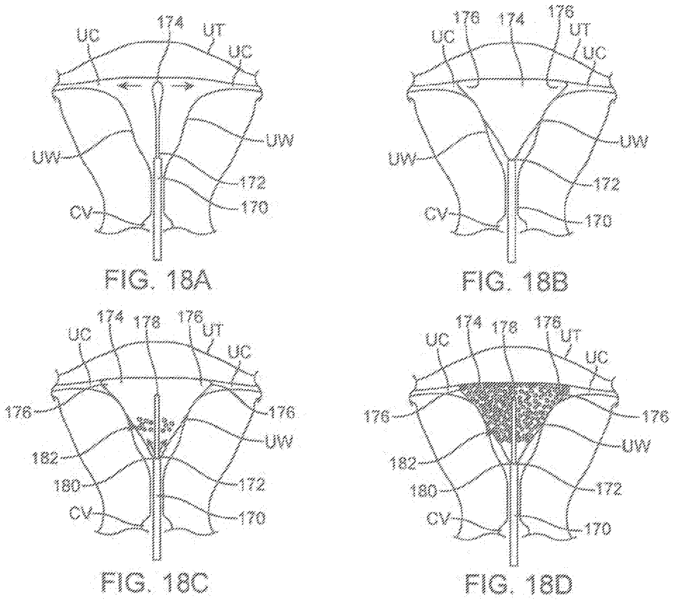

In another variation, to facilitate the balloon expanding and conforming readily against the tissue walls of the uterus **UT,** the balloon may be inflated with a gas or liquid. Alternatively, as shown in Figures 18A to 18D, the balloon may be filled partially or completely with a conductive material. As shown in Figure 18A, once the elongate shaft **170** has been introduced through the cervix **CV** and into the uterus **UT,** the distal opening **172** of the shaft **170** may be positioned distal to the internal os and balloon **174** may be deployed either from within the shaft **170** or from an external sheath (described below in further detail). The balloon may be deployed and allowed to unfurl or unwrap within the uterus **UT,** as shown in Figure 18B. The cooling probe **178** may be introduced through the shaft **172** and into the balloon interior (or introduced after insertion of the conductive elements).

Because the balloon **174** is used to contact the tissue and thermally conduct the heat through the balloon, the balloon material may be comprised of various materials such as polyurethane, fluorinated ethylene propylene (FEP), polyether ether ketone (PEEK), low density polyethylene, polyethylene terephthalate (PET), polyvinylidene fluoride (PVDF), or any number of other conformable polymers. Moreover, the balloon material may have a thickness which remains flexible and strong yet sufficiently thermally conductive, e.g., about 0.0127 to 0.381 mm (0.0005 to 0.015 in.). Such a thickness may allow for the balloon to remain supple enough to conform desirably to the underlying tissue anatomy and may also provide sufficient clarity for visualizing through the material with, e.g., a hysteroscope.

The conductive elements **182** may be introduced into the balloon interior through an annular opening **180** within the distal end **172** of the shaft, as shown in Figure 18C, until the balloon **174** is at least partially or completely filled with the elements **182.** The conductive elements **182** may generally comprise any number of thermally conductive elements such as copper spheres or some other inert metal such as gold. These conductive elements **182** may be atraumatic in shape and are small enough to fill the balloon interior and conform the balloon walls against the uterine walls **UW** to ensure consistent contact with the tissue, e.g., about 20 ml in volume of the elements **182.** The conductive elements **182** may also help to fill any air pockets which may form particularly near the tapered portions **176** of the balloon and insulate the tissue from the ablative effects of the cryoablative fluid. For instance, the conductive elements **182** may be formed into spheres having a diameter of, e.g., 0.8 mm to 4 mm or larger. To ensure that conductive elements **182** are fully and evenly dispersed throughout the balloon interior, the elements **182** may be introduced through the shaft **170** via an ejector or push rod, auger, compressed air, etc. In particular, the conductive elements **182** may fill the tapered portions **176** of the balloon **174** to ensure that the balloon is positioned proximate to and in contact with the uterine cornu **UC** to fully treat the interior of the uterus **UT,** as shown in Figure 18D.

With the conductive elements **182** placed within the balloon **174,** the cryoablative fluid may be introduced within and through the balloon **174** such that the conductive elements **182** facilitate the thermal transfer from the contacted uterine walls **UW.** Once the cryoablative treatment has been completed, the conductive elements **182** may be removed through the shaft **170** via a vacuum force or other mechanical or electromechanical mechanisms and the balloon **174,** once emptied, may also be withdrawn from the uterus **UT.**

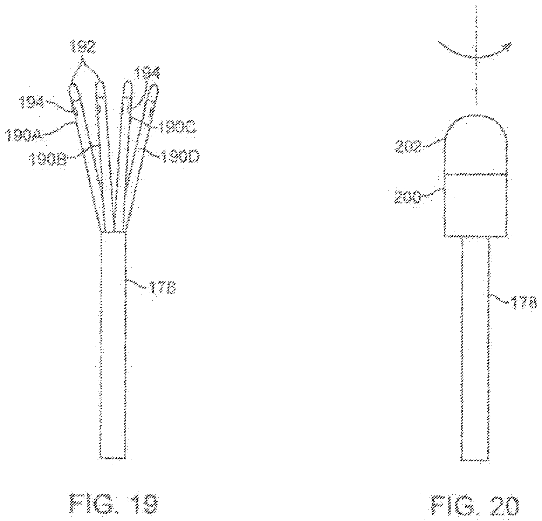

The cooling probe **178** introduced into the interior of the balloon **174** may comprise a number of different configurations which facilitate the introduction of the cryoablative fluid into the balloon **174.** One such variation, similar to the variation shown above in Figure 14B, is illustrated in the detail view of Figure 19. In this variation, the shaft **178** may have one or more cooling members **190A, 190B, 190C, 190D** which project from the distal end of the shaft **178** at various angles. Although illustrated with four cooling members extending from the shaft **178,** any number of cooling members may be used at a variety of different angles and lengths as desired. Moreover, the cooling members may be fabricated from a number of materials, e.g., polyimide, Nitinol, etc., which are sufficiently strong and temperature resistant for the relatively low temperature of the fluid. Each of the cooling members **190A, 190B, 190C, 190D** in this example may each have an occluded tip **192** and at least one opening **194** defined along the side of the cooling member. The cryoablative fluid may be flowed through the shaft **178** and into each cooling member where the fluid may then be sprayed or ejected through the respective openings **194** for distribution throughout the interior of the balloon for cooling the contacted uterine tissue.

Another variation of the cooling probe is illustrated in the detail view of Figure 20 which shows elongate shaft **178** having a rotating base **200** and spray member **202** positioned upon shaft **178.** The spray member **202** may have a surface which is meshed, latticed, perforated, etc. such that the cryoablative fluid introduced through the shaft **178** may enter the rotating base **200** and spray member **202** where it may be evenly dispersed through the spray member **202** and into the interior of the balloon **174** for treatment. The pressure of the fluid may rotate the base **200** about its longitudinal axis, as shown, to further facilitate the distribution of the cryoablative fluid within the balloon **174.**

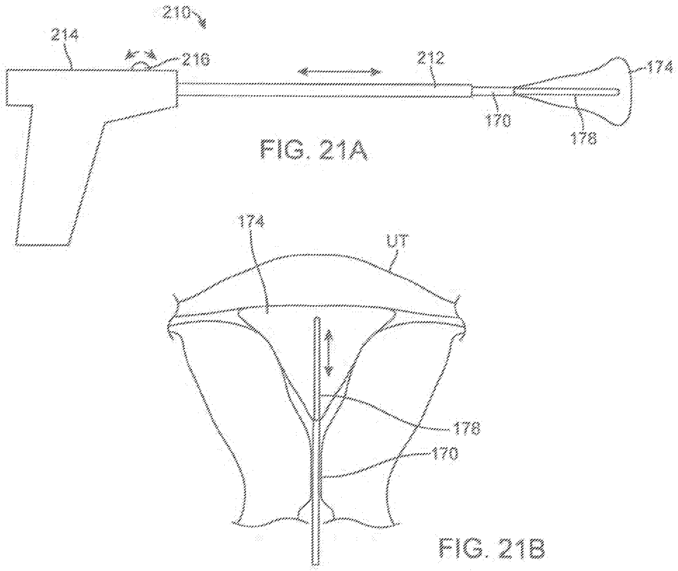

The cooling probe **178** as well as the balloon assembly may be variously configured, for instance, in an integrated treatment assembly **210** as shown in the side view of Figure 21A. In this variation, the assembly **210** may integrated the elongate shaft **170** having the balloon **174** extending therefrom with the cooling probe **178** positioned translatably within the shaft **170** and balloon **174.** A separate translatable sheath **212** may be positioned over the elongate shaft **170** and both the elongate shaft **170** and sheath **212** may be attached to a handle assembly **214.** The handle assembly **214** may further comprise an actuator **216** for controlling a translation of the sheath **212** for balloon **174** delivery and deployment. The sheath **212** may be configured to have a diameter of e.g., 5.5 mm or less, to prevent the need for dilating the cervix.

With the sheath **212** positioned over the elongate shaft **170** and balloon **174,** the assembly **210** may be advanced through the cervix and into the uterus **UT** where the sheath **212** may be retracted via the handle assembly **214** to deploy the balloon **174,** as shown in Figure 21B. As described above, once the balloon **174** is initially deployed from the sheath **212,** it may be expanded by an initial burst of a gas, e.g., air, carbon dioxide, etc., or by the cryogenic fluid. In particular, the tapered portions of the balloon **174** may be expanded to ensure contact with the uterine cornu. The handle assembly **214** may also be used to actuate and control a longitudinal position of the cooling probe **178** relative to the elongate shaft **170** and balloon **174** as indicated by the arrows.

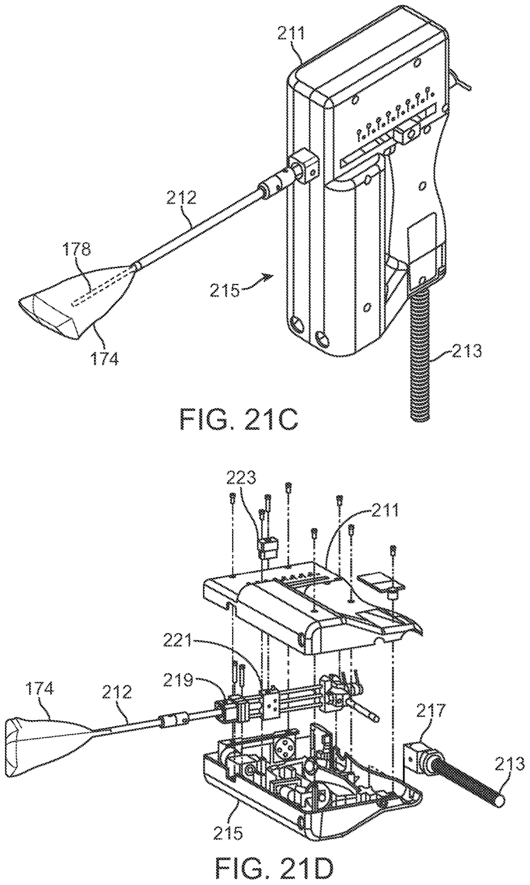

In another variation of the treatment assembly, Figure 21C shows a perspective view of a cryoablation assembly having a handle assembly **211** which may integrate the electronics and pump assembly **215** within the handle itself. An exhaust tube **213** may also be seen attached to the handle assembly **211** for evacuating exhausted or excess cryoablative fluid or gas from the liner **174.** Any of the cryogenic fluids or gases described herein may be utilized, e.g., compressed liquid-to-gas phase change of a compressed gas such as nitrous oxide (N₂O), carbon dioxide (CO₂), Argon, etc. The cooling probe **178** may be seen extending from sheath **212** while surrounded or enclosed by the balloon or liner **174.** Hence, the handle assembly **211** with coupled cooling probe **178** and liner **174** may provide for a single device which may provide for pre-treatment puff-up or inflation of the liner **174,** active cryoablation treatment, and/or post-treatment thaw cycles.

The handle assembly **211** may also optionally incorporate a display for providing any number of indicators and/or alerts to the user. For instance, an LCD display may be provided on the handle assembly **211** (or to a separate control unit connected to the handle assembly **211**) where the display counts down the treatment time in seconds as the ablation is occurring. The display may also be used to provide measured pressure or temperature readings as well as any number of other indicators, symbols, or text, etc., for alerts, instructions, or other indications. Moreover, the display may be configured to have multiple color-coded outputs, e.g., green, yellow, and red. When the assembly is working through the ideal use case, the LED may be displayed as a solid green color. When the device requires user input (e.g. when paused and needing the user to press the button to re-start treatment) the LED may flash or display yellow. Additionally, when the device has faulted and treatment is stopped, the LED may flash or display a solid red color.

Figure 21D shows the handle assembly **211** in a perspective exploded view to illustrate some of the components which may be integrated within the handle **211.** As shown, the liner **174** and sheath **212** may be coupled to a sheath bearing assembly **219** and slider base block assembly **221** for controlling the amount of exposed treatment length along the cooling probe **178** (and as described in further detail below). An actuatable sheath control **223** may be attached to the slider base block assembly **221** for manually controlling the treatment length of the cooling probe **178** as well. Along with the electronics and pump assembly **215** (which may optionally incorporate a programmable processor or controller in electrical communication with any of the mechanisms within the handle **211**), an exhaust valve **217** (e.g., actuated via a solenoid) may be coupled to the exhaust line **213** for controlling not only the outflow of the exhausted cryoablation fluid or gas but also for creating or increasing a backpressure during treatment, as described in further detail below.

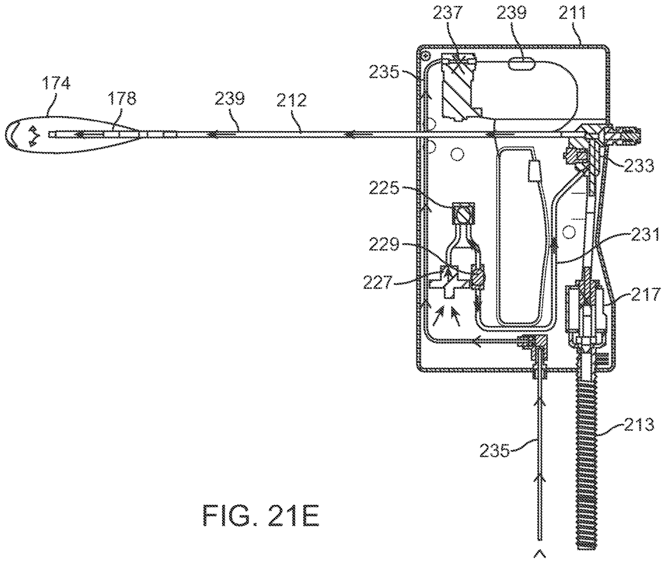

In one example of how the handle assembly **211** may provide for treatment, Figures 21E to 21G illustrate schematic side views of how the components may be integrated and utilized with one another. As described herein, once the sheath **212** and/or liner **174** has been advanced and initially introduced into the uterus, the liner **174** may be expanded or inflated in a pre-treatment puff up to expand the liner **174** into contact against the uterine tissue surfaces in preparation for a cryoablation treatment. As illustrated in the side view of Figure 21E, a pump **225** integrated within the handle assembly **211** may be actuated and a valve **229** (e.g., actuatable or passive) fluidly coupled to the pump **225** may be opened (as indicated schematically by an "O" over both the pump **225** and valve **229**) such that ambient air may be drawn in through, e.g., an air filter **227** integrated along the handle **211,** and passed through an air line **231** within the handle and to an exhaust block **233.** The exhaust block **233** and air line **231** may be fluidly coupled to the tubular exhaust channel which extends from the handle **211** which is further attached to the cooling probe **178.** As the air is introduced into the interior of the liner **174** (indicated by the arrows), the liner **174** may be expanded into contact against the surrounding uterine tissue surface.

A cryogenic fluid line **235** also extending into and integrated within the handle assembly**211** may be fluidly coupled to an actuatable valve **237,** e.g., actuated via a solenoid, which may be manually closed or automatically closed (as indicated schematically by an "X" over the valve **237**) by a controller to prevent the introduction of the cryoablative fluid or gas into the liner **174** during the pre-treatment liner expansion. An infusion line **239** may be fluidly coupled to the valve **237** and may also be coupled along the length of the sheath **212** and probe **178,** as described in further detail below. The exhaust valve **217** coupled to the exhaust line **213** may also be closed (as indicated schematically by an "X" over the valve **217**) manually or automatically by the controller to prevent the escape of the air from the exhaust block **233.**

During this initial liner expansion, the liner **174** may be expanded in a gradual and controlled manner to minimize any pain which may be experienced by the patient in opening the uterine cavity. Hence, the liner **174** may be expanded gradually by metering in small amounts of air. Optionally, the pump **225** may be programmed and controlled by a processor or microcontroller to expand the liner **174** according to an algorithm (e.g., e.g. ramp-up pressure quickly to 10 mm Hg and then slow-down the ramp-up as the pressure increases to 85 mm Hg) which may be stopped or paused by the user. Moreover, the liner **174** may be expanded to a volume which is just sufficient to take up space within the uterine cavity. After the initial increase in pressure, the pressure within the liner **174** may be optionally increased in bursts or pulses. Moreover, visualization (e.g., via a hysteroscope or abdominal ultrasound) may be optionally used during the controlled gradual expansion to determine when the uterine cavity is fully open and requires no further pressurization. In yet another variation, the liner **174** may be cyclically inflated and deflated to fully expand the liner. The inflations and deflations may be partial or full depending upon the desired expansion.

In yet another alternative variation, the system could also use an amount of air pumped into the liner **174** as a mechanism for detecting whether the device is in a false passage of the body rather than the uterine cavity to be treated. The system could use the amount of time that the pump **225** is on to track how much air has been pushed into the liner **174.** If the pump **225** fails to reach certain pressure levels within a predetermined period of time, then the controller may indicate that the device is positioned within a false passage. There could also be a limit to the amount of air allowed to be pushed into the liner **174** as a way to detect whether the probe **178** has been pushed, e.g., out into the peritoneal cavity. If too much air is pushed into the liner **174** (e.g., the volume of air tracked by the controller exceeds a predetermined level) before reaching certain pressures, then the controller may indicate the presence of a leak or that the liner **174** is not fully constrained by the uterine cavity. The liner **174** may also incorporate a release feature which is configured to rupture if the liner **174** is not constrained such that if the system attempts to pump up the liner **174** to treatment pressure (e.g., 85 mmHg), the release feature will rupture before reaching that pressure.

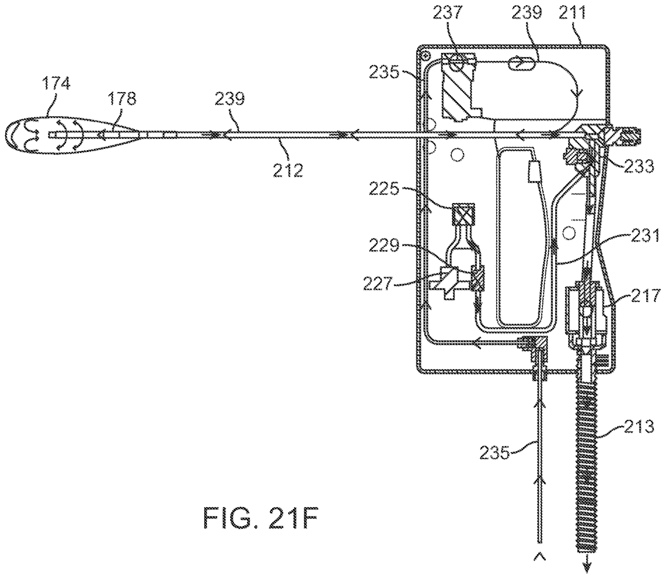

Once the liner **174** has been expanded sufficiently into contact against the uterine tissue surface, the cryoablation treatment may be initiated. As shown in the side view of Figure 21F, the air pump **225** may be turned off and the valve **229** may be closed (as indicated schematically by an "X" over the pump **225** and valve **229**) to prevent any further infusion of air into the liner **174.** With the cryogenic fluid or gas pressurized within the line **235,** valve **237** may be opened (as indicated schematically by an "O" over the valve **237**) to allow for the flow of the cryogenic fluid or gas to flow through the infusion line **239** coupled to the valve **237.** Infusion line **239** may be routed through or along the sheath **212** and along the probe **178** where it may introduce the cryogenic fluid or gas within the interior of liner **174** for infusion against the liner **174** contacted against the surrounding tissue surface.

During treatment or afterwards, the exhaust valve **217** may also be opened (as indicated schematically by an "O" over the valve **217**) to allow for the discharged fluid or gas to exit or be drawn from the liner interior and proximally through the cooling probe **178,** such as through the distal tip opening. The fluid or gas may exit from the liner **174** due to a pressure differential between the liner interior and the exhaust exit and/or the fluid or gas may be actively drawn out from the liner interior, as described in further detail herein. The spent fluid or gas may then be withdrawn proximally through the probe **178** and through the lumen surrounded by the sheath **212,** exhaust block **233,** and the exhaust tube **213** where the spent fluid or gas may be vented. With the treatment fluid or gas thus introduced through infusion line **239** within the liner **174** and then withdrawn, the cryogenic treatment may be applied uninterrupted.

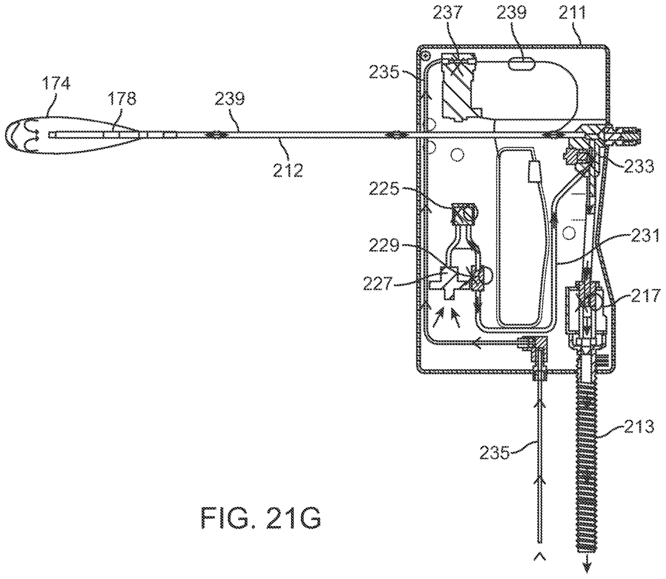

Once a treatment has been completed, the tissue of the uterine cavity may be permitted to thaw. During this process, the cryogenic fluid delivery is halted through the infusion line **239** by closing the valve **237** (as indicated schematically by an "X" over the valve **237**) while continuing to exhaust for any remaining cryogenic fluid or gas remaining within the liner **174** through probe **178,** through the lumen surrounded by sheath **212,** and exhaust line **213,** as shown in Figure 21G. Optionally, the pump **225** and valve **229** may be cycled on and off and the exhaust valve **217** may also be cycled on and off to push ambient air into the liner **174** to facilitate the thawing of the liner **174** to the uterine cavity. Optionally, warmed air or fluid (e.g., saline) may also be pumped into the liner **174** to further facilitate thawing of the tissue region.

As the spent cryogenic fluid or gas is removed from the liner **174,** a drip prevention system may be optionally incorporated into the handle. For instance, a passive system incorporating a vented trap may be integrated into the handle which allows exhaust gas to escape but captures any vented liquid. The exhaust line **213** may be elongated to allow for any vented liquid to evaporate or the exhaust line **213** may be convoluted to increase the surface area of the exhaust gas tube to promote evaporation.

Alternatively, an active system may be integrated into the handle or coupled to the handle **211** where a heat sink may be connected to a temperature sensor and electrical circuit which is controlled by a processor or microcontroller. The heat sink may promote heat transfer and causes any liquid exhaust to evaporate. When the temperature of the heat sink reaches the boiling temperature of, e.g., nitrous oxide (around -89 °C), the handle may be configured to slow or stop the delivery of the cryogenic fluid or gas to the uterine cavity.

The pre-treatment infusion of air as well as the methods for treatment and thawing may be utilized with any of the liner, probe, or apparatus variations described herein. Moreover, the pre-treatment, treatment, or post-treatment procedures may be utilized altogether in a single procedure or different aspects of such procedures may be used in varying combinations depending upon the desired results.

Additionally and/or optionally, the handle **211** may incorporate an orientation sensor to facilitate maintaining the handle **211** in a desirable orientation for treatment. One variation may incorporate a ball having a specific weight covering the exhaust line **213** such that when the handle **211** is held in the desirable upright orientation, the treatment may proceed uninterrupted. However, if the handle **211** moved out of its desired orientation, the ball may be configured to roll out of position and trigger a visual and/or auditory alarm to alert the user. In another variation, an electronic gyroscopic sensor may be used to maintain the handle **211** in the desired orientation for treatment.

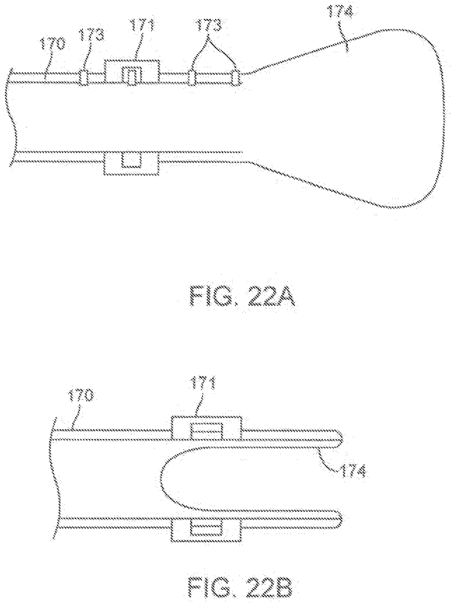

Figure 22A shows an example of one variation of a design of a system which may be used to deploy the balloon **174** into the uterus **UT** after properly setting the depth of the uterine cavity (or some other anatomical measurement). The elongate shaft **170** may have the balloon **174** attached along or near the distal end of the shaft **170** via a clamp or O-ring **171** placed along the outside of the shaft **170.** One or more indicators 173 along the outer surface of the cannula may correspond to clinical measurements of the uterine length which may be measured by the clinician prior to a cryoablative procedure. With the measured uterine cavity known, the balloon **174** may be adjustably clamped along the length of the shaft **170** at any one of the indicators **173** which may correspond to the measured cavity length. With the balloon **174** suitably clamped in place, it may be pushed into the shaft lumen, as shown in Figure 22B, using a pusher or some other instrument for delivery into the uterus **UT.** The elongate shaft **170** and balloon **174** may then be introduced into the uterus **UT** where the balloon **174** may be deployed from the shaft **170** and having a suitable length which may correspond to the particular anatomy of the patient.

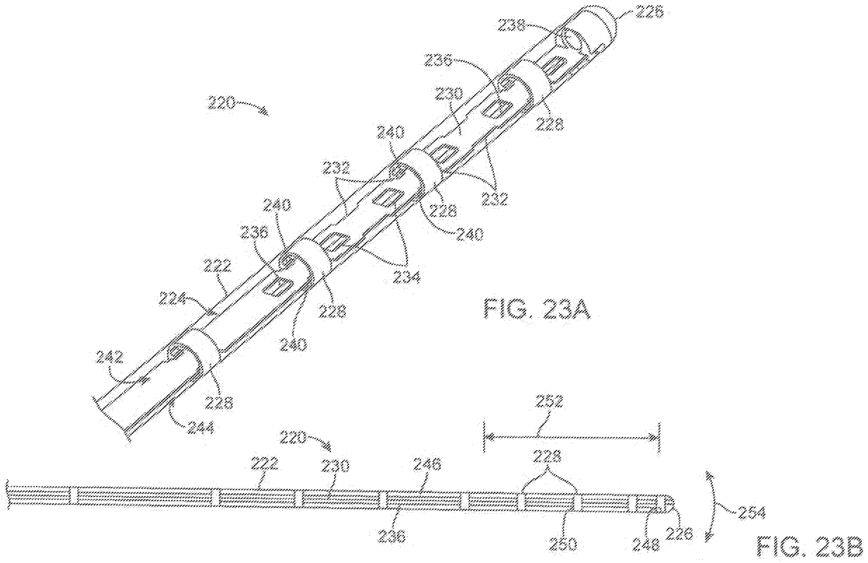

The cooling probe positioned within the balloon **174** may be variously configured, as described above, and may include further variations. As illustrated in the perspective and side views of Figures 23A and 23B, respectively, the cooling probe assembly **220** in this variation may comprise an exhaust catheter **222** which may define a lumen **224** therethrough. While the diameter of the exhaust catheter **222** may be varied, its diameter may range anywhere from, e.g., 4.5 to 4.75 mm. The exhaust catheter **222** may be formed from various materials, such as extruded polyurethane, which are sufficiently flexible and able to withstand the lowered treatment temperatures. The distal end of the catheter **222** may have an atraumatic tip **226** which may be clear and/or which may also define a viewing window or opening through which an imaging instrument such as a hysteroscope **246** may be positioned. One or more supporting members or inserts **228,** e.g., made from a polymer such as polysulfone, may be positioned throughout the length of the lumen **224** to provide structural support to the catheter **222** and to prevent its collapse. The inserts **228** have a relatively short length and define a channel therethrough through which a probe support **230** (e.g., flat wire, ribbon, etc.) may extend. The probe support **230** shown in this variation may comprise a flat wire defining one or more notches **232** along either side which may lock with one or more of the inserts **228** via insert supports **240** to stabilize the probe support **230.**

The probe support **230** itself may be fabricated from a material such as stainless steel and may have a thickness of, e.g., 0.2032 mm (0.008 in.). The probe support **230** may be supported within the lumen **224** via the inserts **228** such that the probe support **230** separates the lumen **224** into a first channel **242** and a second channel **244** where the cooling lumens **236** may be positioned along the probe support **230** within the second channel **244** while the first channel **242** may remain clear for the optional insertion of a hysteroscope **246.** In the event that a hysteroscope **246** is inserted within first channel **242,** the hysteroscope **246** may be advanced selectively along the catheter lumen **224** for visualizing the surrounding tissue or the hysteroscope **246** may be advanced through the length of the catheter **222** until it is positioned within a scope receiving channel **238** defined within the catheter tip **226.**

Because of the thickness of the probe support **230** relative to its width, the probe support **230** may be flexed or curved in a single plane, e.g., in the plane defined by the direction of flexion **254** shown in Figure 23B, while remaining relatively stiff in the plane transverse to the plane defined by the direction of flexion **254.** This may allow for the probe **220** to be advanced into and through the patient's cervix **CV** and into the uterus **UT** while conforming to any anatomical features by bending along the direction of flexion **254** (e.g., up to 90 degrees or more) but may further allow the probe **220** to maintain some degree to rigidity and strength in the transverse plane. Additionally and/or alternatively, the catheter **222** may be actively steered along the direction of flexion **254,** e.g., via one or more pull wires, to allow for positioning or repositioning of the catheter **222** within the balloon **174** to facilitate fluid distribution and/or visualization.

The probe **220** may further include one or more cooling lumens **236** which are positioned along the probe support **230** within the second channel **244.** In this example, at least two cooling lumens are used where a first cooling lumen may extend through the probe **220** and terminate at a first cooling lumen termination **248** near the distal tip **226** and a second cooling lumen may also extend through the probe **220** adjacent to the first cooling lumen and terminate at a second cooling lumen termination **250** at a location proximal to the first termination **248.** The termination points may be varied along the length of the probe **220** depending upon the desired length of the active cooling portion **252** of the probe **220,** which may extend from the distal tip **226** to a length ranging anywhere from, e.g., 2 to 14 cm, along the probe length.

The cooling lumens **236A, 236B** may be fabricated from any number of materials suitable to withstand the low temperature fluids, e.g., Nitinol, polyimide, etc. Moreover, the internal diameter of the cooling lumens may be made to range anywhere from, e.g., 0.254 to 0.4572 mm (0.010 to 0.018 in.). In certain variations, the cooling lumens may have an outer diameter of, e.g., 0.508 mm (0.020 in.) and an internal diameter ranging from, e.g., 0.4064 to 0.4572 mm (0.016 to 0.018 in.), with a wall thickness ranging from, e.g., 0.0508 to 0.1016 mm (0.002 to 0.004 in.).

Because the cooling lumens **236** are located along the second channel 244, as separated by the probe support **230,** one or more windows or openings **234** may be defined along the length of the probe support **230** to allow for the passage of any cryoablative fluid to pass through the openings **234** and to then directly exit the catheter **222** through the openings **260** defined along the catheter **222** body (as described below) and into the balloon interior. Alternatively, the cryoablative fluid may instead proliferate through the entire lumen **224** defined by the catheter **222** before exiting the catheter **222** body. These openings **234** may be cut-outs through the probe support **230** and may number anywhere from zero openings to six or more, as shown, and they may be configured in any number of sizes and shapes. Moreover, these openings **234** may be distributed in any spacing arrangement or they may be uniformly spaced, e.g., 8.128 mm (0.320 in.), depending upon the desired cooling arrangement.

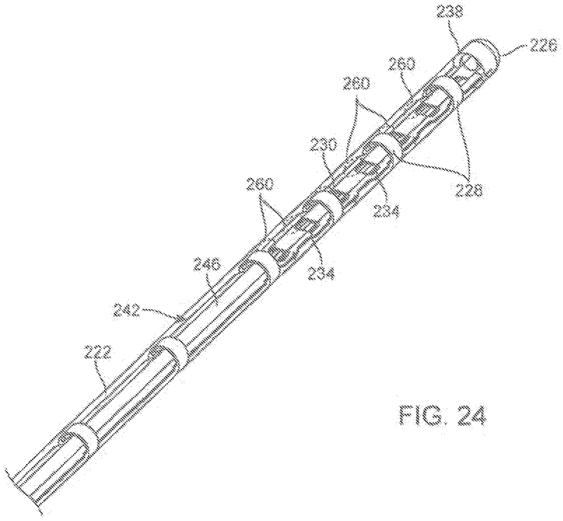

The number of cooling lumens **236** may also be varied to number more than three lumens terminating at different positions along the active portion **252.** Additionally, the activation of the cooling lumens for spraying or introducing the cryoablative fluid may be accomplished simultaneously or sequentially from each of the different cooling lumens depending upon the desired ablation characteristics. While the cooling lumens may simply define a distal opening for passing the fluid, they may be configured to define several openings along their lengths to further distribute the introduction of the cryoablative fluid. The openings **260** along the catheter body **222** for venting the cryoablative fluid into the balloon **174** are omitted from Figure 23A only for clarity purposes but are shown in further detail in the following Figure 24.

As the cryoablative fluid is initially introduced into the catheter lumen **242,** the exhaust catheter **222** may also define one or more openings to allow for the cryoablative fluid to vent or exhaust from the catheter interior and into the interior of the balloon **174.** As shown in the perspective view of Figure 24, one or more openings **260** are illustrated to show one example for how the openings **260** may be defined over the body of catheter **222.** The openings **260** may be positioned along a single side of the catheter **222** or they may be positioned in an alternating transverse pattern, as shown, to further distribute the cooling fluid throughout the balloon interior. In either case, the positioning of the openings **260** may be varied depending upon the desired cryoablation characteristics.

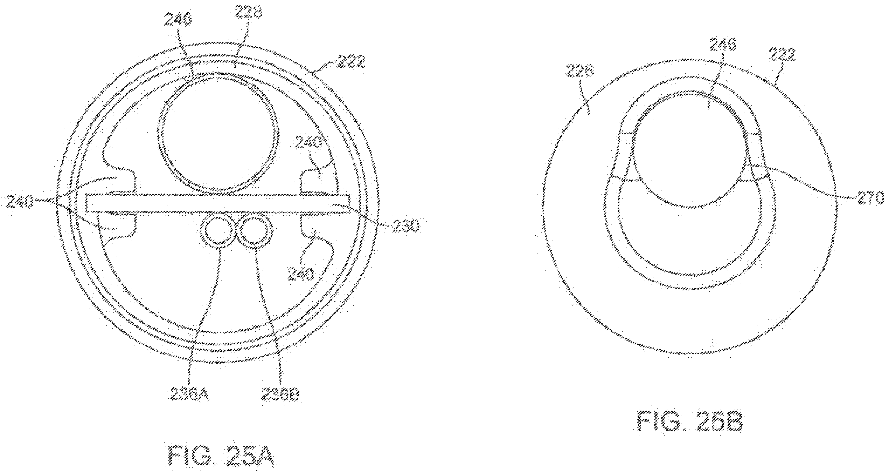

A cross-sectional end view of the cooling probe assembly **220** is shown in Figure 25A illustrating the relative positioning of supporting insert **228** attached to the probe support **230** within the catheter **222.** The two cooling lumens **236A, 236B** are illustrated adjacently positioned along the probe support **230** although they may be positioned elsewhere within the catheter **222** and may also number one lumen or greater than two lumens. Moreover, an optional hysteroscope **246** is also illustrated positioned within the catheter **222** along the probe support **230.** An end view of the distal tip **226** is also illustrated in Figure 25B showing one variation where the distal tip **226** may define a viewing window **270** through which the hysteroscope **246** may be advanced for visualizing within the balloon **174** and uterus **UT.** In other variations, the viewing window **270** may be omitted and the distal tip **226** may be transparent for allowing visualization directly through the tip **226** by the hysteroscope **246.**

With such an arrangement of the cooling probe assembly **220** positioned within the balloon **174** (as illustrated above in Figure 21B), the assembly **210** may be used to treat the surrounding uterine tissue in close conformance against the balloon **174** exterior surface. Introduction of the cryoablative fluid, e.g., nitrous oxide, through the cooling probe **220** may allow for the ablation of the surrounding tissue to a depth of e.g., 4 to 8 mm.

One example for a treatment cycle using a two cycle process may include the introduction of the cryoablative fluid for a treatment time of two minutes where the surrounding tissue is frozen. The fluid may be withdrawn from the balloon **174** and the tissue may be allowed to thaw over a period of five minutes. The cryoablative fluid may be then reintroduced and the tissue frozen again for a period of two minutes and the fluid may then be withdrawn again to allow the tissue to thaw for a period of five minutes. The tissue may be visually inspected, e.g., via the hysteroscope **246,** to check for ablation coverage. If the tissue has been sufficiently ablated, the assembly **210** may be removed from the uterus **UT,** otherwise, the treatment cycle may be repeated as needed. In other alternatives, a single cycle may be utilized or more than two cycles may be utilized, as needed, to treat the tissue sufficiently. Furthermore, during the treatment cycle, a minimum pressure of, e.g., 40 to 80 mm Hg, may be optionally maintained by the cryogenic liquid or by a gas (e.g., air, carbon dioxide, etc.) to keep the balloon **174** and uterus **UT** open.

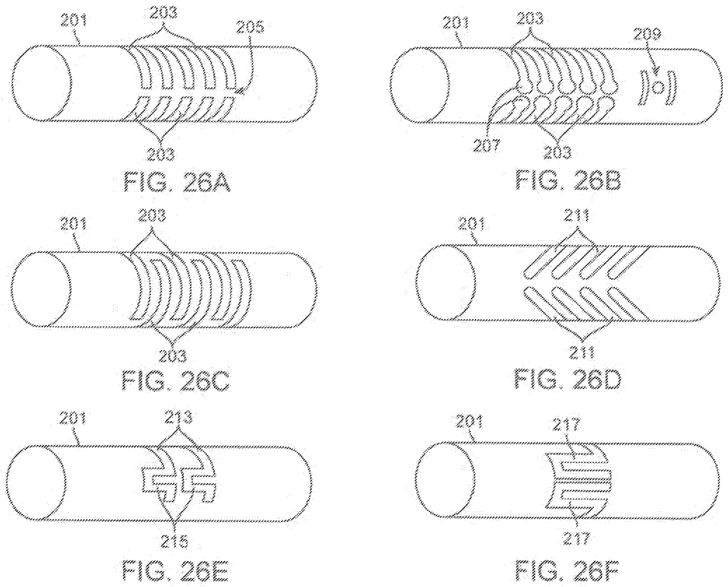

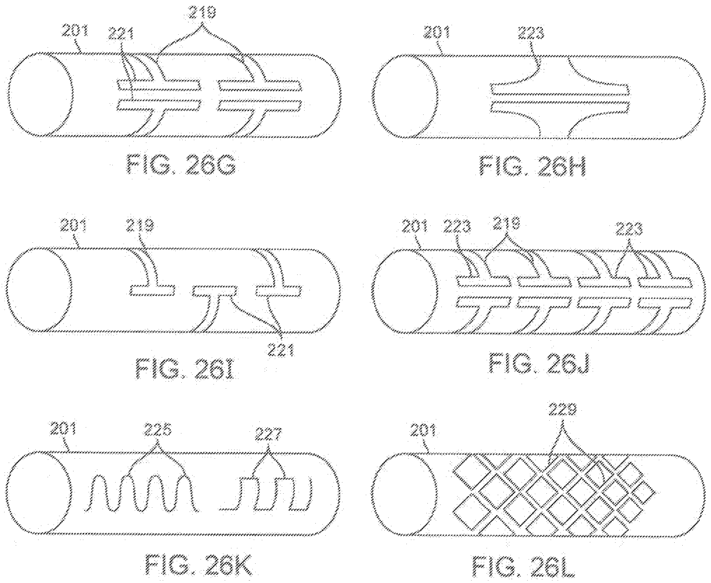

In yet another alternative, aside from having a catheter **222** made as an extruded lumen, the catheter may be formed into tubing **201** such as a hypotube fabricated from a material such as, e.g., stainless steel, nitinol, etc. A tubing **201** formed from a metal may provide additional strength to the catheter and may remove the need for any inserts to maintain a patent lumen. To increase the flexibility of the tubing **201,** one or more slots **203** may be formed or cut along the body of the tubing **201,** as shown in the example of Figure 26A, which illustrates a perspective view of tubing **201** having one or more slots **203** cut transversely relative to the tubing **201.** Aside from increased flexibility, the slots **203** may be aligned to provide for preferential bending or curvature along predetermined planes by the tubing while inhibiting the bending or curvature along other planes, e.g., planes transverse to the bending plane, similar to the preferential bending or curvature provided by the probe support **230.**

The ends of the slots **203** may be formed to provide a separation **205** between the ends of the slots **203.** Figure 26B shows another variation where each of the transverse slots **203** may have a strain relief feature **207** formed at the distal ends of each slot **203** such that bending of the tubing **201** over the slotted region may occur with reduced stress imparted to the slots **203** and tubing **201.** An additional feature may include optional tabs **209** which may be formed along the body of the tubing **201** to extend internally for holding a cooling lumen within the lumen of the tubing **201.**

Another variation is shown in Figure 26C which shows transverse slots **203** formed along the body of the tubing **201** where the slots **203** may be formed in an alternating pattern with respect to one another. Figure 26D shows yet another variation where angled slots **211** may be formed relative to tubing **201.** Figure 26E shows another variation having one or more serpentine slots **213** for preventing pinching where a distal end of each slot **213** may have a transverse slot **215** formed. Figure 26F shows another variation where one or more slots **217** having a transverse and longitudinal pattern may be formed along tubing **201.**

Figure 26G shows another variation where a transverse slot **219** may have a longitudinal slot **221** formed at its distal end. Figure 26H shows yet another variation where one or more tapered slots **223** may be formed along tubing **201.** Figure 261 shows another variation where a transverse slot **219** may have a longitudinal slot **221** formed where each of the longitudinal slots **221** may be aligned longitudinally along the body of tubing **201.** Figure 26J shows another variation where transverse slots **219** may have longitudinal slots **223** aligned adjacent to one another and having rounded ends. Figure 26K shows another variation where either a curved serpentine slot **225** or an angled slot **227** may be formed along the tubing **201.** Alternatively, both curved serpentine slot **225** and angled slot **227** may both be formed. Another variation shows tubing **201** having a plurality of slots **229** formed into a lattice structure over the body of tubing **201.**

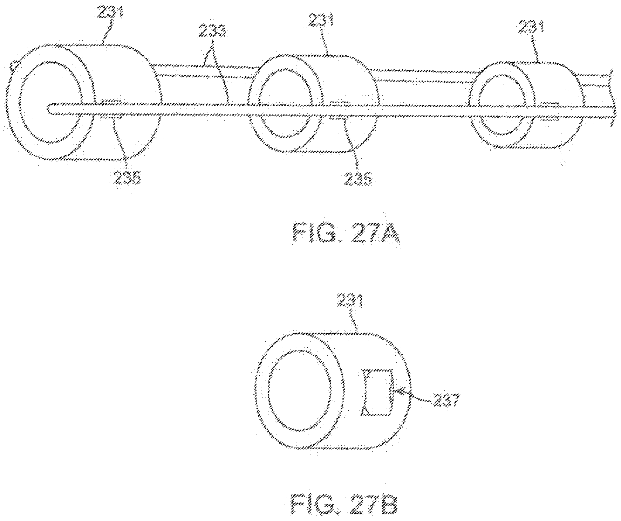

Aside from utilizing a continuous body of tubing **201** for the length of the cooling probe, discrete tubing reinforcing ring **231** may instead be formed from tubing **201.** Figure 27A shows an example where a plurality of reinforcing rings **231** may be separated into discrete ring elements and attached to one another in a linear manner with one or more longitudinal beam members **233** which may be attached to each reinforcing ring **231** at an attachment point **235,** e.g., weld, adhesive, etc. One or more of the reinforcing rings **231** may be formed to have, e.g., a bent-in tab **237,** for supporting beam **233** rather than utilizing a weld, adhesive, etc., as shown in the detail perspective view of Figure 27B. The assembly of the reinforcing ring **231** and beams **233** may be covered with a membrane or other covering to form a uniform structure.

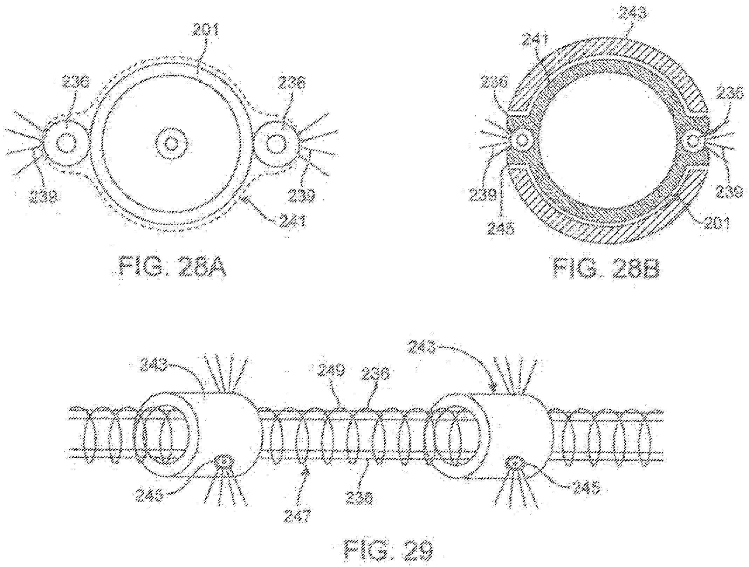

An example of a covering which may be used is shown in the end view of Figure 28A which shows a portion of tubing **201** or reinforcing ring **231** and cooling lumens **236** positioned on either side of tubing **201** or reinforcing ring **231.** A heat shrink **241** material may be placed over the probe assembly while maintaining clearance for openings **239** to allow for delivery of the cryoablative fluid.

Another variation is shown in the cross-sectional end view of Figure 28B which shows the tubing **201** and respective cooling lumens **236** positioned within an insert **243** which define insert openings **245** for introducing the cryoablative fluid. Yet another variation is shown in the perspective view of Figure 29 which may incorporate a wound spring **247** which may be tightly wound or packed to provide flexibility and to further provide a lumen **249** for the exhaust. One or more inserts **243** may be positioned longitudinally along the length of the spring **247** and the cooling lumens **236** may be routed through the spring **247** and coupled to each insert **243.**

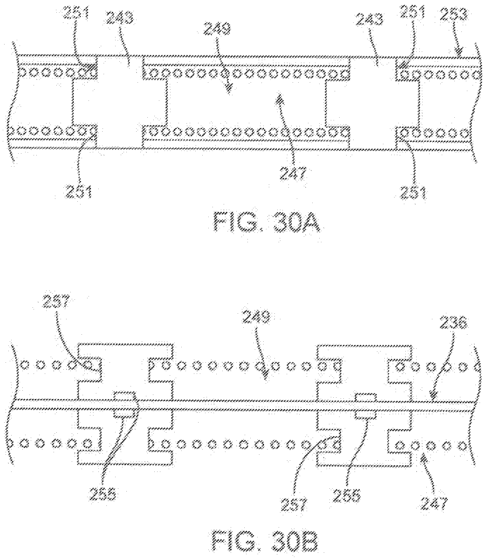

Another variation is shown in the partial cross-sectional side view of Figure 30A which illustrates how one or more inserts **243** may each define a step **251** for securement to the spring **247.** The entire assembly may then be covered by a covering **253,** e.g., flexible extrusion. Each of the inserts **243** may remain uncovered by either the spring **247** or covering **253** to ensure that the cryoablative fluid has an unhindered pathway to the balloon interior. Figure 30B shows another variation where each of the inserts **243** may define a respective receiving channel **257** on either side of the insert **243** for securement to the spring **247.** An example of a cooling lumen **236** is shown attached to each of the inserts **243** via an attachment **255,** e.g., weld, adhesive, etc.

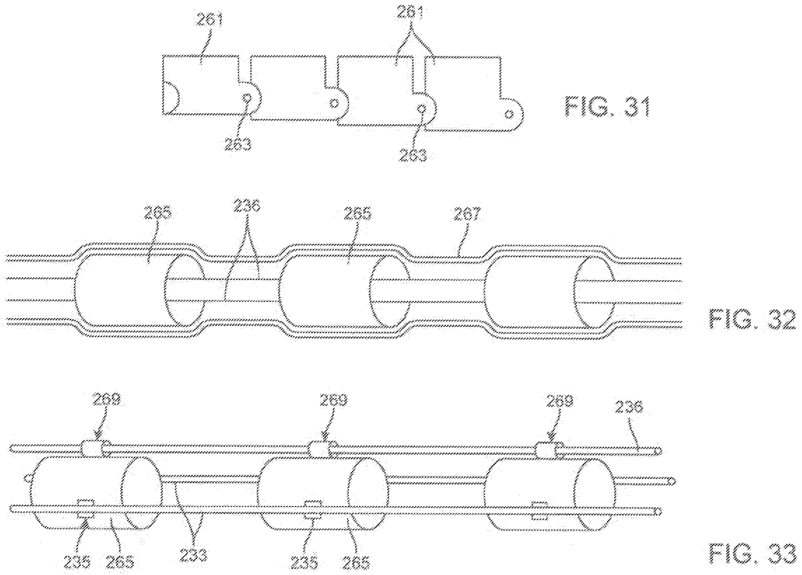

Aside from increasing the flexibility of the tubing or cooling probe, the cooling lumen may be configured to increase its flexibility as well. An example is shown in Figure 31 which shows a portion of a cooling lumen wall **261** having a plurality of pivoted attachments **263.** Such an arrangement may allow for each segment of the cooling lumen wall **261** to pivot such that the cooling lumen cumulatively provides sufficient flexibility to bend and curve as the cooling probe assembly is advanced and positioned within the uterus. Such a cooling lumen may be incorporated into any of the probe variations described herein.

Another example of a cooling probe assembly is illustrated in the perspective view of Figure 32 which shows discrete embedded insert **265** and one or more cooling lumens **236** attached to each respective insert **265** covered with a covering **267.** In this example, the covering **267** may be implemented without any additional features or structures. Figure 33 shows yet another example where individual inserts **265** may be aligned and coupled with one or more beams **233,** as previously described. An additional sliding joint **269** may be attached or integrated along each insert **265** to provide support to one or more cooling lumens **236** which may be translatably positioned through each aligned sliding joint **269.**

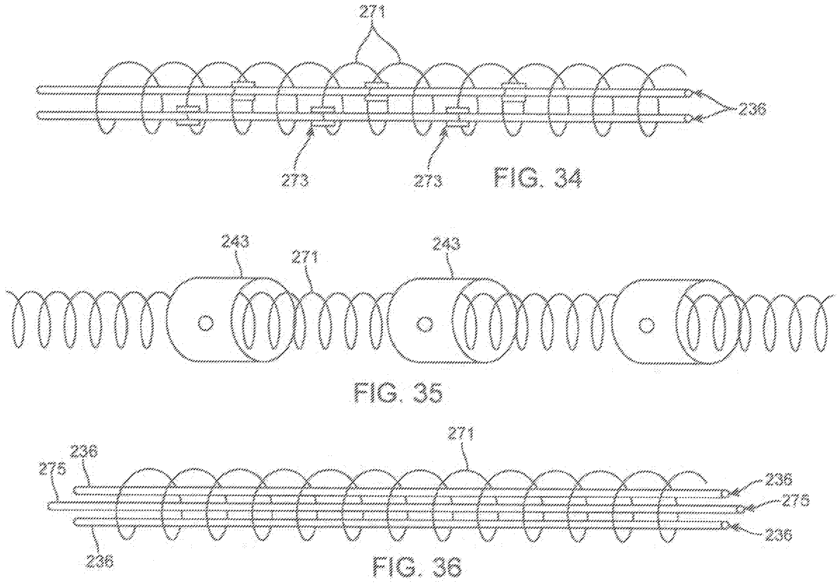

Yet another variation is illustrated in the side view of Figure 34 which shows a wound spring element **271** having one or more cooling lumens **236** aligned longitudinally along the spring element **271.** The one or more cooling lumens **236** may be attached to the spring element **271** via connectors **273** which may be aligned relative to one another to receive and secure the cooling lumens **236.** A covering may be optionally secured over the spring assembly.

Figure 35 shows another variation where spring element **271** may incorporate one or more respective inserts **243.** In this variation, the spring element **271** has the one or more cooling lumens **236** coupled to the spring element **271** itself Figure 36 shows yet another variation where the spring element **271** and the one or more cooling lumens **236** (which may be coupled directly to the spring element **271**), may have an optional secondary lumen **275** passing through the spring element **271** and optionally attached to the spring itself. The second lumen **275** may be sized for receiving an instrument such as a hysteroscope **246.** The second lumen 275 may provide a redundant liquid or gas pathway should the primary lumen become partially or fully obstructed. The redundant pathway may exist between the optional instrument, e.g.hysteroscope, and primary lumen or within the full second lumen 275.

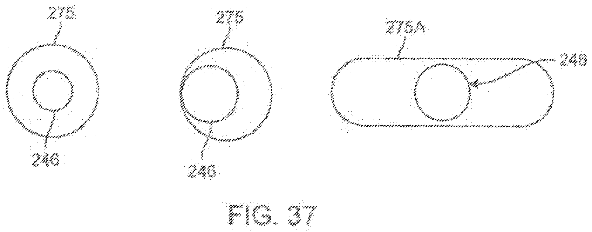

The secondary lumen **275** may be shown in various cross-sections in the end views of Figure 37. A first variation is illustrated shown secondary lumen **275** having a circular cross-sectional area with a hysteroscope **246** passed through a center of the lumen **275.** A second variation is illustrated where the hysteroscope **246** may be passed along a side of the lumen **275** and a third variation is illustrated showing a secondary lumen **275A** having an elliptical cross-sectional area.

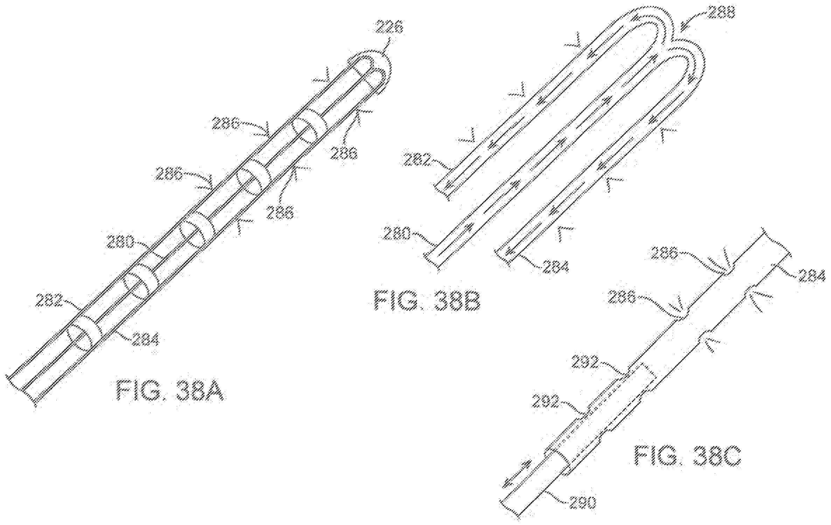

Another variation for a cooling probe assembly, which is not covered by the subject-matter of the claims, is shown in the perspective views of Figures 38A to 38C. In this variation, the catheter body **222** is omitted for clarity purposes only but a main delivery line **280** is shown extending through the catheter with at least two side delivery lines **282, 284** positioned near the surface of the catheter body, as shown in Figure 38A. The main delivery line **280** may be in fluid communication with the side delivery lines **282, 284** via a junction **288,** shown in Figure 38B, near or within the distal tip **226.** As the cryoablative fluid is introduced into the main delivery line **280,** the fluid in the side delivery lines **282, 284** may be vented through one or more openings **286** defined therealong for venting through and into the catheter and balloon interior. An optional mandrel **290,** as shown in Figure 38C, may be slidingly fitted within each of the side delivery lines **282, 284** and actuated automatically along with the retraction of the sheath **212** or by the user to slide along the interior of one or both side delivery lines **282, 284** to selectively obstruct the openings **286** and thereby control the amount of cryoablative fluid delivered. As shown, one or more obstructed openings **292** may be blocked by the mandrel **290** by selectively sliding the mandrel **290** accordingly. In other variations, rather than using mandrels inserted within the delivery lines **282, 284,** a sheath or mandrel placed over the delivery lines **282, 284** may be used instead to achieve the same results.

As described above, the retraction of the mandrel **290** may be optionally actuated to follow along with the retraction of the sheath **212.** Accordingly, the retraction of the mandrel **290** may occur simultaneously with the retraction of the sheath **212** but the retraction may optionally occur at different rates as the amount of cryoablative fluid delivered may be related to the length of the uterine cavity to be treated. For instance, a sheath retraction of, e.g., 7 cm, may result in 10 unobstructed openings **286** whereas a sheath retraction of, e.g., 4 cm, may result in, e.g., 6 unobstructed openings **286.**

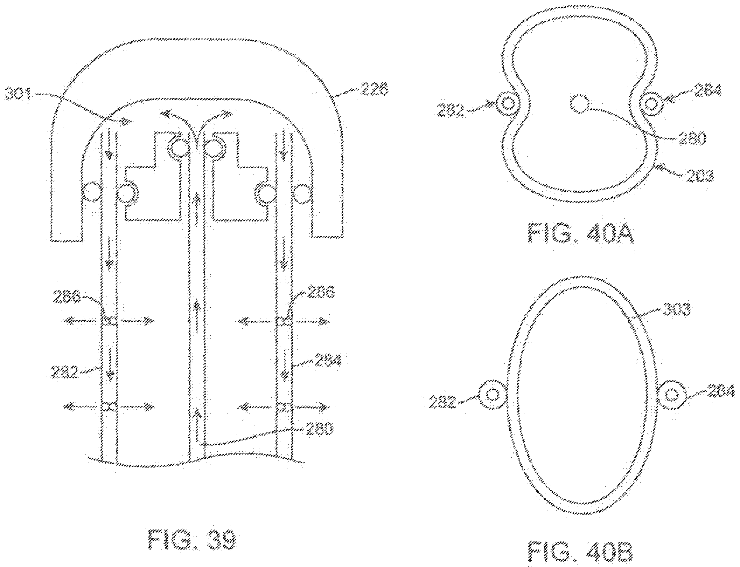

Another variation of the cooling probe assembly is illustrated in the detail cross-sectional side view of Figure 39. In this variation, a single main delivery line **280** may pass through and into communication with distal tip **226.** Rather than having the side delivery lines **282, 284** coupled directly to the main delivery line **280,** each respective line may be coupled to a common chamber **301** defined within the distal tip **226.** Such an assembly may be used with alternative variations of the exhaust lumen **303** as shown in one example in the cross-sectional end view of Figure 40A. in this example, the exhaust lumen 303may be formed to have an indented cross-sectional area to accommodate the side delivery lines **282, 284.** Alternatively, the exhaust lumen **303** may be shaped to have an elliptical cross-sectional area instead, as shown in Figure 40B.

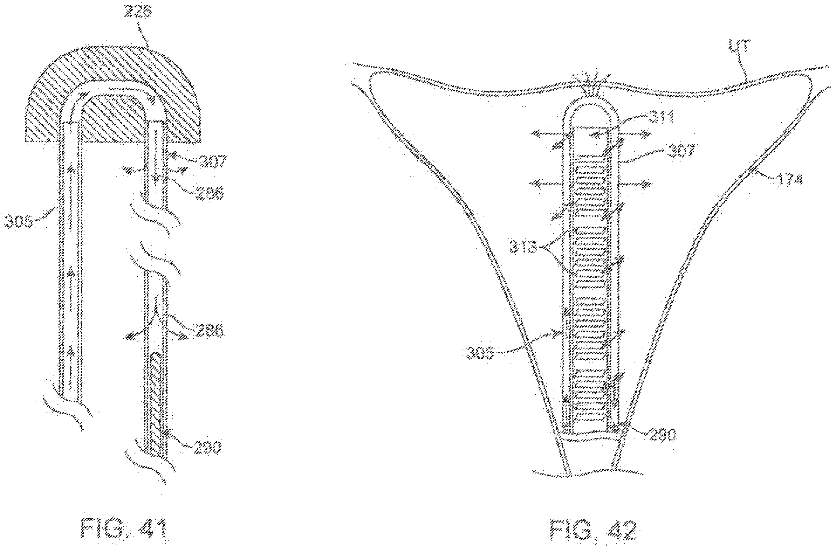

In yet another alternative, the cooling lumens may be formed to have a single introduction or infusion line **305** and a single delivery line **307** where the delivery line **307** may be in fluid communication directly with the introduction or infusion line **305** through the distal tip **226,** as shown in the cross-sectional side view of Figure 41. The infusion line **305** and delivery line **307** may be formed as separate lines or they may formed as a single continuous line where the infusion line **305** enters distal tip **226** and is curved to redirect the ablative fluid proximally through the delivery line **307.** In this variation, as in the previous variations, a translatable mandrel **290** may be slidably positioned within the delivery line **307** or optionally along an outer surface of the delivery line **307** to selectively obstruct the openings **286** defined along the line **307.** In other variations, one or more openings may also be optionally aligned along the infusion line **305** in addition to the openings **286** along delivery line **307.** Moreover, the mandrel **290** may be actuated to slide (either at the same or different rate) along with the retraction of the sheath. Figure 42 illustrates an example where the cooling probe assembly may be introduced into the interior of balloon **174** when deployed within the uterus **UT**. Alternatively, the balloon **174** may be attached directly along an outer surface of the cooling probe assembly itself. The expanded length of balloon **174** may be fixed along the outer surface of the cooling probe assembly proximal to the distal tip or it may be optionally adjustable via the positioning of the outer sheath. As shown, the introduction line **305** may introduce the cryoablative fluid along the cooling probe assembly where it may then be flowed proximally along the delivery line **307** for introduction into the interior of the balloon **174.** As the cryoablative fluid is introduced, a slotted tube **311** having one or more directional slots **313** may be used to optionally direct the flow of the cryoablative fluid into the balloon interior.

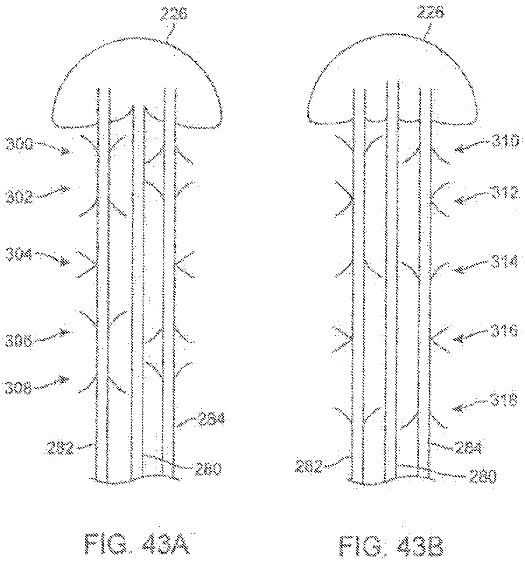

Figures 43A and 43B illustrate additional variations for selectively controlling the configuration of the hole directions along the side delivery lines to optionally control appropriate ablation depths and tapering, as needed or desired. In the variation of Figure 43A, the adjacent side delivery lines **282, 284** from the distal tip **226** may be configured such that openings **300** are configured in an up/down configuration, openings **302** are configured in an down/up configuration, openings **304** are configured in an left/right configuration, openings **306** are configured in an up/down configuration, and openings **308** are configured in an down/up configuration. The hole directions of up/down/left/right are relative to the figures shown and are presented for illustrative purposes.

Likewise, the variation shown in Figure 43B illustrates how the adjacent side delivery lines **282, 284** may be configured such that openings **310** are configured in an up/down configuration, openings **312** are configured in an left/right configuration, openings **314** are configured in an down/up configuration, openings **316** are configured in an left/right configuration, and openings **318** are configured in an up/down configuration. These variations are illustrated as exemplary variations and other variations of hole directions may be accomplished as desired.

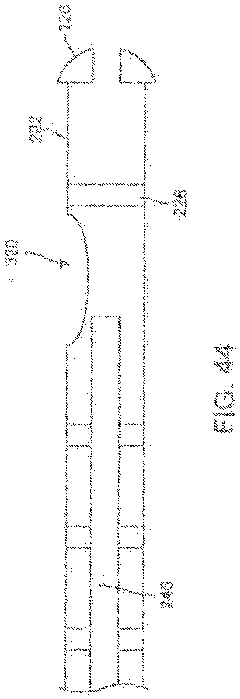

Aside from the positioning of the fluid openings, the catheter body **222** itself may optionally incorporate a skived viewing window **320,** as shown in the side view of Figure 44, to facilitate visualization of the surrounding balloon **174** and tissue by the hysteroscope **246** which may be advanced into proximity to the window **320** or entirely through as desired.

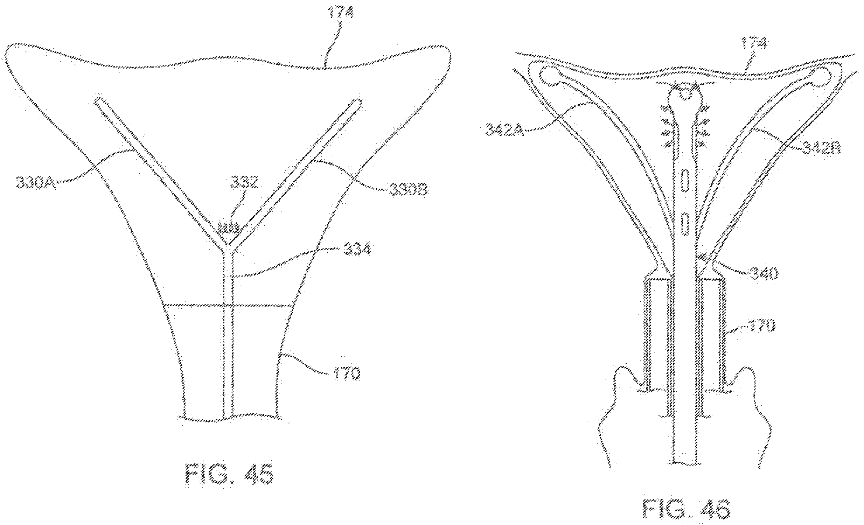

As previously described, the balloon **174** may be expanded within the uterus **UT** and particularly into the uterine cornu **UC** by an initial burst of gas or liquid. Other mechanisms may also be used to facilitate the balloon expansion. One variation is shown in Figure 45 which illustrates a balloon **174** having one or more supporting arms **330A, 330B** extending from a support **334** which may be deployed within the balloon **174.** The supporting arms **330A, 330B** may be variously configured although they are shown in this example in a Y-configuration. Each of the distal ends of the arms may extend from a linear configuration into the expanded Y-configuration, e.g., via a biasing mechanism **332,** which may bias the arms to extend once the sheath **212** is retracted. The distal ends of the arms **330A, 330B** may extend into the tapered corners of the balloon **174** to facilitate the balloon **174** expansion into the uterine cornu **UC** and may also help to center the balloon **174** within the uterus **UT.**

Figure 46 shows a partial cross-sectional side view of another variation of an expansion mechanism contained within the balloon **174** where one or more supporting arms **342A, 342B** may be mechanically actuated to extend, e.g., via a biasing mechanism, push/pull wires, etc. Moreover, the arms **342A, 342B** may be integrated into the design of the cooling probe **340** as an integrated assembly.

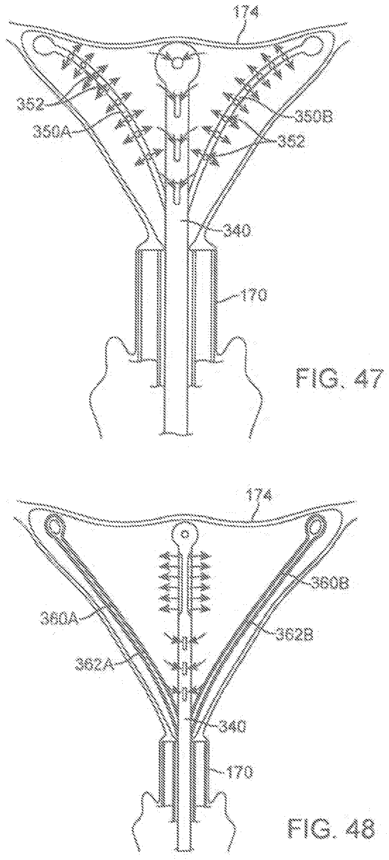

Figure 47 shows a partial cross-sectional side view of another variation where the supporting arms **350A, 350B** may also integrate one or more openings **352** for the infusion of the cryoablative fluid. In this example the arms **350A, 350B** may be integrated with the cooling probe **340** or separated. In either case, the inclusion of the openings **352** may facilitate the distribution of the fluid into the balloon **174** interior.

Figure 48 shows yet another variation where the supporting arms **360A, 360B** may be incorporated into elongate channels or pockets **362A, 362B** defined along the balloon **174** itself. In this and other variations shown, the supporting arm members may optionally integrate the one or more openings for cryoablative fluid delivery and may also be integrated into elongate channels as practicable.

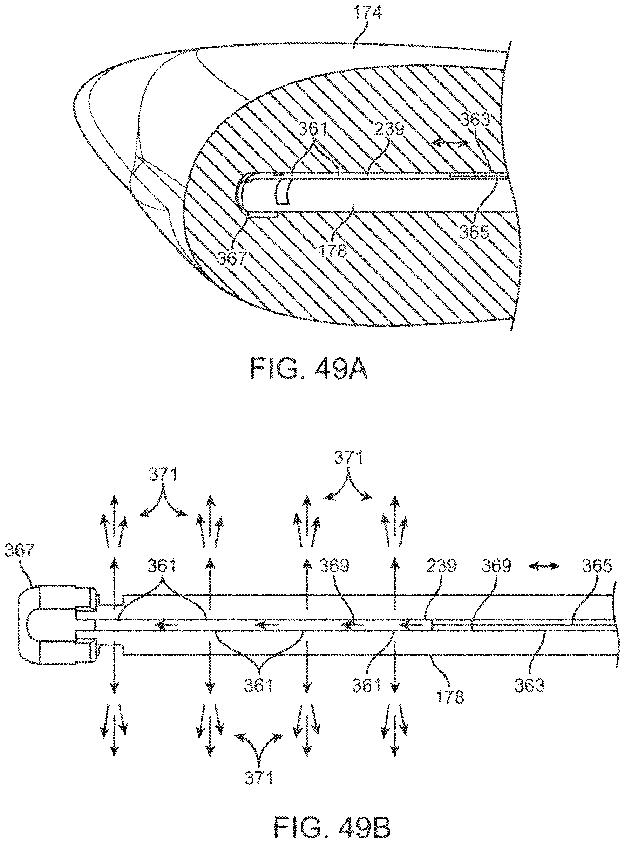

Figures 49A and 49B show cross-sectional side views of yet another variation of a cooling probe which utilizes a single infusion line in combination with a translatable delivery line. To accommodate various sizes and shapes of uterine cavities, the cooling probe may have a sliding adjustment that may be set, e.g., according to the measured length of the patient's uterine cavity. The adjustment may move along the sheath along the exhaust tube as well as the delivery line within the infusion line. The sheath may constrain the liner **174** and also control its deployment within the cavity.

In this variation, an infusion line **239** (as described above) may pass from the handle assembly and along or within the sheath and into the interior of liner **174.** The infusion line **239** may be aligned along the probe **178** such that the infusion line **239** is parallel with a longitudinal axis of the probe **178** and extends towards the distal tip **367** of the probe **178.** Moreover, the infusion line **239** may be positioned along the probe **178** such that the line **239** remains exposed to the corners of the liner **174** which extend towards the cornua. With the infusion line **239** positioned accordingly, the length of the line **239** within the liner **174** may have multiple openings formed along its length which act as delivery ports for the infused cryogenic fluid or gas. A separate translating delivery line **365,** e.g., formed of a Nitinol tube defining an infusion lumen therethrough, may be slidably positioned through the length of the infusion line **239** such that the delivery line **365** may be moved (as indicated by the arrows in Figure 49A) relative to the infusion line **239** which remains stationary relative to the probe **178.**

The openings along the length of the infusion line **239** may be positioned such that the openings are exposed to the sides of the interior of the liner **174,** e.g., crossdrilled. As the cryogenic fluid or gas is introduced through the delivery line **365,** the infused cryogenic fluid or gas **369** may pass through the infusion line **239** and then out through the openings defined along the infusion line **239.** By adjusting the translational position of the delivery line **365,** the delivery line **365** covers a selected number of the openings resulting in a number of open delivery ports **361** as well as closed delivery ports **363** which are obstructed by the delivery line **365** position relative to the infusion line **239,** as shown in the top view of Figure 49B.

By translating the delivery line **365** accordingly, the number of open delivery ports **361** and closed delivery ports **363** may be adjusted depending on the desired treatment length and further ensures that only desired regions of the uterine tissue are exposed to the infused cryogenic fluid or gas **369.** Once the number of open delivery ports **361** has been suitably selected, the infused cryogenic fluid or gas **369** may bypass the closed delivery ports **363** obstructed by the delivery line **365** and the fluid or gas may then be forced out through the open delivery ports **361** in a transverse direction as indicated by the infusion spray direction **371.** The terminal end of the infusion line **239** may be obstructed to prevent the distal release of the infused fluid or gas **369** from its distal end. Although in other variations, the terminal end of the infusion line **239** may be left unobstructed and opened.

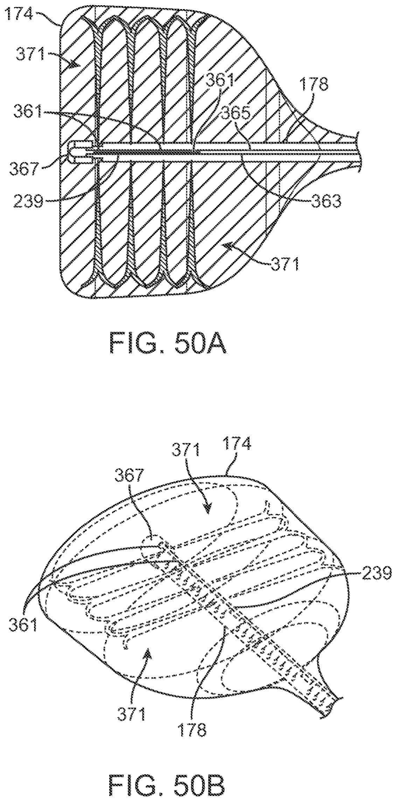

Figures 50A and 50B show top and perspective views of the expanded liner **174** with four pairs of the open delivery ports **361** exposed in apposed direction. Because the infused fluid or gas **369** may be injected into the liner **174,** e.g., as a liquid, under relatively high pressure, the injected cryogenic liquid may be sprayed through the open delivery ports **361** in a transverse or perpendicular direction relative to the cooling probe **178.** The laterally infused cryogenic fluid **371** may spray against the interior of the liner **174** (which is contacted against the surrounding tissue surface) such that the cryogenic liquid **371** coats the interior walls of the liner **174** due to turbulent flow causing heavy mixing. As the cryogenic liquid **371** coats the liner surface, the sprayed liquid **371** may absorb heat from the tissue walls causing rapid cooling of the tissue while also evaporating the liquid cryogen to a gas form that flows out through the cooling probe **178.** This rapid cooling and evaporation of the cryogenic liquid **371** facilitates the creation of a fast and deep ablation over the tissue. During treatment, the temperature within the cavity typically drops, e.g., -89° C, within 6-7 seconds after the procedure has started. While the interior walls of the liner **174** are first coated with the cryogenic liquid **371,** the cryogenic liquid **371** may no longer change phase as the procedure progresses.

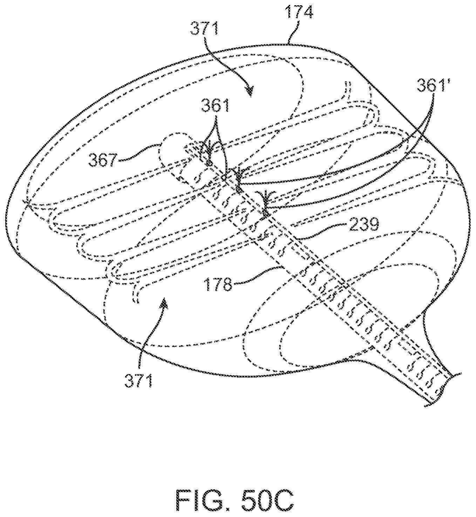

While four pairs of the open delivery ports **361** are shown, the number of exposed openings may be adjusted to fewer than four pairs or more than four pairs depending on the positioning of the delivery line **365** and also the number of openings defined along the infusion line **239** as well as the spacing between the openings. Moreover, the positioning of the openings may also be adjusted such that the sprayed liquid **371** may spray in alternative directions rather than laterally as shown. Additionally and/or alternatively, additional openings may be defined along other regions of the infusion line **239.** For instance, one or more openings **361',** e.g., one to three holes or more, may be added along the length of the infusion line **239** such that the openings directly face the portion of the liner **174** placed against the anterior portion of the contacted tissue, as shown in the perspective view of Figure 50C.

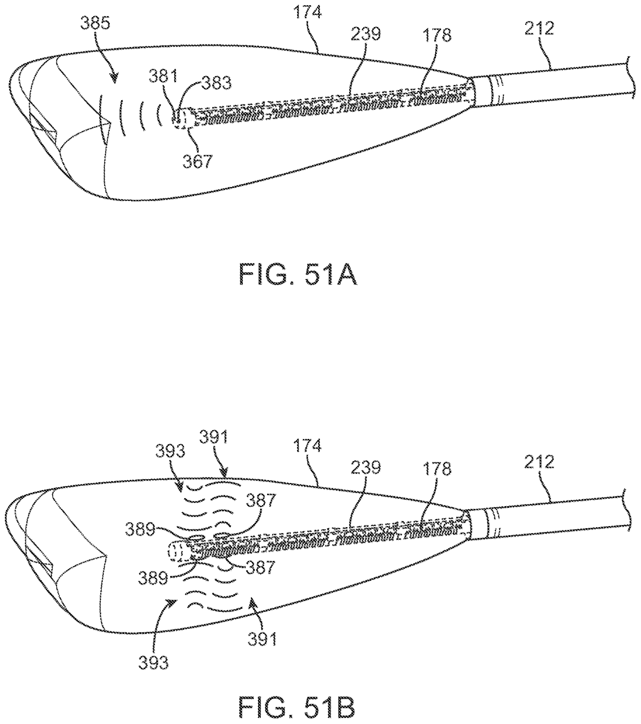

Prior to or during treatment, the positioning of the cooling probe **178** within the interior of the liner **174** and the uterus may be determined through various mechanisms. Visualization may be optionally provided by use of hysteroscopy, endoscopy, fluoroscopy, or ultrasound or other more invasive modalities. However, other variations for determining the cooling probe **178** position may include use of a transmitter **381,** e.g., light, ultrasound, etc., which may be placed on the distal tip **367** of the probe **178,** as shown in the perspective view of Figure 51A.

In the event that a light **381** such as an LED light is placed upon the distal tip **367,** the user may simply visually monitor the patient for the transmission of the light through the tissue and skin of the patient to determine whether the tip **367** is properly positioned within the uterus or the peritoneal cavity depending on where the light is emitted directly through the body. In another variation, a sensor or receiver **383** may be placed upon the distal tip **367** adjacent to the transmitter **381.** As the transmitter **381** emits a light or ultrasound signal **385,** the signal **385** may reflect off the surrounding tissue surface (and through the liner **174**). Depending on the wavelength of the reflected signals collected by the receiver **383,** a processor or microcontroller can be used to determine the general color of the tissue in front of the end of the probe as the inner wall of the uterus, intestine, and bladder should all have distinct color signatures.

In yet another variation, one or more transmitters **387,** e.g., light, ultrasound, etc., may be placed along the probe **178** along opposite surfaces to facilitate determining the amount of cavity expansion, e.g., during initial pre-treatment liner expansion. The transmitted signals **391** may be emitted as discrete pulses of light which are returned as reflected signals **393** to corresponding sensors or receivers **389** which are adjacent to the transmitters **387.** By measuring the time it takes for the reflected signals **393** to return to the sensors or receivers **389,** the processor or microcontroller can determine the amount of cavity expansion which has occurred. The transmitters and/or sensors/receivers may be incorporated in any of the variations of the devices and methods described herein.

Once a cryoablation treatment procedure has been completed and the interior of the liner is vented, the device may be removed from the patient body. To facilitate the removal of the liner **174** from the treated tissue surface, negative pressure may be applied to the interior of the liner **174** to quickly remove the discharged cryogenic fluid or gas as well as to help pull the liner **174** away from the tissue surface. Removing the liner **174** from the tissue surface may be relatively easy when the removal force is normal to the tissue surface. Hence, use of negative pressure or a suction force at the end of an ablation procedure may facilitate the removal or peeling of the liner **174** from the uterine tissue surface.

The pump which is used to introduce air initially into the liner interior may be used to also remove the discharged cryogenic fluid or gas particularly if the pump (such as pump **225** shown above in Figure 21G) is configured as a reversible pump, e.g., connected to H-bridge circuitry which may allow for the polarity of the voltage on the pump to be reversed which will allow for the reversal of the flow direction of the pump. Another variation may utilize a separate pump which is configured to draw a suction force upon the liner interior separate from the pump used to introduce air into the liner.

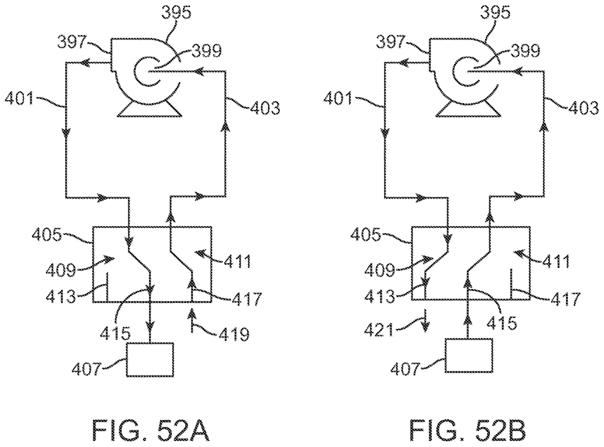

Yet another variation may utilize a non-reversible pump **395** which is fluidly coupled to a 5-port, 2 position, 4-way valve **405** which allows for the pressured pump output **397** to be connected to the exhaust/liner **407** through output line **401** and through liner line **415** within valve **405.** A first switch **409** within the valve **405** may be switched to fluidly couple the output line **401** with the liner line **415.** The negative pressure pump input **399** may be opened to the ambient air **419** through ambient line **417** within the valve **405** for initially expanding the liner **174,** as shown in the schematic illustration of Figure 52A. The ambient air **419** may be fluidly coupled to the input line **403** via second switch **411** within the valve **405.**

When the ablation procedure has been completed and the liner **407** to be vented and collapsed, the valve **405** may be switched such that first switch **409** fluidly couples output line **401** to ambient line **413** and second switch **411** switches to fluidly couple the liner **407** to liner line **415** and input line **403,** as shown in the schematic illustration of Figure 52B. The fluid and/or gas within the liner **407** may be drawn out by the negative pressure created within input line **403** which may then force the discharged fluid or gas through output line **401,** through ambient line **413** within valve **405,** and out as exhausted fluid or gas **421.** Thus, a single directional pump **395** may be used for both inflation and deflation with the valve **405.**

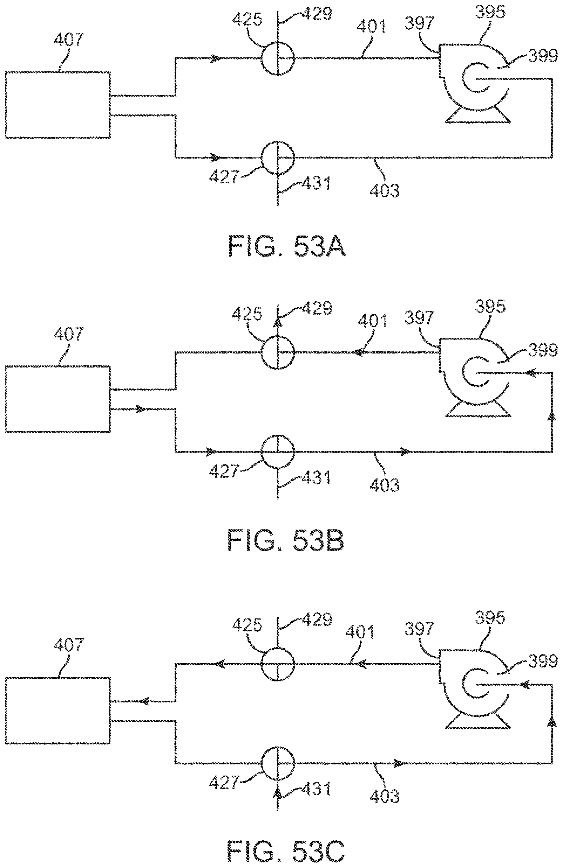

Yet another variation is shown in the schematic diagrams of Figures 53A to 53C which also illustrates the use of a non-reversible pump **395** for both inflation and deflation. In this variation, the pump output line **401** may incorporate a first 3-way valve **425** and the pump input line **403** may incorporate a second 3-way valve **427.** The first and/or second valves **425, 427** may comprise, e.g., 3-way solenoid valves, which may remain unpowered with both valves **425, 427** connecting the pump **395** to the ambient environment through respective ambient lines **429, 431,** as shown in Figure 53A. The configuration shown may keep any fluid or gas within the liner **407** from being pumped or leaked to the environment.

If the liner **407** is to be evacuated, the first valve **425** may be actuated or energized such that the pump output line **401** is fluidly connected to the ambient line **429** and the second valve **427** may be actuated or energized to fluidly couple the liner **407** with the pump input line **403,** as shown in Figure 53B. With the pump **395** actuated, the input line **403** may draw a negative pressure to suction out the fluid or gas within liner **407** while the pump output pushes air or the suctioned fluid or gas out through ambient line **429.**

If the liner is to be initially expanded and/or the cryogenic fluid or gas is to be to introduced into the liner **407** for treatment, the first valve **425** may be actuated or energized to fluidly couple the output line **401** from the pump **395** to the liner **407** while the second valve **427** may be actuated or energized to fluidly couple the pump input line **403** with the ambient line **431,** as shown in Figure 53C. Moreover, the actuation of the first and second valves **425, 427** may be coordinated such that simultaneous or individual actuation of the valves is controlled by a processor. Alternatively, one or both valves **425, 427** may be controlled manually by the user. As above, this valve configuration may be used with any of the different liner, probe, handle assembly, or treatment methods described herein.

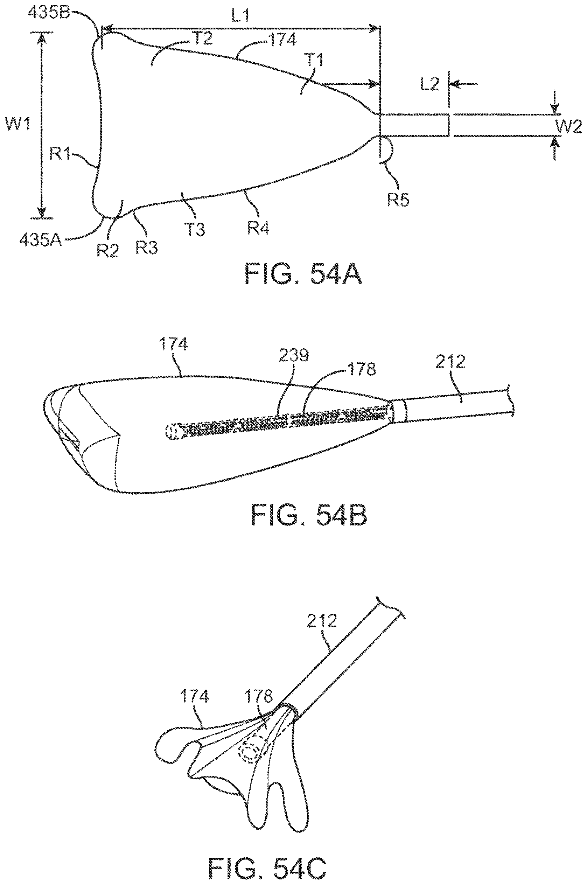

Turning now to the liner itself, the liner may be formed to have, e.g., a nominal 0.03048 mm (0.0012 in.) thick flexible membrane such as pellethane. The liner **174** may be optionally formed as a composite from one or more sheets of material, e.g., two sheets of membrane which are RF welded. When laid out in a flattened shape, the liner **174** may shaped in a manner, as shown in the top view of Figure 54A, which allows the liner **174** to inflate or expand into a contoured shape which conforms closely to a uterine cavity, as shown in the perspective view of Figure 54B. Figure 54A shows one example of a flattened liner **174** which gently tapers from the opening to a curved shape forming a first curved portion **435A** and a second curved portion **435B** opposite to the first curved portion **435A.** The liner **174** may hence taper gently from a first width **W1,** e.g., about 60.96 mm (2.4 in.) down to a second width **W2,** e.g., about 7.62 mm (0.3 in.) over a length **L1** of, e.g., about 88.9 mm (3.5 in.). The neck may form a length **L2,** e.g., about 22.86 mm (0.9 in.).

The region between each of the first and second curved portions **435A, 435B** may also be curved to have a radius **R1** of e.g., about 88.9 mm (3.5 in.), while curved portions **435A, 435B** may also be curved to each have a radius **R2** of, e.g., about 7.62 mm (0.3 in.). The portion of the liner proximal to the portions **435A, 435B** may also have a radius **R3,** e.g., about 27.94 mm (1.1 in.) and an oppositely radiused portion of radius **R4,** e.g., about 203.2 mm (8.0 in.). The region between the liner **174** and neck may further have a radius **R5,** e.g., about 5.08 mm (0.2 in.).

The liner **174** itself may be formed to have a uniform thickness over the entire liner. Alternatively, different portions of the liner **174** may also be formed to have differing thicknesses depending upon the desired degree of treatment along differing portions of the liner **174.** For instance, the liner **174** may have varying regions of thickness **T1, T2, T3** along proximal portions of the liner relative to distal portions of the liner.

Moreover, to facilitate smooth retraction of the sheath and consistent deployment, the liner **174** may be pleated to fold and collapse in a consistent manner, as shown in the perspective view of Figure 54C. The liner **174** may be pleated, e.g., using a fixture during manufacturing.

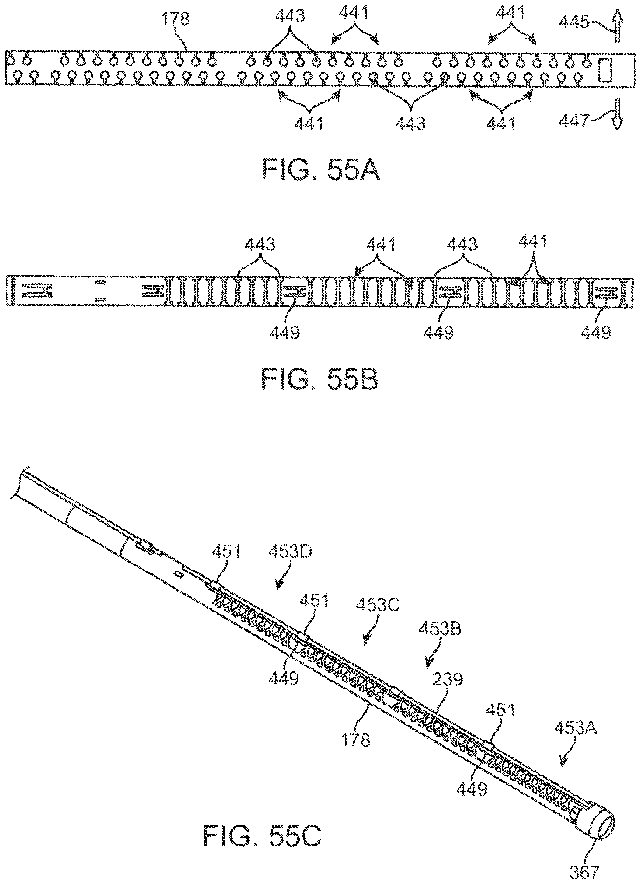

As previously described, the probe **178** may be advanced into and through the patient's cervix **CV** and into the uterus **UT** while conforming to any anatomical features by bending along an anterior direction of flexion **445** or posterior direction of flexion **447** (e.g., up to 90 degrees or more) but may further allow the probe **178** to maintain some degree to rigidity and strength in the transverse plane. The probe **178** may accordingly have a plurality of cut patterns **441,** e.g., laser-cut, along the anterior and posterior surfaces of the probe **178,** as shown in the side and top views of Figures 55A and 55B. These cut patterns **441** may be cut partially through the probe **178** along opposing surfaces, e.g., in an alternating manner, and may further define portions of removed material **443** along the ends of each cut pattern **441.** In addition to the cut patterns **441,** one or more H-slots **449** may also be cut periodically along, e.g., the anterior surface of the probe **178** to allow for anchoring locations for the infusion line **239.**

An example is illustrated in the perspective view of Figure 55C which shows a probe **178** having multiple probe sections **453A, 453B, 453C, 453D** (e.g., four sections in this variation) separated by H-slots **449.** One or more anchors **451** (e.g., collars, clips, etc.) may couple the infusion line **239** to the probe **178** along its anterior surface via the anchors **451** secured to the H-slots **449,** as shown.

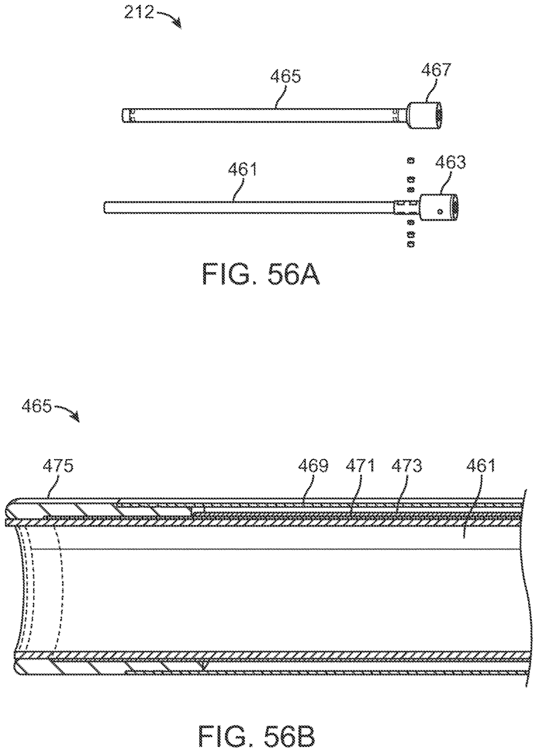

Aside from the liner or balloon itself and the use of balloons for obstructing the os, internal os, and/or external os, as described above, balloons or inflatable liners may also be used to insulate the cryogenic fluid during delivery into the balloon to protect the surrounding tissue structures which are not to be ablated, such as the cervix **CV.** One variation is illustrated in the perspective assembly view of Figure 56A which shows a cervical protection assembly having an inner sheath **461** (e.g., PTFE or other polymer) which may be inserted within an outer sheath **465** (e.g., double walled stainless steel). The inner sheath **461** may have an inner sheath hub **463** at its proximal end which may securely contact an outer sheath hub **467** also located at its proximal end. With the inner sheath **461** inserted entirely within outer sheath **465,** the inner sheath **461** may prevent the liner **174** from inflating into contact against the outer double walled sheath **465.**

As shown in the cross-sectional perspective view of Figure 56B, the double walled outer sheath **465** may have an inner tubular member **471** and a surrounding outer tubular member **469** (e.g., stainless steel, polymer, etc.) forming an insulating annular gap or spacing **473** (e.g., 0.2921 mm (0.0115 in.) spacing). In the event that the tubular members **469, 471** are made of a polymer, the tubular members may be made with a wall thickness of, e.g. 0.00635 to 0.0762 mm (0.00025 to 0.003 in.). The distal end of the outer sheath **465** may have distal tip **475** to maintain the spacing between the tubular members of the outer sheath **465.** With the annular gap or spacing **473** and the presence of the inner sheath **461,** the sheath assembly may provide thermal insulation for the cervical region by insulating the surrounding tissue from the cryogenic fluid or gas passed through the infusion line **239** and from the cryogenic fluid or gas withdrawn through the inner sheath **461** from the liner **174.**

While the annular gap or spacing **473** may have air within the spacing function as an insulator, the spacing may alternatively be evacuated of air to provide for a vacuum insulator. In another alternative, active flow of warmed or ambient temperature fluid (air or gas) may be included between the inner and outer tubular members **469, 471** or insulative materials may instead be placed within the gap or spacing (e.g., inflatable balloons, spiral wound balloons, cotton, wool, synthetic fibers. Neoprene, etc.). In yet another alternative, additional tubular members may be incorporated to create multiple annular gaps.

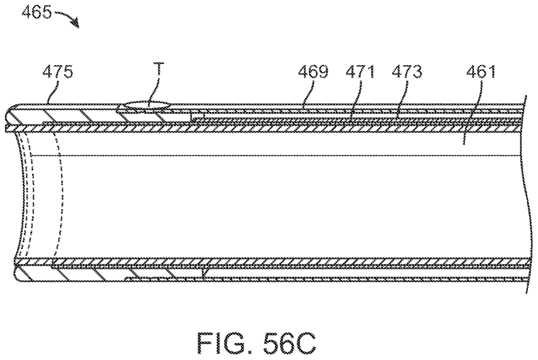

Additionally and/or optionally, the temperature of the sheath **212** may also be monitored so that the temperature may be provided in a feedback loop to a processor or microcontroller for ensuring the surrounding tissue (e.g., cervix) is maintained at a safe level. Accordingly, one or more temperature sensors **T,** e.g., thermocouples, may be placed along the sheath **212,** as shown in Figure 56C, and in communication with the processor or microcontroller. The processor or microcontroller may accordingly be programmed with a feedback loop which could start, pause, or stop the delivery of the cryogenic fluid or gas based upon the temperature (e.g. -89.5 °C for nitrous oxide) of the sheath **212.**

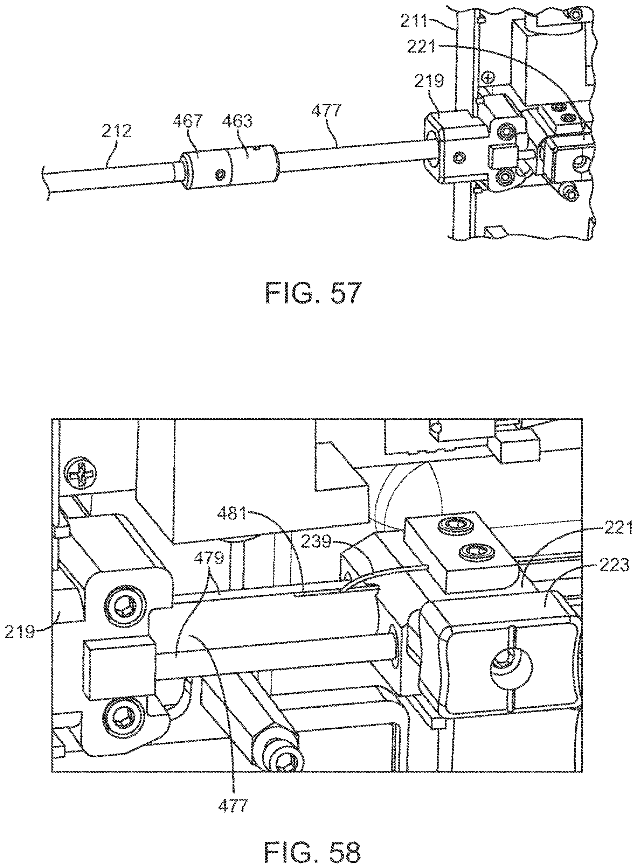

In actuating and controlling the translation of the sheath **212** and probe **178,** the handle assembly **211** may further incorporate a sheath bearing tube **477** coupled to the sheath **212** assembly. Figure 57 illustrates one variation where the sheath bearing tube **477** slidingly pass through a sheath bearing assembly **219** and then attached to a slider base block assembly **221** which is positioned within the handle assembly **211.** The actuatable sheath control **223** may be attached to the slider base block assembly **221** for advancing and retracting the slider base block assembly **221** to control the positioning of the sheath **212** to ensure that the sheath **212** covers the cervical region during a treatment procedure.

Figure 58 shows a detail perspective view of the connection between the sheath bearing tube **477** and slider base block assembly **221.** One or two linear rails **479** may be aligned adjacent to the sheath bearing tube **477** within handle **211** to serve as bearing surfaces for the slider base block assembly **221.** Additionally, a receiving channel **481** may be defined along the anterior surface of the sheath bearing tube **477** to function as an access channel for the infusion line **239** into or along the sheath assembly. Accordingly, as the slider base block assembly **221** is translated linearly along the rails **479,** the sheath bearing tube **477** and infusion line **239** may be advanced distally and/or proximally relative to the probe **178** to control the relative positioning of the sheath **212.**

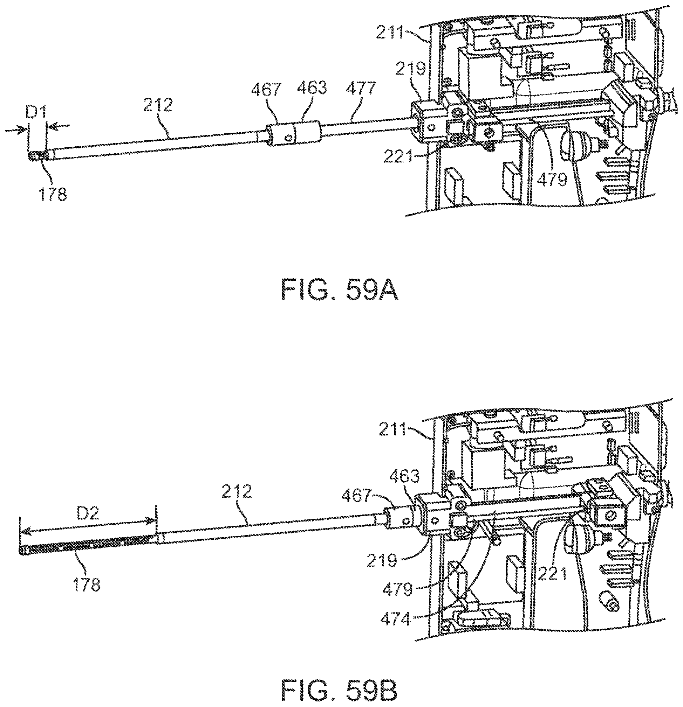

As illustrated in the perspective views of Figures 59A and 59B, when the slider base block assembly **221** is advanced distally relative to the handle assembly **211,** the sheath **212** may be translated distally such that a nominal exposed length **D1** of the probe **178** may be seen. (The liner **174** is not shown for clarity only.) As the slider base block assembly **221** is advanced proximally, the sheath **212** may be accordingly retracted to expose the probe **178** further, as indicated by the exposed length **D2.** The full travel of the sheath and slider base block assembly **221** may range anywhere from, e.g., 1 cm to 8 cm or more, as measured from the distal end of sheath **212** to the distal end of the tip of probe **178.** The positioning of the slider **221** and sheath **212** may be maintained via any number of locking or positioning mechanisms to ensure that the ablation length is maintained during a treatment procedure. Hence, the locking of the slider **221** and sheath **212** may be accomplished by locking mechanisms, e.g., friction fitting, detention features such as notches along the length of the slider travel, grabbing mechanism such as a brake between the slider and handle, actuating features, etc.

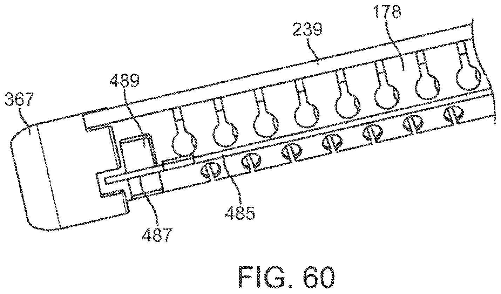

By measuring the pressure decay within the liner **174** during treatment, the rate at which the cryogenic liquid is being converted to a gaseous state may be determined since the lower the cavity pressure the less cryogenic fluid is being converted from liquid to gas. One or more optional pressure sensing lines **485** may be incorporated along the probe **178,** as shown in the perspective view of Figure 60. (A secondary line may be provided for redundant measurement of the pressure.) The pressure sensing line **485** may have a pressure sensor **487** positioned along a cutout window **489** defined along the probe **178** for monitoring the pressure internal to the liner **174** during treatment. The cutout window **489** may be provided to prevent the ends of the pressure lines **485** from contacting the interior of the liner **174.** The pressure lines **485** may be routed along the full length of the sheath **212** and the proximal ends of the lines **485** may be attached to connectors which connect to the pressure sensors via short silicone tube sections.

Additionally, the pressure sensing lines **485** and sensors **487** may also be used as safety feature for the system. The lines **485** and sensors **487** can trigger the actuatable valve **237** which is fluidly coupled to the cryogenic line **235** (shown above in Figure 21E) to close and stop the flow of the cryogenic fluid or gas in the event of pressures detected in the uterine cavity which are higher than expected.

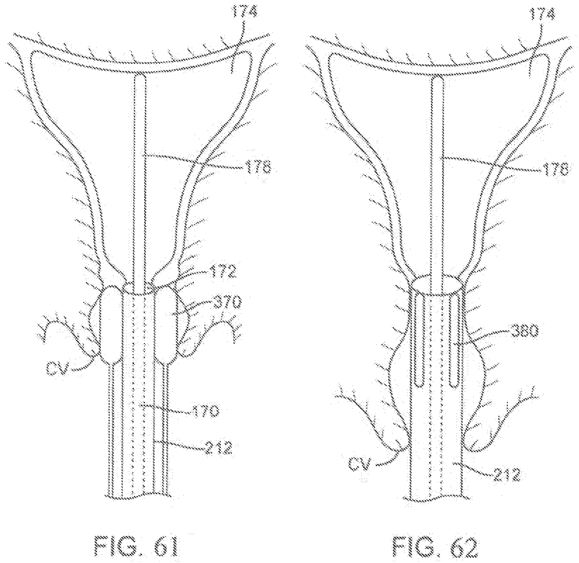

Figure 61 shows a partial cross-sectional of another variation where an inflatable balloon **370** may be located along the outside distal surface of sheath **212** for contacting against and directly insulating the cervix **CV.** The liner or balloon **370** may be filled with a gas or liquid such as air, water, carbon dioxide, etc. to act as an insulator to prevent contact between the delivered cryoablative fluid passing through the shaft **170** and the surrounding cervical tissue. The balloon **370** may be inflated prior to or during an ablation treatment and then deflated once the treatment has been completed to facilitate removal of the device. The size of the balloon **370** may be optionally varied, e.g., by the sheath placement location.

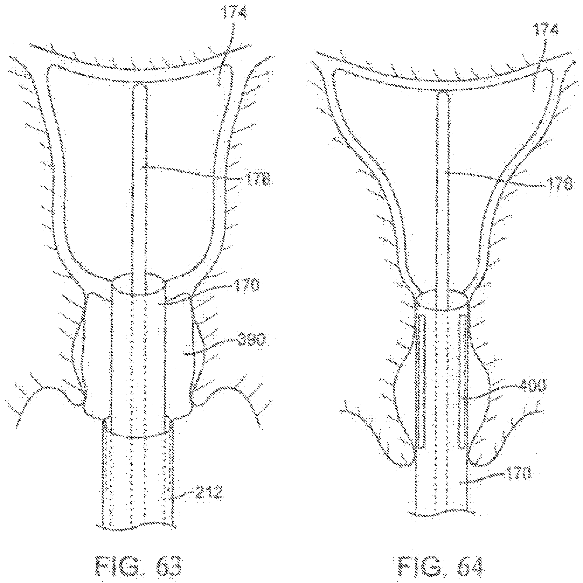

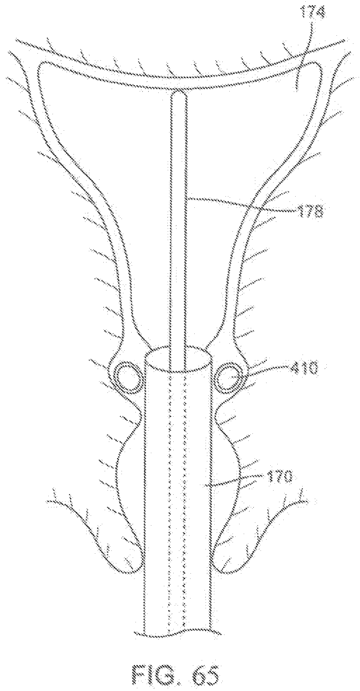

Figure 62 shows a cross-sectional side view of another variation of an inflatable liner or balloon **380** located along the inside distal surface of sheath **212.** In this variation, the balloon **380** may inflate to insulate the cryoablative fluid from the cervical tissue. Figure 63 shows another variation where expandable foam**390** may be deployed via the outer sheath **212** for insulating against the cervix **CV.** Figure 64 shows yet another variation where a heating element **400** may be located along the inner or outer surface of the elongate shaft **170** for heating the surrounding cervical tissue as the cryoablative fluid is delivered during treatment. Figure 65 shows yet another variation where a ring balloon **410** may be inflated along either the sheath **212** or shaft **170** to either insulate the surrounding cervical tissue or to ensure secure placement of the shaft **170** and/or balloon **174** during treatment.

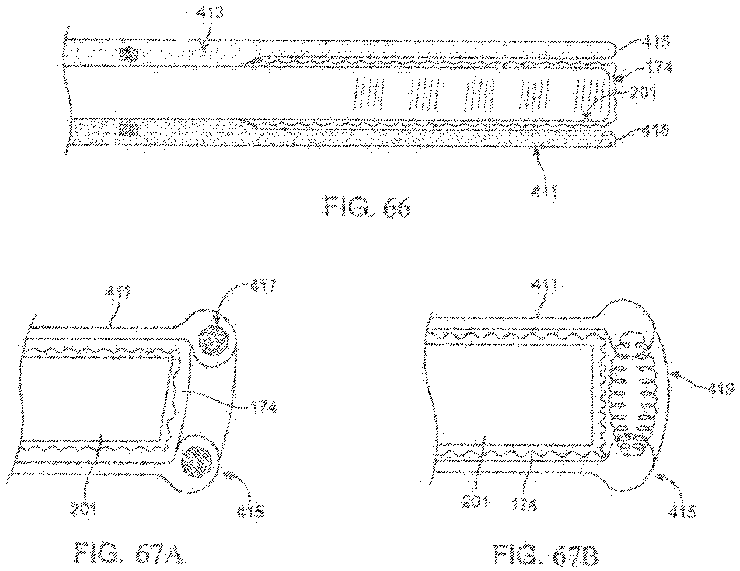

Figure 66 shows a cross-sectional side view of yet another variation of a sheath **411** which may be formed from, e.g., urethane having a thin wall of about 0.0254 mm (0.001 in.), which may be doubled over and sealed such that the sheath **411** contains a volume of liquid or gas **413** such as saline, air, etc. The cooling probe assembly having the tubing **201** and balloon **174** in its collapsed state may also be seen. The sheath distal end **415** may optionally incorporate a deformable member such as an elastic or expandable ring **417** contained circumferentially within the distal end **415,** as shown in the side view of Figure 67A. Alternatively, a biased circular member such as a ring **419** comprised of a circularly-formed spring may be contained circumferentially within the distal end **415,** as shown in Figure 67B. With the sheath **411** positioned with its distal end **415** distal to the tubing **201,** the ring **417** may configure into a ring having a first diameter which at least partially covers the distal opening of the sheath **411.** However, when the tubing **201** is advanced from the sheath **411,** the ring **417** may stretch or deform into a second larger diameter as it conforms to the outer surface of the tubing **201.** The enlarged ring **417** may accordingly form a stop or detent for preventing the proximal over-withdrawal of the sheath **411** relative to the cervix **CV** as well as facilitating the positioning of the sheath **411** over the cervix **CV** to provide insulation during a procedure. As the outer sheath **411** and enlarged ring **417** is positioned proximally along the tubing **201** to secure a position of the ring **417** against cervical tissue, the sheath retraction may accordingly adjust an expanded length of the balloon **174** within the uterus **UT.**

Moreover, since the positioning of the sheath **411** may also actuate and adjust a position of a mandrel **290** within the one or more lines **307** to selectively obstruct or open a selected number of openings **286** (as illustrated in Figure 41), the single withdrawal and positioning of the outer sheath **411** may not only provide an adjustable securement of the device relative to the cervical tissue, but it may also correspondingly adjust the balloon expanded length and further control the active length of the delivery line **307** via the mandrel **290** positioning. The sheath retraction and securement may be utilized not only in this variation, but in any of the variations shown and described herein, as practicable.

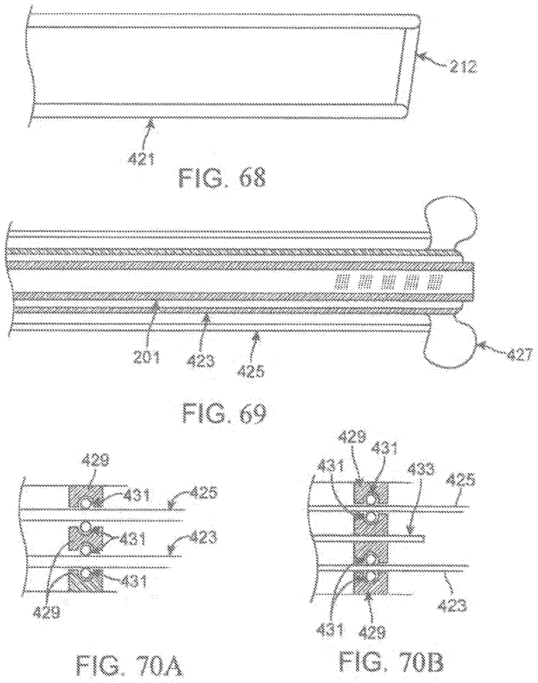

Figure 68 shows another variation of a cervical protection balloon **421** may have a length, e.g., 4 to 8 cm, that may also be positioned along the outside surface of the sheath **212** (as shown) or along the inside surface for placement against the cervical tissue. Figure 69 shows a cross-sectional side view of yet another variation of a dual sheath assembly having an inner sheath **423** and an outer sheath **425** which are longitudinally translatable relative to one another. An annular balloon **427** may be attached to the distal ends of both the inner sheath **423** and outer sheath **425** such that the balloon **427** size and configuration may be altered by the relative movement and positioning of the sheaths **423,425.** Figures 70A and 70B show detail cross-sectional side views of an example of an arrangement for several seals **429** which may be positioned between each respective sheath **423, 425.** Corresponding o-ring seals **431** may be incorporated into the seals **429** to provide for fluid-tight sealing. Also, a fluid line **433** may be passed through one or more seals **429,** as shown, to provide for inflation and deflation of the balloon **174** or annular balloon **427.**

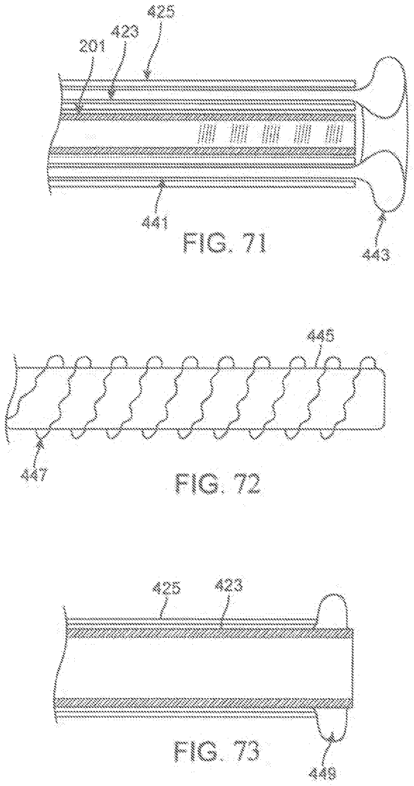

Another variation is shown in the cross-sectional side view of Figure 71 which shows another dual sheath design where the annular balloon may be comprised of a confined balloon **441** having an expandable balloon portion **443.** The balloon, e.g., urethane, may be contained between each respective sheath **423, 425** while a doubledover portion may be positioned to extend from between the distal ends of the sheaths **423, 425.** As inflation fluid is introduced into the balloon, the portion of the balloon constrained between the sheaths **423, 425** may remain collapsed but the unconstrained expandable balloon portion **443** may expand into an annular shape as shown.

Figure 72 shows yet another variation where the sheath **445** may be formed to have a reinforcement member **447,** e.g., wire, braid, mesh, etc., integrated along its body to provide for added strength and space between the sheath **445** and adjacent tissue. Any of the balloon embodiments described herein may be incorporated with the sheath **445** as shown.

Figure 73 shows another variation of a sheath having an annular balloon 449 positioned along the distal end of the inner sheath **423** while constrained by the distal end of the outer sheath **425.** The balloon **449** may be sized according to the relative positioning between the inner and outer sheaths.

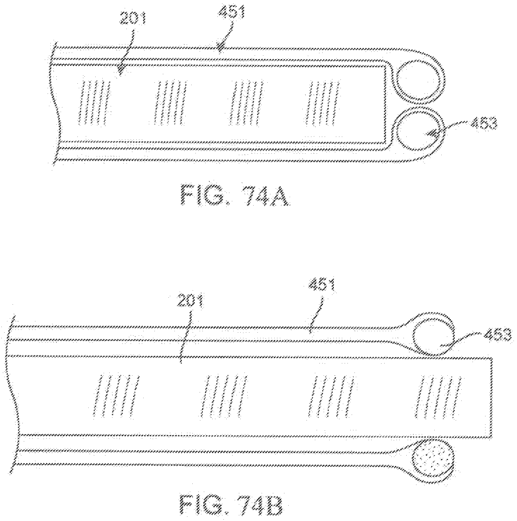

Figures 74A and 74B show partial cross-sectional side views of yet another example of an outer sheath **451** slidably positioned over tubing **201** where the distal end of outer sheath **451** may incorporate an integrated expandable ring **453,** e.g., elastomeric, foam, etc. As previously described in a similar embodiment, the expandable ring **453** may have a first diameter which closes upon the distal end of tubing **201** when the outer sheath **451** is advanced distal to the tubing **201.** As the outer sheath **451** is retracted relative to tubing **201,** the ring **453** may expand to a larger second diameter as it conforms to the outer surface of the tubing **201.** The enlarged profile of the outer sheath **451** may thus function as a stop relative to the cervical tissue during a procedure.

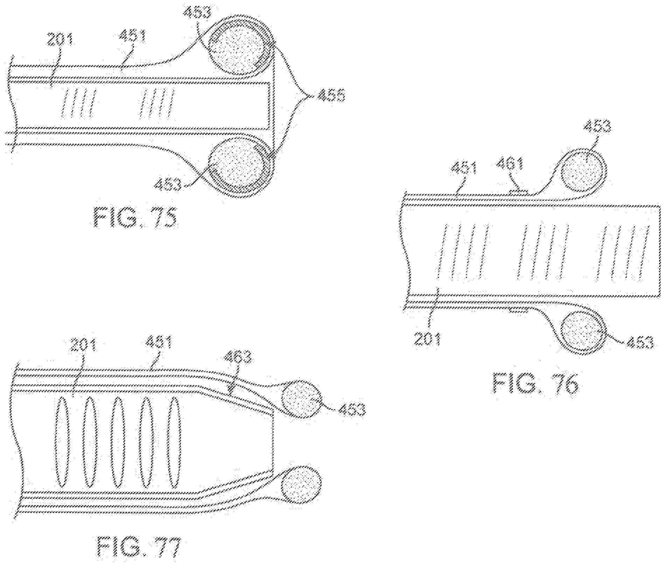

Figure 75 shows a similar variation where the expandable ring **453** may incorporate one or more lubricious surfaces **455** to facilitate the retraction of outer sheath **451,** e.g., by peeling the outer layer relative to the inner layer, and the conformance of the ring **453** relative to the tubing **201.** Figure 76 shows a side view of yet another variation where the outer sheath **451** may instead incorporate a discrete ring section **461** having the expandable ring **453** positioned relative to the tubing **201.** Figure 77 shows yet another variation where the distal end of the tubing **201** may define a tapered distal end **463** to facilitate the expansion of the expandable ring **453** when outer sheath **451** is retracted.

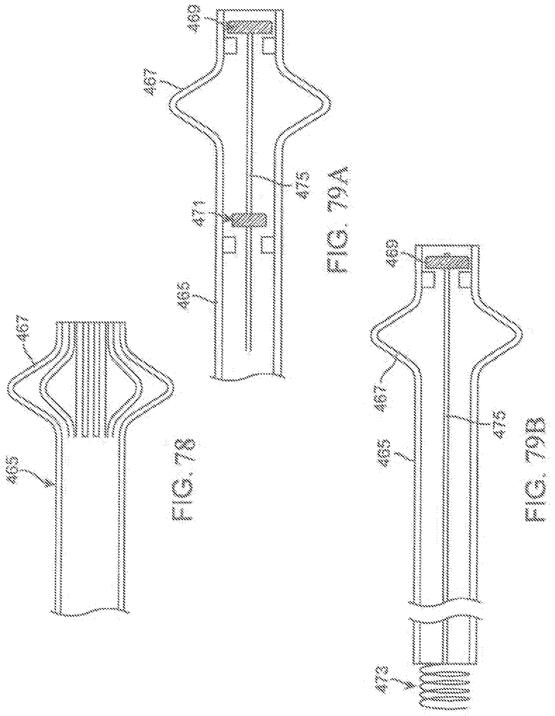

In yet another variation of the outer sheath, Figure 78 shows an embodiment where the outer sheath **465** may have a radially expandable portion **467** formed near or at a distal end of the outer sheath **465.** Prior to or during a procedure to secure a position of the outer sheath **465** relative to the cervical tissue, the expandable portion **467** may be utilized rather than an inflatable balloon. The expandable portion **467** may generally comprise one or more lengths of the outer sheath **465** being reconfigurable along a pivotable or bendable portion such that as the distal end of the outer sheath **465** is retracted relative to the remainder of the sheath **465,** the one or more lengths may pivot and reconfigure into its radial configuration.

A linkage **475** (such as wire, rod, string, ribbon, etc.) may be coupled to the distal end of the outer sheath **465** at a first stop **469,** as shown in the partial cross-sectional side view of Figure 79A. A second stop **471** may be positioned proximally of the first stop **469** which limits the proximal withdrawal of the linkage **475** by a predetermined distance. When the linkage **475** engages the first stop **469** and retracts the sheath distal end to radially extend the expandable portion **467,** the further retraction of linkage **475** may be stopped by the second stop **471.** The outer sheath **465** may define the lumen through which the cooling probe assembly may be advanced without interference from the retraction assembly. Another variation is illustrated in Figure 79B which shows a similar mechanism but where the second stop **471** may be replaced by a biasing element **473,** e.g., spring, positioned proximally of the first stop **469.**

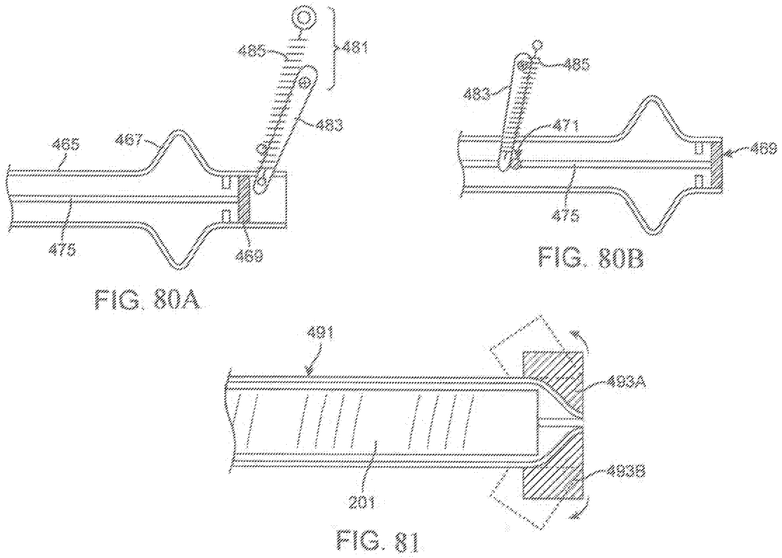

Yet another variation is shown in the side views of Figures 80A and 80B which illustrate a representation of an exemplary overcenter linkage mechanism **481** which may be incorporated with the retraction mechanism. A linkage **483** and corresponding biasing element **485,** e.g., spring, may be coupled to the linkage member **475** attached to the stop **469.** As the linkage **475** is retracted to reconfigure the expandable portion **467,** the overcenter mechanism **481** may also be retracted and actuated to engage a position of the linkage **475** such that the retraction of the expandable portion **467** may be selectively maintained. The overcenter mechanism **481** may be selectively disengaged to release and reconfigure the expandable portion **467.**

Figure 81 shows a side view of yet another variation where the outer sheath **491** may incorporate one or more distal cam members **493A, 493B.** With the outer sheath **491** positioned distally of the tubing **201,** the cam members **493A, 493B** may be configured into a first collapsed configuration. As the outer sheath **491** is retracted relative to tubing **201,** the cam members **493A, 493B** may pivot along outer sheath **491** when urged by the outer surface of the tubing **201** and reconfigure into an expanded configuration as indicated. The reconfigured expanded cam members **493A, 493B** may then be used as a stop for the outer sheath **491** relative to the cervical tissue.

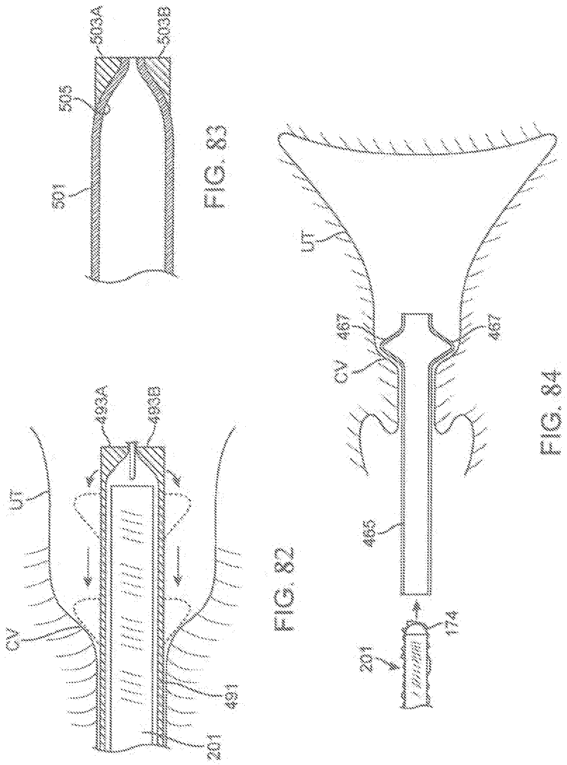

An example of the reconfigured cam members **493A, 493B** used as a stop is illustrated in the exemplary cross-sectional side view of Figure 82. As indicated, as the outer sheath **491** is retracted and the cam members **493A, 493B** reconfigure, the outer sheath **491** may be further retracted until secured against the cervix **CV.** Figure 83 shows another example where the outer sheath **501** having the distal tip cam members **503A, 503B** may be configured to have a tapered distal end **505** to allow for the further pivoting of the cam members **503A, 503B** during sheath retraction.

Figure 84 shows an exemplary illustration of how the outer sheath **465** may be deployed first and secured into position with, e.g., the expandable portion **467,** placed into contact against the cervix **CV.** The cooling probe assembly and collapsed balloon **174** may then be inserted through the outer sheath **465** at a later time and advanced into the uterus **UT** for treatment. In this and any of the other variations described herein, as practicable, the outer sheath may be deployed independently of the cooling probe if so desired.

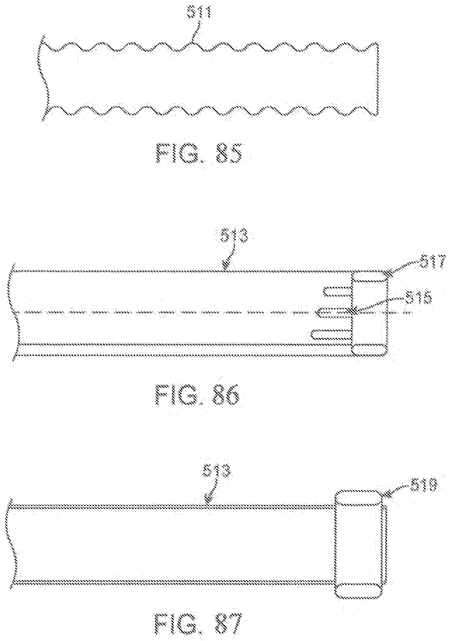

Figure 85 shows yet another variation where the outer sheath may be configured as a corrugated outer sheath **511** to provide a structure which is strong yet flexible. Figures 86 and 87 show additional variations where the outer sheath **513** may comprise an annular balloon **517** located along inner surface of sheath **513.** The sheath distal end may define one or more longitudinal slots **515** for selective expansion of the balloon **517.** Alternatively, the annular balloon **519** may be located along outer surface of sheath **513,** as also previously described.

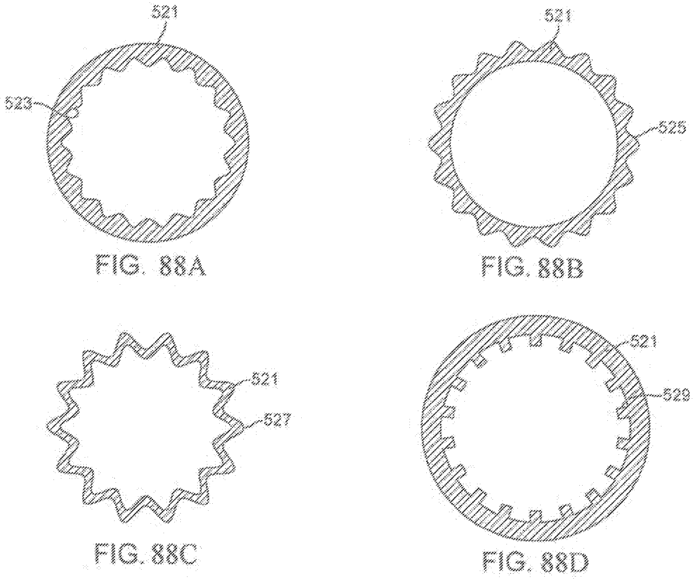

Figures 88A to 88D show yet another variation where the sheath **521** may incorporate an integrated feature to provide further insulation between the cryoablative fluid and the surrounding cervical tissue by creating or forming insulative pockets of air. The variation shown in the cross-sectional end view of Figure 88A shows a sheath **521** defining a plurality of raised and curved surfaces **523** along the inner surface of the sheath **521.** Figure 88B shows another variation where a plurality of raised and curved surfaces **525** may be formed along the outer surface of the sheath **521.** Yet another example is shown in Figure 88C which shows a sheath **521** formed to have both internal and external raised surfaces **527** while the variation of Figure 88D shows a variation where the internal sheath surface may have a plurality of raised projections or fingers extending inwardly.

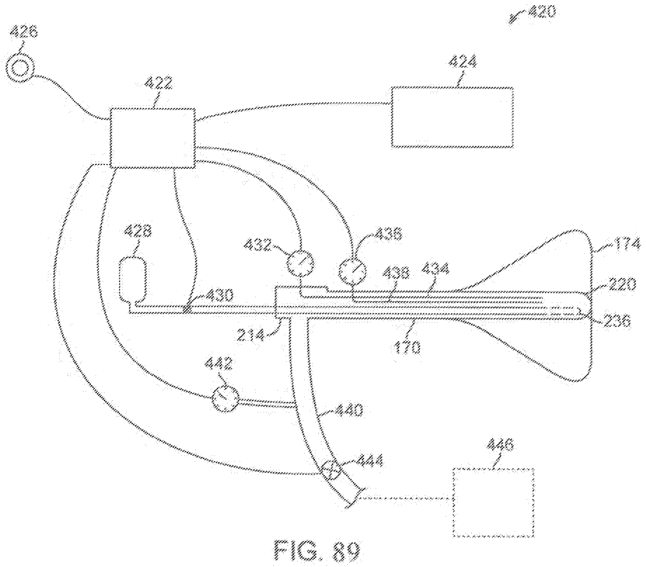

In controlling the ablative treatments described above, the treatment assembly may be integrated into a single cooling system **420,** as shown in the exemplary schematic illustration of Figure 89. The cooling system **420** may be contained entirely within the handle assembly **214** as described above or it may be separated into components, as needed or desired. In either case, the cooling system **420** may generally comprise a microcontroller **422** for monitoring and/or controlling parameters such as cavity temperature, cavity pressure, exhaust pressure, etc. A display **424,** e.g., a digital display which may be located along handle assembly **214,** may be in communication with the microcontroller **422** for displaying parameters such as cavity pressure, cavity temperature, treatment time, etc. Any errors may also be shown on the display **424** as well. A separate indicator **426,** e.g., visual or auditory alarm, may also be in communication with the microcontroller **422** for alerting the user to prompts, errors, etc. through as any number of indicators, symbols, or text, etc., for alerts, as well as for instructions, or other indications.

A coolant reservoir **428,** e.g., nitrous oxide canister in this example, may be fluidly coupled to the handle **214** and/or elongate shaft **170** via a coolant valve **430** which may be optionally controlled by the microcontroller **422.** The coolant reservoir **428** may be in fluid communication with the cooling probe assembly **220** and with the interior of the balloon **174.** One or more pressure sensors **432** may be in communication with a pressure lumen **434** contained within the cooling probe assembly **220** or elongate shaft **170** and one or more temperature sensors **436** in communication with a thermocouple/thermistor wire **438** also contained within the cooling probe assembly **220** or elongate shaft **170** may be incorporated. The one or more pressure sensors **432** and/or temperature sensors **436** may be in communication with the microcontroller **422** as well. Moreover, the pressure sensors **432** may optionally comprise a sensor positioned within the balloon **174** where the sensor is designed for low temperature measurement. Such a pressure sensor may incorporate a closed or open column of liquid (e.g., ethanol, etc.) or gas (e.g., air, carbon dioxide, etc.) which extends through the cooling probe assembly.

The cryoablative fluid contained within the coolant reservoir **428,** such as nitrous oxide, may be pumped (or allowed to flow if reservoir **428** is under pressure) via, e.g., a motor-driven valve such as coolant valve **430,** to control nitrous oxide inflow rate. The valve **430** may also be used to maintain a desired amount of back pressure to separate the walls of the uterus. For instance, a relatively low back pressure of, e.g., 40 to 60 mm Hg, may be used. Alternatively, a simple but precise exhaust flow restriction might be all that is needed, e.g., such as a fixed, non-adjustable valve. In yet another alternative, vacuum pressure may be used to control the rate at which the exhaust gas is pulled-through, e.g., a nitrous oxide deactivation filter.

The rate at which the cryoablative fluid, such as the nitrous oxide, is delivered may be controlled by the temperature measured within the balloon **174** and/or uterine cavity. The target temperature range may range, e.g., between -65 and -80 degrees C. By limiting the temperature measured within the balloon **174** to a value which is lower than the boiling point of nitrous oxide, about -88.5 degrees C, the chance of liquid nitrous oxide build-up in the balloon **174** may be greatly reduced to prevent any excessive intrauterine pressures if the exhaust tube is blocked.

In the event that excessive pressure is measured within the balloon **174** or the pressure differential between two sensors is too large, the system may be programmed to automatically stop the flow of the cryoablative fluid. A separate shut-off valve may be used in-place of the coolant valve **430.** Furthermore, if electrical power is interrupted to the system, the separate shut-off valve may automatically be actuated. In addition, the indicator **426** may signal to the user that excessive pressures were reached and the system shut-down.

The inside diameter of the delivery line may also be sized to deliver cryoablative fluid up to but not exceeding, e.g., a maximum anticipated rate for a large, well-perfuse uterus. By limiting the rate of cryoablative fluid infusion and sizing the exhaust tube appropriately, the system may be able to evacuate the expanded gas even in the event of a catastrophic failure of the delivery line.

Additionally, an exhaust lumen **440** in communication with the elongate probe **170** and having a back pressure valve **444** may also include a pressure sensor **442** where one or both of the back pressure sensor **442** and/or valve **444** may also be in communication with the microcontroller **422.** While the microcontroller **422** may be used to control the pressure of the introduced cryoablative fluid, the pressure of the cryoablative fluid within the balloon **174** interior may also be controlled automatically by the microcontroller **422** adjusting the back pressure valve **444** or by manually adjusting the back pressure valve **444.** In the event that the microcontroller **422** is used to control the back pressure via valve **444,** the microcontroller **422** may be configured or otherwise programmed to adjust the valve **444** based on feedback from other sensors, such as the measured parameters from the one or more pressure sensors **432** and/or temperature sensors **436** to create a closed feedback loop system. A vacuum may also be optionally incorporated into the closed feedback loop.

The exhaust lumen **440** may be fluidly connected, e.g., to a reservoir **446** for collecting or deactivating the exhausted cryoablative fluid. The reservoir **446** may optionally incorporate a filter into the handle **214** or become integrated into a reusable console. Alternatively, the exhausted cryoablative fluid may be simply collected in a reservoir **446** or exhausted into atmosphere.

Generally, redundant pressure lines and sensors, such as pressure lumen **434,** that terminate in the balloon **174** may correspond to sensors located in the handle **214** to make comparison measurements. The pressure lines may be filled with a fluid such as ethanol to prevent freezing during a procedure. Alternatively, a gas such as air may be used in the pressure lines but may utilize temperature compensation.

As at least one thermocouple may be located within the balloon **174** and used to measure temperature during the procedure, additional thermocouples may be optionally included at other locations internal or external to the balloon **174** to provide for additional temperature measurements. For example, a thermocouple may be optionally located on the distal portion of the sheath **212** to monitor temperature within the cervix **CV.**

After completion of the procedure, all unused cryoablative fluid still contained in the reservoir **428** or within the system may be automatically or manually vented, e.g., to the deactivation filter or collection reservoir **446.**

The system **420** may optionally further incorporate an emergency shut-off system which may be actuated in the event that electrical power is lost, if a user manually activates the shut-off system, or in the event that the microcontroller **422** detects a highpressure within the system **420.** One example of the emergency shut-off system may incorporate an emergency shut-off valve which may include valve **430** or which may alternatively incorporate another valve separate from valve **430.** Moreover, in detecting the pressure within the system **420,** a redundant pressure sensor may also be utilized along with the one or more pressure sensors **432** either at the same location or at a different location along the system **420.**

In any of the examples described herein, the system may employ a thermally conductive fluid having a thermal conductivity greater than that of saline. This thermal conductivity may help to ensure that the fluid within the body cavity or lumen is at the same temperature throughout even without agitation or lavage. Such a fluid may be used with the fluid lavage and/or the fluid infusion followed by application of a cryoprobe. The improved thermal conductivity may be achieved via a variety of different options including, but not limited to, choice of a thermally conductive fluid or gel, addition of thermally conductive compounds to the fluid or gel (e.g., metals or metal ions, etc.) and/or agitation of the fluid within the cavity to help achieve equilibration of the temperature. Additionally, the fluid may be infused as a fluid or gel until a set pressure is achieved. The cryoprobe may then be introduced into the body cavity/lumen and heat may be withdrawn from the fluid/gel. Prior to or in concert with the achievement of a cryotherapeutic (ablative or non-ablative) temperature, the fluid or may form a gel or solid. This may be utilized such that fluid or gel within the cavity may be trapped within the target lumen or body cavity with its change in viscosity or state thereby preventing leakage of the fluid or gel and unwanted exposure of adjacent tissues to the cryotherapeutic effect. Due to the higher thermal conductivity or the gelled or frozen fluid or gel, the continued removal of heat from the gelled or frozen mass may be rapidly and uniformly distributed throughout the body cavity or lumen. The solution may also be partially frozen or gelled and then agitated or recirculated to ensure even greater distribution of the cryotherapeutic effect. Moreover, the fluid may also be formulated to have a freezing temperature at the desired ablation temperature such that the fluid remains at the desired ablation temperature for a significant amount of time while the fluid changed from a liquid to a solid or vice versa.

Furthermore, the fluid or gel may be made thermally conductive by the addition of a biocompatible metal or metallic ion. Any metal or conductive material may be used for this purpose, e.g., silver, gold, platinum, titanium, stainless steel, or other metals which are biocompatible. Alternatively the thermally conductive fluid may be used to transmit the thermal energy to tissues in order to provide thermal ablation as opposed to the extraction of energy with cryoablation. In either embodiment, with sufficient thermal conductivity the fluid may act as an extension of the ablative energy source and provide a custom ablation tip for the application of or removal of energy from any body tissues, body cavities, or body lumens. Another benefit is consistency of treatment since cryoablation may require use of ultrasound in the setting of uterine ablation. Any of the devices herein may allow for the use of temperature tracking or simple timed treatment in order to automate the ablation (with or without ultrasound monitoring). For example, application of -80 C for 3 minutes has been shown to provide the correct depth of ablation for many uterine cavities. The devices herein may allow for the tracking of temperature such that once a desired temperature is reached (e.g., -60 C) a timer may be triggered which automatically discontinues therapy and warms the cavity based on time alone. This may be used in the setting of a fixed volume infusion (e.g., 10 to 15 cc of thermally conductive fluid/gel for all patients) or in the setting of infusion of a fluid/gel to a set pressure (with variable volumes). This timed ablation may also be used in concert with any of the device herein to allow for elimination of the burdensome requirement for ultrasound tracking of the cryogenically treated regions.

Alternatively, this thermally conducting fluid (which may optionally include solid particles of metal) may be infused into a balloon which conforms to the uterus, esophagus or other body cavity or lumen at relatively low pressures (e.g., less than 150 mmHg), as also described above. The thermally conducting material may alternatively be comprised entirely of a solid (e.g., copper spheres or a copper chain) within the conforming balloon wherein the thermally conductive solid and/or fluid may be reversibly delivered into the conforming balloon under low pressure after which a cryoprobe, cryogenic liquid and/or cryogenic gas may be delivered into the balloon and activated to ablate the entirety of the uterus **UT** at once. The cryogen source may also be positioned within the balloon to obtain maximum cryoablation within the body of uterus with less ablative effect proximally and in the cornua. Vaseline, oils or other thermally resistive materials may also be used in conjunction with this or other modalities in order to protect certain areas of the uterus, cervix and vagina.

In creating the optimal thermally conductive fluid and/or gel, any conductive material may be added to the fluid or gel including, e.g., gold, silver, platinum, steel, iron, titanium, copper or any other conductive metal, ion, or molecule. If a metal is used as a dopant to increase the thermal conductivity, the added metal may take any shape or form including spheres, rods, powder, nanofibers, nanotubes, nanospheres, thin filaments or any other shape that may be suspended in a solution or gel. The fluid or gel may itself also be thermally conductive and may be infused and then removed or may be left in the cavity and allowed to flow naturally from the uterus as with normal menstruation. The thermally conductive polymer may also be biocompatible, as well, but this may not be necessary if the fluid/gel is extracted immediately following the procedure.

Despite the potential for toxicity, ethanol may be well suited for a liquid lavage in that it resists freezing down to -110 C and is, other than dose dependent toxicity, biocompatible. Solutions of 75% to 99.9% ethanol concentrations may be used to good effect and have been demonstrated to show that a freeze layer develops very rapidly inhibiting further ethanol absorption. An ethanol copper composition may also be used since ethanol resists freezing whereas aqueous fluids freeze and expand thereby moving the metal particle out of direct contact with the tissue.

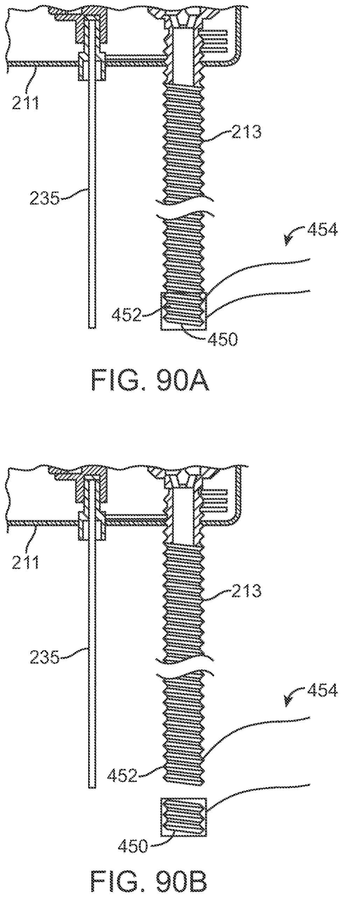

In another variation for creating back pressure within the system, Figures 90A and 90B show a device for closing the exhaust flow path to facilitate a liner integrity check and to also increase the pressure within the uterine cavity. As the liner is initially expanded (e.g., up to 100 mmHg of pressure), the transition between initial expansion and treatment should desirably occur with a minimal change in cavity pressure. Hence, the pressure within the liner and system during the first 30 seconds of treatment is ideally maintained 85 +/- 15 mmHg. The variation shown in Figure 90A illustrates an end cap **450** which may be used to cover or obstruct the exhaust line **213** to facilitate the increase in back pressure during the initial expansion. The end cap **450** may contact the exhaust tube contact **452** over the exhaust line **213** such that the end cap **450** and exhaust tube contact **452** are electrically coupled to form a circuit via conductive lines **454.**

Once sufficient back pressure has been created in the system and cryoablation treatment is ready to begin, the end cap **450** may be removed from the exhaust tube contact **452** (manually or automatically) breaking the electrical connection, as shown in Figure 90B. The processor or microcontroller in the system may detect the electrical break and automatically initiate infusion of the cryoablative fluid or gas to begin the treatment. Moreover, because the exhaust line **213** is now unobstructed, the discharged fluid or gas may also be vented or removed from the system through exhaust line **213.**

In other variations for maintaining exhaust back pressure within the system, the pump (with or without a filter) may be used to increase the pressure. Alternatively, a compressed gas (e.g., coupled via a bladder, tank, etc.) may be infused into the liner and system to increase the pressure. In either case, any of these methods may be used in various combinations with any of the system or device variations described herein.

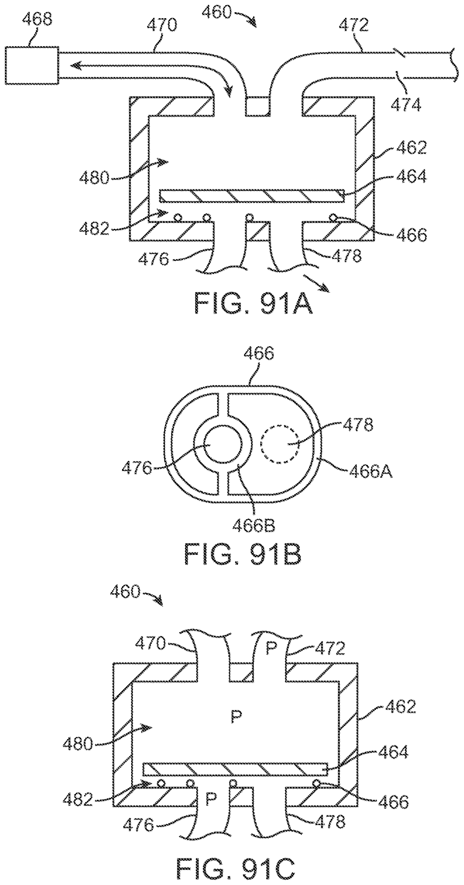

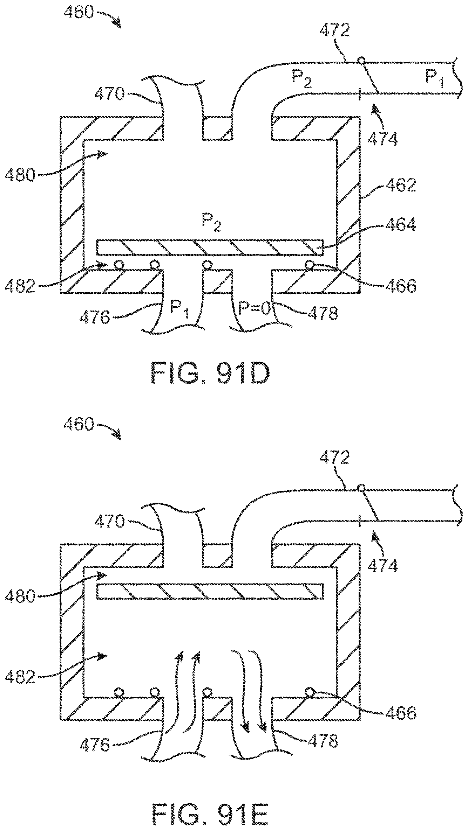

Alternative mechanisms may also be used for maintaining a sufficient back pressure within the system and the liner. Another variation is shown in the schematic side view of Figure 91A of flapper valve **460** such as an electromechanical flapper valve which may be incorporated within the handle assembly **211** (e.g., positioned between the pump and the liner) for fluidly coupling to the liner and exhaust. Figure 91 illustratively shows flapper valve **460** having a chamber **462** with a translating piston **464** within the chamber **462.** The pump **468** may be fluidly coupled via pump line **470** to the first section **480** of the chamber **462** (e.g., at a top of the chamber **462)** and exhaust and/or liner channel **472** may also be similarly fluidly coupled to the first section **480** of chamber **462.** The exhaust and/or liner channel **472** may also incorporate a unidirectional valve **474** within the channel **472** such that fluid or gas may pass through channel **472** and past unidirectional valve **474** but is prevented from flowing back into first chamber **480.**

The exhaust and/or liner channel **476** may be fluidly coupled to the second section **482** of chamber **462** (e.g., at the bottom of the chamber **462)** in proximity to ambient line **478.** A sealing gasket **466** having a first sealing ring **466A** which encircles the periphery of the chamber **462** and a second sealing ring **466B** which encircles the entry to the exhaust and/or liner channel **476** may also be incorporated in the second chamber **482,** as shown in the top view of Figure 91B.

The flapper valve **460** may remain closed by the system (e.g., motor, linear actuator, magnetic, electromagnetically, etc.) but opened by the system as the ablation treatment begins. The flapper valve may be actuated, e.g., by a separate button or actuator, positioned upon the handle assembly **211.**

When actuated, the pump **468** may be turned on and the piston **464** may remain positioned at the bottom of the chamber **462** upon the sealing gasket **466,** as shown in the side view of Figure 91C. The piston **464** may form a moderate seal while sitting upon the gasket **466** but as the pressure in the chamber **462** is increased, the sealing between the piston **464** and gasket **466** may be increased accordingly. The gasket **466** itself may form different regions of relatively low pressure in the area between the first sealing ring **466A** and the second sealing ring **466B** (e.g., at atmospheric pressure) and high pressure in the area surrounded by the second sealing ring **466B** (e.g., at the same pressure as within the liner/exhaust) during the initial pump up phase.

After the liner has been pumped up to its pre-determined pressure level to properly distend the cavity and deploy the liner (e.g., 85 mmHg), the pump **468** may be reversed to remove the pressure from the chamber **462.** The one-way or uni-directional valve **474** may prevent the vacuum from pulling on the liner as would the seal at the bottom of the chamber **462** formed by the piston **464.**

When **P2** is lowered, the force **of P1** upon the bottom of the piston **464** may cause it to lift within the chamber **462,** as shown in Figure 91D. This piston movement will cause **P1** to drop as the seal at the bottom breaks and as **P1** begins to drop, the vacuum may shut off and the treatment may be initiated. The flow of the cryogenic fluid or gas through the chamber **462** may cause the piston **464** to move to the top of the chamber **462** which may allow for the free flow of nitrous to the environment, as shown in Figure 91E.

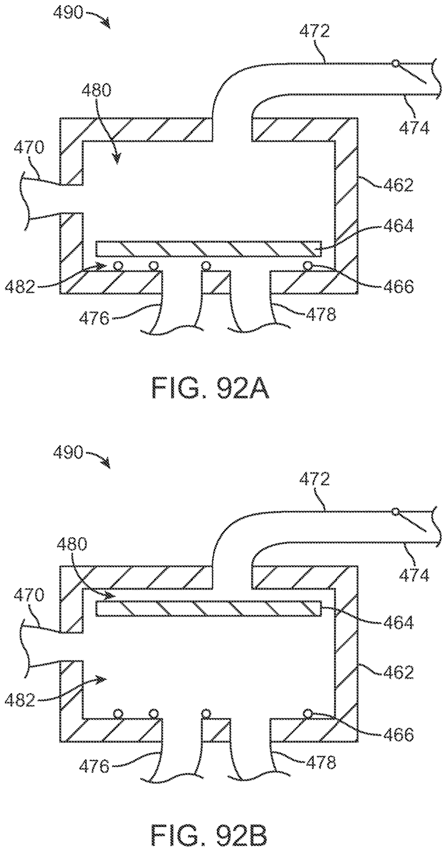

Figures 92A and 92B show an alternative flapper valve **490** which may function similarly except for the pump/vacuum line **470** may be positioned along a side wall of the chamber **462.** With the vacuum/pump line **470** so positioned, the line **470** may remain below the piston **464** while the cryogenic fluid or gas flows through the chamber **462,** as shown in Figure 92B. If the back pressure in the system is too high, the pump or a vacuum pump **468** may be actuated to provide a slightly negative pressure or suction that may lower the back pressure on the system.

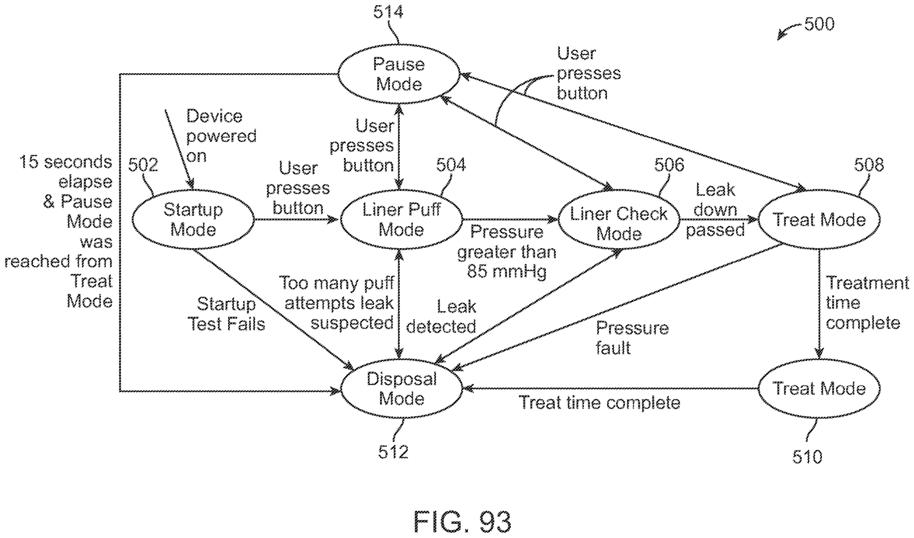

As previously described, the system may be programmed to enable a number of different processes. The processor or microcontroller may be accordingly programmed with a number of different algorithms depending on the functional mode to be performed. Figure 93 illustrates how the different algorithms **500** may be programmed into the processor or microcontroller and how they may functionally interact with one another. Once the device is initially powered on, the system may enter a Start-Up Mode **502** where the system performs a self-check of the sensors and the overall system. Some of the checks may include, e.g., ensuring pressure sensors have a detected initial reading of between 0 and 10 mmHg which do not deviate from one another by more than 5 mmHg; detecting a battery voltage reading of greater than 7.5 V; and/or detecting any memory feature which may be used to record various readings and parameters as well as load any number of additional features into the system.

Once the Start-Up Mode **502** has been initiated and/or completed, the user may, e.g., actuate the system to enter a Liner Puff Mode **504.** In Liner Puff Mode **504,** the system may slowly add ambient filtered air into the liner until target treatment pressure is reached, as described above. The pump may be turned on until the pressure has increased by, e.g., 5 mmHg, and may then be shutoff for, e.g., 2 seconds. The pump may cycle on and off until the system measures, e.g., 85 mmHg. Typically, the full ramp-up may take, e.g., 15-20 seconds. When 85 mmHg is reached, the device may then enter Liner Check Mode **506.** In this mode, the system may detect for leaks in the system. The system may waits for, e.g., 5 seconds, after Liner Puff Mode **504** is complete and then detect whether the pressure is still above, e.g., 40 mmHg or more. If the system is holding pressure, the device may then enter Treat Mode **508.**

During Treat Mode **508,** the system may deliver the cryogenic fluid or gas to the liner while looking for pressure faults. An example of a pressure fault may include a detected pressure value greater than, e.g., 150 mmHg. The treatment may be stopped automatically and user restart may be halted at least temporarily by the system. Another example of a pressure fault may include when a detected pressure deviates from other pressure readings by, e.g., 20 mmHg or greater for 3 consecutive seconds. Again, treatment may be stopped automatically and user restart may be halted at least temporarily by the system. Once a predetermined time period has elapsed, e.g., 2 minutes and 30 seconds, the device may enter Thaw Mode **510.**

In Thaw Mode **510,** the system may push puffs of ambient filtered air into the liner to warm it up for removal from the patient. The pump may push air into the cavity while the exhaust solenoid is closed until, e.g., 10 mmHg pressure in the cavity is measured. The pump may be then turned off and the exhaust solenoid opened for, e.g., 2 seconds to allow the air to escape the cavity. The pump/open cycle may be repeated for, e.g., 2 minutes. Additionally and/or optionally, a vacuum may also be used in Thaw Mode **510,** e.g., to facilitate peeling of the liner **174** from the frozen uterine walls and to expedite the removal of the device.

Once Thaw Mode **510** has been completed, the system may then enter Disposal Mode **512** where the liner and system may be evacuated of any cryogenic fluid or gas. In the event that any of the different modes detects a failure, e.g., if the Startup Mode **502** fails, too many puff attempts are detected during the Liner Puff Mode **504,** a leak is detected during the Liner Check Mode **506,** or a pressure fault is detected by the system during the Treat Mode **508,** then the system may automatically default into entering the Disposal Mode **512.**

In the event that the user pauses the system anytime during the Liner Puff Mode **504** or the Liner Check Mode **506,** the system may enter into the Pause Mode **514** where the system releases pressure and waits indefinitely for the user to re-initiate the puff up sequence by initiating the system again. In the event that the user pauses the system anytime during the Treat Mode **508,** the user may have a predetermined period of time, e.g., 15 seconds, to re-start treatment or the system may stop entirely. The time limit may be imposed so that the frozen tissue does not thaw significantly which could affect the ultimate depth of ablation. When treatment is re-started, the treatment time resumes from where it was paused.

Additionally and/or optionally, the processor or microcontroller may also be programmed to monitor the overall system. Hence, the system may be programmed to transmit a signal on a regular basis, e.g., every second, to a monitoring system. If the monitoring system fails to receive the signal, the entire system may be shut down such that the valves automatically close, the exhaust valve opens, and the pumps shut down.

Additional features may also be incorporated for safety. For instance, in order to prevent high pressure from developing within the uterus due to the accumulation of the cryogenic fluid or gas, safety features may be integrated into the system. Examples of some of the safety features may include: redundant, kink-resistant pressure lines and sensors to monitor intrauterine cavity pressure; flow rate-limited delivery line to minimize the amount of cryogenic fluid or gas introduced into the cavity in the event of a catastrophic failure; pressure relief valve in the exhaust flow path which opens if the primary path is obstructed; flexible yet crush-resistant, laser-cut, stainless steel exhaust tube within the liner; fail-safe solenoid valve on the cryogenic fluid or gas inflow path which closes in the event of a power failure or other control system failure; and/or software watchdog to safely shutdown the device in the event of a software malfunction.

Moreover, in order to prevent cryogenic fluid or gas in the exhaust from coming into contact with the physician or patient, additional safety provisions may also be implemented. Examples of some of these safety provisions may include: heat sink in the exhaust pathway to promote the conversion of cryogenic fluid or gas from liquid to gaseous states; and/or convoluted tube at the end of the exhaust path to further promote cryogenic fluid conversion from liquid to gaseous states and also prevent cryogenic fluid or gas from contacting the physician or patient.

As described herein, various visualization modalities may be utilized with any of the system variations described. For instance, hysteroscopic visualization may be accomplished by utilizing scope having, e.g., a 2.9 mm outer diameter and 39 cm length. Such a scope may be introduced through, e.g., the handle assembly **211,** through a seal that interfaces with the scope and allows for the inflation of the liner with the scope in place if a more broad view of the cavity is desired. The scope may be removed before treatment is initiated.

Additionally and/or alternatively, ultrasound visualization may also be utilized to aid the user in the placement of the device within the cavity. During treatment, the cryogenic fluid or gas is visible under ultrasound as is the ice front.

While the treatment assembly may be comprised of a completely disposable handheld device which is provided sterile to the user, the cryogenic fluid or gas, e.g., liquid N₂O, may be contained in a canister which is integrated into the device. Alternatively, the assembly may be tethered to a reusable liquid N₂O tank. Electronic components may be relocated from the handheld device to a reusable console which could also contain the liquid N₂O tank. In another alternative, a simple disposable element of the system may attach to a reusable handle tethered to a liquid N₂O tank. The reusable handle could be re-sterilizable and may contain the majority of the components normally contained within the disposable handle. The disposable element could consist of the flexible probe, liner, pressure-sensing lumens and the N₂O delivery line.

While the system is described above for the cryoablative treatment of the uterine cavity, the system may also be used for other applications. For instance, the system may be utilized for shrinking or killing uterine fibroids where the probe may be inserted and the liner inflated with gas, as previously described, and a visualization element (ultrasound or hysteroscopy) may be used to position a freezing element up against the fibroid and freezing may be initiated. The freezing element may be positioned against the endometrial wall or may be inserted into the fibroid itself. The time of the freeze may be determined based upon the size of the fibroid with longer freezes for larger fibroids.

In addition to the initial positioning, the freeze may also be tracked using ultrasound, hysteroscopy or other visualization tools and stopped once a sufficient freeze has been achieved. The cryogen may be infused into a small balloon or liner inflated within the larger, gas-filled liner which may extract energy across both liners from the fibroid. The cryogen transmitting lumen can also be flexibly directed to the surface of the fibroid under visualization and the cryogen transmission balloon can be sized to best fit the endometrial surface of the fibroid and the freeze can be initiated and tracked under visualization. The small cryogen transmission balloon may also be replaced with a metal or other thermally conductive tip in which the cryogen undergoes a phase change and/or extracts energy. The gas-filled liner will allow for good visualization of the uterus and the freezing probe. In alternative variations, hyperthermal or other destructive energy may also be transmitted across the liner in order to destroy the fibroid with the liner simply acting as a source of controlled visualization without the introduction of saline or other distention media.

In yet another application, the system may be used to treat conditions such as Barrett's Esophagus. The probe may be inserted under endoscopic visualization and the liner sized to the length of the esophagus to be treated. The liner may then be inflated and visualization may be repeated across the liner to ensure optimal distention and contact with tissues to be treated. The cryogen delivery probe may then deliver a liquid cryogen or, preferably, a cold gas to the interior of the liner to treat the tissues adjacent to the liner. If needed or desired, the liner may then be deflated, repositioned and cryogen may be reinfused one or more times. This may be particularly the case for a relatively smaller esophagus in which the oversized liner may have more extreme folds which could prevent energy transmission. Repositioning and redeployment of the liner may allow for these folds to occur in different areas and will allow for a more consistent ablation. The use of cold gas may also allow for a more consistent, light ablation, as well, due to the smaller gradient in temperature and lack of the powerful phase change. Benefits of this design include the ability to prevent overlapping circumferential ablations which have been found to cause strictures and stenosis. Additional benefit can be found from the consistent treatment of all or part of the esophagus, including the gastroesophageal junction.

In yet another application, the system may be used to treat conditions such as treating benign prostatic hyperplasia (BPH) by shrinking the prostrate. The prostate tissue is sensitive to cryotherapy as evidenced by the efficacy of cryogenic freezing of the prostate for oncology. In this variation of the device, the liner may be placed in the urethra in the region of the prostate and cryogen may be infused (liquid or gas) into the liner. The energy may be transmitted across the wall of the liner and the urethra into the prostate causing apoptosis and death of the prostatic cells that are generating the symptoms of benign prostatic hyperplasia. The urethra may be temporarily stented open to allow for epithelialization without stricture formation either before or after the therapy. The stent could be configured to degrade over time, pass on its own and provide symptomatic relief and protection from urine during the healing period (i.e., be an occlusive barrier).

Due to the ease of visualization of the freeze, this therapy may also be conducted under direct visualization to ensure optimal freezing. This freeze may be stopped and/or restarted by the user, but may optimally be a programmed time or time at temperature. The therapy may also be performed without visualization in which case the freeze probe may be inserted with a location indicator (i.e., a balloon that is inflated in the bladder and drawn back to the urethral outlet) and therapy initiated once the correct position has been obtained.

While illustrative examples are described above, it will be apparent to one skilled in the art that various changes and modifications may be made therein. Moreover, various apparatus or procedures described above are also intended to be utilized in combination with one another, as practicable. The appended claims are intended to cover all such changes and modifications that fall within the scope of the invention.

## Claims

1. A tissue treatment system (420), comprising:
an elongate probe (170) having a distal tip (226) and a flexible length (12);
at least one infusion lumen (18, 239) positioned through or along the elongate probe, wherein the infusion lumen defines one or more openings (194, 260) along its length;
at least one delivery lumen (20, 365) slidingly positioned through or along the infusion lumen; and
a liner (22, 24, 174) expandably enclosing the probe such that a cryoablative fluid introduced through the unobstructed openings is sprayed into contact with an interior surface of the liner and coats the interior surface, **characterized in that**:
translation of the delivery lumen (20, 365) relative to the infusion lumen (18, 239) controls a number of unobstructed openings (194, 260) along the infusion lumen via a positioning of the delivery lumen obstructing a selected number of openings along the infusion lumen, such that proximal retraction of the delivery lumen relative to the infusion lumen from a first location increases the number of unobstructed openings, and distal translation of the delivery lumen relative to the infusion lumen from the first location decreases the number of unobstructed openings.

2. The system of claim 1 wherein the elongate probe (170) defines an exhaust lumen for an ablative fluid.

3. The system of claim 1 wherein the infusion lumen (18, 239) and delivery lumen (20, 365) are in fluid communication with one another.

4. The system of claim 1 wherein the one or more openings (194, 260) along the infusion lumen (18, 239) are defined opposite to one another along the infusion lumen.

5. The system of claim 1 wherein the delivery lumen (20, 365) comprises a nitinol delivery tube slidingly positioned through the infusion lumen (18, 239).

6. The system of claim 1 further comprising a reservoir (428) of the cryoablative fluid in fluid communication with the delivery lumen (20, 365).

7. The system of claim 1 wherein the cryoablative fluid comprises nitrous oxide.

8. The system of claim 1 wherein the elongate probe (170) defines an active treatment portion near or at the distal tip (226).

9. The system of claim 1 further comprising a sheath assembly (212) slidably positioned over the elongate probe (170).

10. The system of claim 9 wherein the sheath assembly (212) comprises an inner sheath (461) and an outer sheath (425) defining an annular spacing or gap.

11. The system of claim 1 further comprising one or more transmitters (381) and receivers (383) positioned upon the elongate probe (170).

12. The system of claim 1 further comprising a pump (225) in fluid communication with the liner interior.

13. The system of claim 12 further comprising a valve (405) fluidly coupled to the pump (225) and which is reconfigurable to divert positive and negative pressure from the pump to the liner interior.

14. The system of claim 1 further comprising one or more pressure sensors (487) along the elongate probe.

15. The system of claim 1 wherein the liner comprises at least two tapered portions (176) extending from the distal end of the elongate probe (170) such that the tapered portions are configured to contact a corresponding uterine comu.

## Patentansprüche

1. Gewebebehandlungssystem (420), umfassend:
eine längliche Sonde (170) mit einer distalen Spitze (226) und einer flexiblen Länge (12);
wenigstens ein Infusionslumen (18, 239), das durch oder entlang der länglichen Sonde angeordnet ist, wobei das Infusionslumen eine oder mehrere Öffnungen (194, 260) entlang seiner Länge definiert;
wenigstens ein Abgabelumen (20, 365), das gleitend durch oder entlang des länglichen Lumens positioniert ist; und
eine Auskleidung (22, 24, 174), die die Sonde ausdehnbar umschließt, sodass ein kryoablatives Fluid, das durch die unversperrten Öffnungen eingeführt wird, in Kontakt mit einer Innenraumoberfläche der Auskleidung gesprüht wird und die Innenraumoberfläche beschichtet, **dadurch gekennzeichnet, dass**:
eine Translation des Abgabelumens (20, 365) relativ zu dem Infusionslumen (18, 239) eine Anzahl von unversperrten Öffnungen (194, 260) entlang des Infusionslumens über eine Positionierung des Abgabelumens steuert, die eine ausgewählte Anzahl von Öffnungen entlang des Infusionslumens versperrt, sodass die proximale Retraktion des Abgabelumens relativ zu dem Infusionslumen von einer ersten Stelle aus die Anzahl der unversperrten Öffnungen erhöht und die distale Translation des Abgabelumens relativ zu dem Infusionslumen von der ersten Stelle aus die Anzahl der unversperrten Öffnungen verringert.

2. System nach Anspruch 1, wobei die längliche Sonde (170) ein Auslasslumen für ein ablatives Fluid definiert.

3. System nach Anspruch 1, wobei das Infusionslumen (18, 239) und das Abgabelumen (20, 365) in Fluidverbindung miteinander stehen.

4. System nach Anspruch 1, wobei die eine oder die mehreren Öffnungen (194, 260) entlang des Infusionslumens (18, 239) einander gegenüberliegend entlang des Infusionslumens definiert sind.

5. System nach Anspruch 1, wobei das Abgabelumen (20, 365) einen Nitinolabgabeschlauch umfasst, der gleitend durch das Infusionslumen (18, 239) positioniert ist.

6. System nach Anspruch 1, ferner umfassend ein Reservoir (428) für das kryoablative Fluid, das in Fluidverbindung mit dem Abgabelumen (20, 365) steht.

7. System nach Anspruch 1, wobei das kryoablative Fluid Distickstoffoxid umfasst.

8. System nach Anspruch 1, wobei die längliche Sonde (170) einen aktiven Behandlungsabschnitt nahe oder an der distalen Spitze (226) definiert.

9. System nach Anspruch 1, ferner umfassend eine Hüllenbaugruppe (212), die gleitend über der länglichen Sonde (170) positioniert ist.

10. System nach Anspruch 9, wobei die Hüllenbaugruppe (212) eine innere Hülle (461) und eine äußere Hülle (425) umfasst, die einen ringförmigen Abstand oder Spalt definieren.

11. System nach Anspruch 1, ferner umfassend einen oder mehrere Sender (381) und Empfänger (383), die auf der länglichen Sonde (170) positioniert sind.

12. System nach Anspruch 1, ferner umfassend eine Pumpe (225), die in Fluidverbindung mit dem Auskleidungsinnenraum steht.

13. System nach Anspruch 12, ferner umfassend ein Ventil (405), das mit der Pumpe (225) fluidisch gekoppelt ist und das rekonfigurierbar ist, um positiven und negativen Druck von der Pumpe zu dem Auskleidungsinnenraum zu leiten.

14. System nach Anspruch 1, ferner umfassend einen oder mehrere Drucksensoren (487) entlang der länglichen Sonde.

15. System nach Anspruch 1, wobei die Auskleidung wenigstens zwei sich verjüngende Abschnitte (176) umfasst, die sich von dem distalen Ende der länglichen Sonde (170) erstrecken, sodass die sich verjüngenden Abschnitte dazu konfiguriert sind, ein entsprechendes Gebärmutterhorn zu berühren.

## Revendications

1. Système de traitement de tissus (420), comprenant :
une sonde allongée (170) ayant une pointe distale (226) et une longueur flexible (12) ;
au moins une lumière de perfusion (18, 239) positionnée à travers ou le long de la sonde allongée, la lumière de perfusion définissant une ou plusieurs ouvertures (194, 260) sur sa longueur ;
au moins une lumière d'administration (20, 365) positionnée de manière coulissante à travers ou le long de la lumière de perfusion ; et
un revêtement (22, 24, 174) enfermant de manière expansible la sonde de telle sorte qu'un fluide cryoablatif introduit à travers les ouvertures non obstruées soit pulvérisé en contact avec une surface intérieure du revêtement et recouvre la surface intérieure, **caractérisé en ce que** :
la translation de la lumière d'administration (20, 365) par rapport à la lumière de perfusion (18, 239) contrôle un certain nombre d'ouvertures non obstruées (194, 260) le long de la lumière de perfusion via un positionnement de la lumière d'administration obstruant un nombre sélectionné d'ouvertures le long de la lumière de perfusion, de telle sorte que la rétraction proximale de la lumière d'administration par rapport à la lumière de perfusion à partir d'un premier emplacement augmente le nombre d'ouvertures non obstruées, et la translation distale de la lumière d'administration par rapport à la lumière de perfusion à partir du premier emplacement diminue le nombre d'ouvertures non obstruées.

2. Système selon la revendication 1, dans lequel la sonde allongée (170) définit une lumière d'échappement pour un fluide ablatif.

3. Système selon la revendication 1, dans lequel la lumière de perfusion (18, 239) et la lumière d'administration (20, 365) sont en communication fluidique l'une avec l'autre.

4. Système selon la revendication 1, dans lequel l'une ou plusieurs ouvertures (194, 260) le long de la lumière de perfusion (18, 239) sont définies à l'opposé les unes des autres le long de la lumière de perfusion.

5. Système selon la revendication 1, dans lequel la lumière d'administration (20, 365) comprend un tube de délivrance de nitinol positionné de manière coulissante à travers la lumière de perfusion (18, 239).

6. Système selon la revendication 1, comprenant en outre un réservoir (428) de fluide cryoablatif en communication fluidique avec la lumière d'administration (20, 365).

7. Système selon la revendication 1, dans lequel le fluide cryoablatif comprend de l'oxyde nitreux.

8. Système selon la revendication 1, dans lequel la sonde allongée (170) définit une partie de traitement actif à proximité ou au niveau de l'extrémité distale (226).

9. Système selon la revendication 1, comprenant en outre un ensemble de gaine (212) positionné de manière coulissante sur la sonde allongée (170).

10. Système selon la revendication 9, dans lequel l'ensemble de gaine (212) comprend une gaine interne (461) et une gaine externe (425) définissant un espacement ou espace annulaire.

11. Système selon la revendication 1, comprenant en outre un ou plusieurs émetteurs (381) et récepteurs (383) positionnés sur la sonde allongée (170).

12. Système selon la revendication 1, comprenant en outre une pompe (225) en communication fluidique avec l'intérieur du revêtement.

13. Système selon la revendication 12, comprenant en outre une vanne (405) couplée fluidiquement à la pompe (225) et qui est reconfigurable pour dévier la pression positive et négative de la pompe vers l'intérieur du revêtement.

14. Système selon la revendication 1, comprenant en outre un ou plusieurs capteurs de pression (487) le long de la sonde allongée.

15. Système selon la revendication 1, dans lequel le revêtement comprend au moins deux parties coniques (176) s'étendant depuis l'extrémité distale de la sonde allongée (170) de telle sorte que les parties coniques sont configurées pour entrer en contact avec une corne utérine correspondante.
